(19) Europäisches Patentamt
European Patent Office
Office européen des brevets

(11) Publication number: **0 589 039 A1**

(12) **EUROPEAN PATENT APPLICATION published in accordance with Art. 158(3) EPC**

(21) Application number: **92908856.5**

(22) Date of filing: **30.04.92**

(86) International application number: **PCT/JP92/00570**

(87) International publication number: **WO 92/19585 (12.11.92 92/28)**

(51) Int. Cl.[5]: **C07C 217/14**, C07C 217/28, C07C 217/42, C07C 219/06, C07C 251/32, C07D 295/08, C07D 317/50, C07F 9/12, A61K 31/135, A61K 31/15, A61K 31/36

(30) Priority: **30.04.91 JP 124583/91**
**30.04.91 JP 124584/91**
**31.05.91 JP 156268/91**
**04.07.91 JP 189495/91**
**06.08.91 JP 219377/91**
**13.08.91 JP 226419/91**
**17.10.91 JP 296641/91**

(43) Date of publication of application: **30.03.94 Bulletin 94/13**

(84) Designated Contracting States: **CH DE ES FR GB IT LI**

(71) Applicant: **ASAHI KASEI KOGYO KABUSHIKI KAISHA**
**2-6, Dojimahama 1-chome**
**Kita-ku**
**Osaka-shi, Osaka 530(JP)**

(72) Inventor: **KOUJI, Hiroyuki**
**179-5, Nakamaru**
**Fuji-shi Shizuoka 416(JP)**
Inventor: **ANDO, Satoshi**
**100-824, Kawanarijima**
**Fuji-shi Shizuoka 416(JP)**

(74) Representative: **Boeters, Hans Dietrich, Dr. et al**
**Patentanwälte Boeters Bauer Koepe**
**Bereiteranger 15**
**D-81541 München (DE)**

(54) **TRIPHENYLETHYLENE DERIVATIVE AND PHARMACEUTICAL PREPARATION CONTAINING THE SAME.**

(57) A triphenylalkene derivative represented by general formula (1), a pharmaceutically acceptable acid addition salt thereof, and a pharmaceutical composition thereof having a tumor inhibitory action and an osteoporosis curing activity. In general formula (1) $R_1$ represents a group selected among those represented by formulae (2), (3) and (4); $R_6$ and $R_7$ represent each hydrogen, alkyl or cycloalkyl, or $R_6$ and $R_7$ together form a heterocyclic group with the adjacent nitrogen atom, provided that $R_6$ and $R_7$ should not be hydrogen atoms at the same time; $R_8$ represents hydrogen or alkylcarbonyl; $R_2$ represents alkyl or cycloalkyl; $R_3$ represents phenyl or 3,4-methylenedioxyphenyl, provided that when $R_3$ represents phenyl, the case where $R_1$ represents a group of formula (4) is excluded; $R_4$ represents hydrogen, hydroxyl, $R_9C(O)O$-, $R_{10}OCH_2O$-, $-OPO(OH)_2$ or $CH = NOR_{11}$; $R_9$ represents alkyl; $R_{10}$ represents alkyl or alkylcarbonyl; $R_5$ represents hydrogen or $CH = NOR_{11}$; and $R_{11}$ represents hydrogen, alkyl or substituted alkyl.

EP 0 589 039 A1

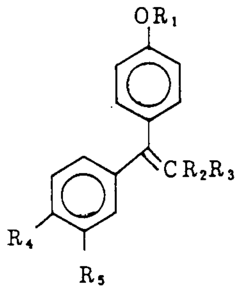

(1)

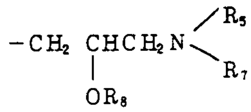

(2)

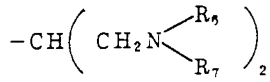

(3)

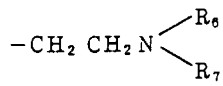

(4)

## FIELD OF THE INVENTION

This invention relates to a triphenylalkene derivative and a pharmaceutical composition which contains the derivative and has a tumor inhibition activity and an osteoporosis curing activity.

## BACKGROUND OF THE INVENTION

It is known that a compound having 1,1,2-triphenyl-1-butene as the basic structure and which is substituted by an aminoalkoxy group at the 1-position phenyl group is capable of showing a non-steroidal antiestrogen activity. A typical example of such a type of compound is (Z)-2-[4-(1,2-diphenyl-1-butenyl)-phenoxy]-N,N-dimethylethylamine (Tamoxifen) which is currently used as an effective drug for the treatment of hormone dependent breast cancer because of the compound's strong antiestrogen activity (British Patent No. 1,013,907).

In addition, it has been reported recently that Tamoxifen is also capable of showing an effect to cure osteoporosis (for example in *Breast Cancer Research and Treatment*, vol.10, pp.31 - 35, 1987; and Abstracts of Papers, 11th Annual Meeting of the American Society for Bone and Mineral Research, title no.425, p.S180).

An object of the present invention is to provide a triphenylalkene derivative which has an excellent anti-breast tumor effect superior to Tamoxifen and is also useful as an osteoporosis curing drug.

## DISCLOSURE OF THE INVENTION

The novel triphenylalkene derivative of the present invention is a triphenylalkene derivative represented by the following general formula (1) or a pharmaceutically acceptable acid addition salt thereof:

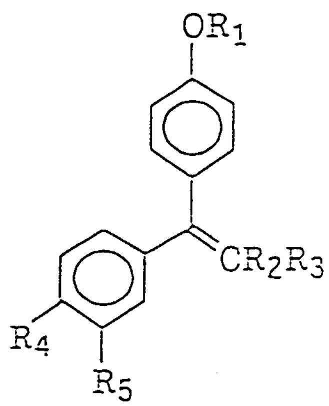

(1)

wherein $R_1$ is selected from the following formulae (2), (3) and (4):

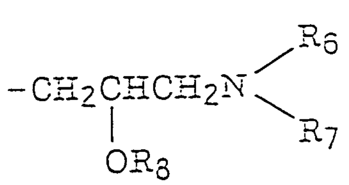

(2)

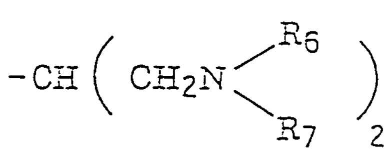

(3)

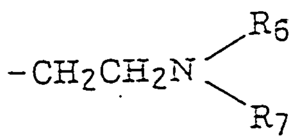

(4)

wherein $R_6$ and $R_7$ may be the same or different and each represents a hydrogen atom, a lower alkyl group or a lower cycloalkyl group, or a group which forms a heterocyclic group containing or not containing a hetero atom (for example, nitrogen, sulfur or oxygen) together with the adjoining nitrogen atom, but $R_6$ and $R_7$ are not hydrogen atoms at the same time; and $R_8$ represents a hydrogen atom or a lower alkylcarbonyl group; $R_2$ represents a lower alkyl group or a lower cycloalkyl group; $R_3$ represents a phenyl group or a 3,4-methylenedioxyphenyl group, provided that $R_1$ is not the formula (4) when $R_3$ is a phenyl group; $R_4$ represents a hydrogen atom, a hydroxyl group, $R_9C(O)O$-, $R_{10}OCH_2O$-, $-OPO(OH)_2$ or $CH=NOR_{11}$ where $R_9$ represents a lower alkyl group and $R_{10}$ represents a lower alkyl group or a lower alkylcarbonyl group; and $R_5$ represents a hydrogen atom or $CH=NOR_{11}$ where $R_{11}$ represents a hydrogen atom, a lower alkyl group, a phenyl group or an alkoxycarbonyl group. In this instance, the term "lower" means that the respective group has 1 to 6 carbon atoms.

The present invention provides a pharmaceutical drug composition having a tumor inhibition function or a pharmaceutical composition having an activity as an osteoporosis curing drug, which is characterized in that it contains the triphenylalkene derivative represented by the above general formula (1) or its pharmaceutically acceptable acid addition salt together with a pharmaceutically acceptable diluent or carrier.

The triphenylalkene derivative of the general formula (1) has E and Z forms which are geometrical isomers against the carbon-carbon double bond of the derivative. These isomers can be distinguished clearly from each other based on the nuclear magnetic resonance signals generated from proton of the ether linkage-adjacent methylene group. A mixture of these E and Z isomers and each of the isolated isomers are all included in the present invention. Also, the triphenylalkene derivative of the general formula (1) sometimes has R and S forms which are optical isomers in relation to a carbon atom to which a hydroxyl group of an aminoalkyl side chain as shown in the formula (2) is linked. These isomers can be distinguished and separated clearly from each other by liquid chromatography using an optical isomer separation column. In addition, each of the R and S forms can be obtained as an optically active triphenylalkene derivative making use of an optically active oxirane derivative as a raw material. Also, it is possible to effect optical resolution by forming a salt using an optically active acid. A mixture of these R and S isomers and each of the isolated isomers are also included in the present invention.

The following are illustrative examples of the triphenylalkene derivative of the present invention:

1-[4-(3-dimethylamino-2-hydroxypropoxy)phenyl]-1-(4-hydroxyphenyl)-2-(3,4-methylenedioxyphenyl)-1-butene;

1-[4-(3-diethylamino-2-hydroxypropoxy)phenyl]-1-(4-hydroxyphenyl)-2-(3,4-methylenedioxyphenyl)-1-butene;

1-[4-(3-ethylmethylamino-2-hydroxypropoxy)phenyl]-1-(4-hydroxyphenyl)-2-(3,4-methylenedioxyphenyl)-1-butene;

1-[4-(3-pyrrolidino-2-hydroxypropoxy)phenyl]-1-(4-hydroxyphenyl)-2-(3,4-methylenedioxyphenyl)-1-butene;

1-[4-(3-piperidino-2-hydroxypropoxy)phenyl]-1-(4-hydroxyphenyl)-2-(3,4-methylenedioxyphenyl)-1-butene;

1-[4-(3-cyclohexylmethylamino-2-hydroxypropoxy)-phenyl]-1-(4-hydroxyphenyl)-2-(3,4-methylenedioxyphenyl)-1-butene;

1-[4-(3-dimethylamino-2-hydroxypropoxy)phenyl]-1-phenyl-2-(3,4-methylenedioxyphenyl)-1-butene;

1-[4-(3-diethylamino-2-hydroxypropoxy)phenyl]-1-phenyl-2-(3,4-methylenedioxyphenyl)-1-butene;

1-[4-(3-ethylmethylamino-2-hydroxypropoxy)phenyl]-1-phenyl-2-(3,4-methylenedioxyphenyl)-1-butene;

1-[4-(3-pyrrolidino-2-hydroxypropoxy)phenyl]-1-phenyl-2-(3,4-methylenedioxyphenyl)-1-butene;

1-[4-(3-piperidino-2-hydroxypropoxy)phenyl]-1-phenyl-2-(3,4-methylenedioxyphenyl)-1-butene;

1-[4-(3-cyclohexylmethylamino-2-hydroxypropoxy)-phenyl]-1-phenyl-2-(3,4-methylenedioxyphenyl)-1-butene;

1-[4-(3-dimethylamino-2-hydroxypropoxy)phenyl]-1-(4-methoxymethoxyphenyl)-2-(3,4-methylenedioxyphenyl)-1-butene;

1-[4-(3-diethylamino-2-hydroxypropoxy)phenyl]-1-(4-methoxymethoxyphenyl)-2-(3,4-methylenedioxyphenyl)-1-butene;

1-[4-(3-ethylmethylamino-2-hydroxypropoxy)phenyl]-1-(4-methoxymethoxyphenyl)-2-(3,4-methylenedioxyphenyl)-1-butene;

1-[4-(3-pyrrolidino-2-hydroxypropoxy)phenyl]-1-(4-methoxymethoxyphenyl)-2-(3,4-methylenedioxyphenyl)-1-butene;

1-[4-(3-piperidino-2-hydroxypropoxy)phenyl]-1-(4-methoxymethoxyphenyl)-2-(3,4-methylenedioxyphenyl)-1-butene;

1-[4-(3-cyclohexylmethylamino-2-hydroxypropoxy)-phenyl]-1-(4-methoxymethoxyphenyl)-2-(3,4-methylenedioxyphenyl)-1-butene;

1-[4-(3-dimethylamino-2-hydroxypropoxy)phenyl]-1-(4-acetoxyphenyl)-2-(3,4-methylenedioxyphenyl)-1-butene;

1-[4-(3-diethylamino-2-hydroxypropoxy)phenyl]-1-(4-acetoxyphenyl)-2-(3,4-methylenedioxyphenyl)-1-butene;

1-[4-(3-ethylmethylamino-2-hydroxypropoxy)phenyl]-1-(4-acetoxyphenyl)-2-(3,4-methylenedioxyphenyl)-1-butene;

1-[4-(3-pyrrolidino-2-hydroxypropoxy)phenyl]-1-(4-acetoxyphenyl)-2-(3,4-methylenedioxyphenyl)-1-butene;

1-[4-(3-piperidino-2-hydroxypropoxy)phenyl]-1-(4-acetoxyphenyl)-2-(3,4-methylenedioxyphenyl)-1-butene;

1-[4-(3-cyclohexylmethylamino-2-hydroxypropoxy)-phenyl]-1-(4-acetoxyphenyl)-2-(3,4-methylenedioxyphenyl)-1-butene;

1-[4-(3-dimethylamino-2-hydroxypropoxy)phenyl]-1-(4-dihydroxyphosphinooxyphenyl)-2-(3,4-methylenedioxyphenyl)-1-butene;

1-[4-(3-diethylamino-2-hydroxypropoxy)phenyl]-1-(4-dihydroxyphosphinooxyphenyl)-2-(3,4-methylenedioxyphenyl)-1-butene;

1-[4-(3-ethylmethylamino-2-hydroxypropoxy)phenyl]-1-(4-dihydroxyphosphinooxyphenyl)-2-(3,4-methylenedioxyphenyl)-1-butene;

1-[4-(3-pyrrolidino-2-hydroxypropoxy)phenyl]-1-(4-dihydroxyphosphinooxyphenyl)-2-(3,4-methylenedioxyphenyl)-1-butene;

1-[4-(3-piperidino-2-hydroxypropoxy)phenyl]-1-(4-dihydroxyphosphinooxyphenyl)-2-(3,4-methylenedioxyphenyl)-1-butene;

1-[4-(3-cyclohexylmethylamino-2-hydroxypropoxy)-phenyl]-1-(4-dihydroxyphosphinooxyphenyl)-2-(3,4-methylenedioxyphenyl)-1-butene;

1-[4-(3-dimethylamino-2-hydroxypropoxy)phenyl]-1-(4-benzoyloxyphenyl)-2-(3,4-methylenedioxyphenyl)-1-butene;

1-[4-(3-diethylamino-2-hydroxypropoxy)phenyl]-1-(4-benzoyloxyphenyl)-2-(3,4-methylenedioxyphenyl)-1-butene;

1-[4-(3-ethylmethylamino-2-hydroxypropoxy)phenyl]-1-(4-benzoyloxyphenyl)-2-(3,4-methylenedioxyphenyl)-1-butene;

1-[4-(3-pyrrolidino-2-hydroxypropoxy)phenyl]-1-(4-benzoyloxyphenyl)-2-(3,4-methylenedioxyphenyl)-1-butene;

1-[4-(3-piperidino-2-hydroxypropoxy)phenyl]-1-(4-benzoyloxyphenyl)-2-(3,4-methylenedioxyphenyl)-1-butene;

1-[4-(3-cyclohexylmethylamino-2-hydroxypropoxy)-phenyl]-1-(4-benzoyloxyphenyl)-2-(3,4-methylenedioxyphenyl)-1-butene;

1-[4-(3-dimethylamino-2-hydroxypropoxy)phenyl]-1-(4-pivaloyloxymethoxyphenyl)-2-(3,4-methylenedioxyphenyl)-1-butene;

1-[4-(3-diethylamino-2-hydroxypropoxy)phenyl]-1-(4-pivaloyloxymethoxyphenyl)-2-(3,4-methylenedioxyphenyl)-1-butene;

1-[4-(3-ethylmethylamino-2-hydroxypropoxy)phenyl]-1-(4-pivaloyloxymethoxyphenyl)-2-(3,4-methylenedioxyphenyl)-1-butene;

1-[4-(3-pyrrolidino-2-hydroxypropoxy)phenyl]-1-(4-pivaloyloxymethoxyphenyl)-2-(3,4-methylenedioxyphenyl)-1-butene;

1-[4-(3-piperidino-2-hydroxypropoxy)phenyl]-1-(4-pivaloyloxymethoxyphenyl)-2-(3,4-methylenedioxyphenyl)-1-butene;

1-[4-(3-cyclohexylmethylamino-2-hydroxypropoxy)-phenyl]-1-(4-pivaloyloxymethoxyphenyl)-2-(3,4-methylenedioxyphenyl)-1-butene;

1-[4-(3-dimethylamino-2-hydroxypropoxy)phenyl]-1-(4-hydroxyphenyl)-2-(3,4-methylenedioxyphenyl)-2-cyclopropylethene;

1-[4-(3-diethylamino-2-hydroxypropoxy)phenyl]-1-(4-hydroxyphenyl)-2-(3,4-methylenedioxyphenyl)-2-cyclopropylethene;

1-[4-(3-ethylmethylamino-2-hydroxypropoxy)phenyl]-1-(4-hydroxyphenyl)-2-(3,4-methylenedioxyphenyl)-2-cyclopropylethene;

1-[4-(3-pyrrolidino-2-hydroxypropoxy)phenyl]-1-(4-hydroxyphenyl)-2-(3,4-methylenedioxyphenyl)-2-cyclopropylethene;

1-[4-(3-piperidino-2-hydroxypropoxy)phenyl]-1-(4-hydroxyphenyl)-2-(3,4-methylenedioxyphenyl)-2-cyclopropylethene;

1-[4-(3-cyclohexylmethylamino-2-hydroxypropoxy)-phenyl]-1-(4-hydroxyphenyl)-2-(3,4-methylenedioxyphenyl)-2-cyclopropylethene;

1-[4-(3-dimethylamino-2-hydroxypropoxy)phenyl]-1-(4-cyclopropanecarbonyloxyphenyl)-2-(3,4-methylenedioxyphenyl)-1-butene;

1-[4-(3-diethylamino-2-hydroxypropoxy)phenyl]-1-(4-cyclopropanecarbonyloxyphenyl)-2-(3,4-methylenedioxyphenyl)-1-butene;

1-[4-(3-ethylmethylamino-2-hydroxypropoxy)phenyl]-1-(4-cyclopropanecarbonyloxyphenyl)-2-(3,4-methylenedioxyphenyl)-1-butene;

1-[4-(3-pyrrolidino-2-hydroxypropoxy)phenyl]-1-(4-cyclopropanecarbonyloxyphenyl)-2-(3,4-methylenedioxyphenyl)-1-butene;

1-[4-(3-piperidino-2-hydroxypropoxy)phenyl]-1-(4-cyclopropanecarbonyloxyphenyl)-2-(3,4-methylenedioxyphenyl)-1-butene;

1-[4-(3-cyclohexylmethylamino-2-hydroxypropoxy)-phenyl]-1-(4-cyclopropanecarbonyloxyphenyl)-2-(3,4-methylenedioxyphenyl)-1-butene;

1-[4-(3-dimethylamino-2-hydroxypropoxy)phenyl]-1-(4-hydroxyphenyl)-2-phenyl-1-butene;

1-[4-(3-diethylamino-2-hydroxypropoxy)phenyl]-1-(4-hydroxyphenyl)-2-phenyl-1-butene;

1-[4-(3-ethylmethylamino-2-hydroxypropoxy)phenyl]-1-(4-hydroxyphenyl)-2-phenyl-1-butene;

1-[4-(3-pyrrolidino-2-hydroxypropoxy)phenyl]-1-(4-hydroxyphenyl)-2-phenyl-1-butene;

1-[4-(3-piperidino-2-hydroxypropoxy)phenyl]-1-(4-hydroxyphenyl)-2-phenyl-1-butene;

1-[4-(3-cyclohexylmethylamino-2-hydroxypropoxy)-phenyl]-1-(4-hydroxyphenyl)-2-phenyl-1-butene;

1-[4-(3-dimethylamino-2-hydroxypropoxy)phenyl]-1,2-diphenyl-1-butene;

1-[4-(3-diethylamino-2-hydroxypropoxy)phenyl]-1,2-diphenyl-1-butene;

1-[4-(3-ethylmethylamino-2-hydroxypropoxy)phenyl]-1,2-diphenyl-1-butene;

1-[4-(3-pyrrolidino-2-hydroxypropoxy)phenyl]-1,2-diphenyl-1-butene;

1-[4-(3-piperidino-2-hydroxypropoxy)phenyl]-1,2-diphenyl-1-butene;

1-[4-(3-cyclohexylmethylamino-2-hydroxypropoxy)-phenyl]-1,2-diphenyl-1-butene;

1-[4-(3-dimethylamino-2-hydroxypropoxy)phenyl]-1-(4-methoxymethoxyphenyl)-2-phenyl-1-butene;

1-[4-(3-diethylamino-2-hydroxypropoxy)phenyl]-1-(4-methoxymethoxyphenyl)-2-phenyl-1-butene;

1-[4-(3-ethylmethylamino-2-hydroxypropoxy)phenyl]-1-(4-methoxymethoxyphenyl)-2-phenyl-1-butene;

1-[4-(3-pyrrolidino-2-hydroxypropoxy)phenyl]-1-(4-methoxymethoxyphenyl)-2-phenyl-1-butene;

1-[4-(3-piperidino-2-hydroxypropoxy)phenyl]-1-(4-methoxymethoxyphenyl)-2-phenyl-1-butene;

1-[4-(3-cyclohexylmethylamino-2-hydroxypropoxy)-phenyl]-1-(4-methoxymethoxyphenyl)-2-phenyl-1-butene;

1-[4-(3-dimethylamino-2-hydroxypropoxy)phenyl]-1-(4-acetoxyphenyl)-2-phenyl-1-butene;

1-[4-(3-diethylamino-2-hydroxypropoxy)phenyl]-1-(4-acetoxyphenyl)-2-phenyl-1-butene;

1-[4-(3-ethylmethylamino-2-hydroxypropoxy)phenyl]-1-(4-acetoxyphenyl)-2-phenyl-1-butene;

1-[4-(3-pyrrolidino-2-hydroxypropoxy)phenyl]-1-(4-acetoxyphenyl)-2-phenyl-1-butene;

1-[4-(3-piperidino-2-hydroxypropoxy)phenyl]-1-(4-acetoxyphenyl)-2-phenyl-1-butene;

1-[4-(3-cyclohexylmethylamino-2-hydroxypropoxy)-phenyl]-1-(4-acetoxyphenyl)-2-phenyl-1-butene;

1-[4-(3-dimethylamino-2-hydroxypropoxy)phenyl]-1-(4-dihydroxyphosphinooxyphenyl)-2-phenyl-1-butene;

1-[4-(3-diethylamino-2-hydroxypropoxy)phenyl]-1-(4-dihydroxyphosphinooxyphenyl)-2-phenyl-1-butene;

1-[4-(3-ethylmethylamino-2-hydroxypropoxy)phenyl]-1-(4-dihydroxyphosphinooxyphenyl)-2-phenyl-1-butene;

1-[4-(3-pyrrolidino-2-hydroxypropoxy)phenyl]-1-(4-dihydroxyphosphinooxyphenyl)-2-phenyl-1-butene;

1-[4-(3-piperidino-2-hydroxypropoxy)phenyl]-1-(4-dihydroxyphosphinooxyphenyl)-2-phenyl-1-butene;

1-[4-(3-cyclohexylmethylamino-2-hydroxypropoxy)-phenyl]-1-(4-dihydroxyphosphinooxyphenyl)-2-phenyl-1-butene;

1-[4-(3-dimethylamino-2-hydroxypropoxy)phenyl]-1-(4-benzoyloxyphenyl)-2-phenyl-1-butene;

1-[4-(3-diethylamino-2-hydroxypropoxy)phenyl]-1-(4-benzoyloxyphenyl)-2-phenyl-1-butene;
1-[4-(3-ethylmethylamino-2-hydroxypropoxy)phenyl]-1-(4-benzoyloxyphenyl)-2-phenyl-1-butene;
1-[4-(3-pyrrolidino-2-hydroxypropoxy)phenyl]-1-(4-benzoyloxyphenyl)-2-phenyl-1-butene;
1-[4-(3-piperidino-2-hydroxypropoxy)phenyl]-1-(4-benzoyloxyphenyl)-2-phenyl-1-butene;
1-[4-(3-cyclohexylmethylamino-2-hydroxypropoxy)-phenyl]-1-(4-benzoyloxyphenyl)-2-phenyl-1-butene;
1-[4-(3-dimethylamino-2-hydroxypropoxy)phenyl]-1-(4-pivaloyloxymethoxyphenyl)-2-phenyl-1-butene;
1-[4-(3-diethylamino-2-hydroxypropoxy)phenyl]-1-(4-pivaloyloxymethoxyphenyl)-2-phenyl-1-butene;
1-[4-(3-ethylmethylamino-2-hydroxypropoxy)phenyl]-1-(4-pivaloyloxymethoxyphenyl)-2-phenyl-1-butene;
1-[4-(3-pyrrolidino-2-hydroxypropoxy)phenyl]-1-(4-pivaloyloxymethoxyphenyl)-2-phenyl-1-butene;
1-[4-(3-piperidino-2-hydroxypropoxy)phenyl]-1-(4-pivaloyloxymethoxyphenyl)-2-phenyl-1-butene;
1-[4-(3-cyclohexylmethylamino-2-hydroxypropoxy)-phenyl]-1-(4-pivaloyloxymethoxyphenyl)-2-phenyl-1-butene;
1-[4-(3-dimethylamino-2-hydroxypropoxy)phenyl]-1-(4-hydroxyphenyl)-2-phenyl-2-cyclopropylethene;
1-[4-(3-diethylamino-2-hydroxypropoxy)phenyl]-1-(4-hydroxyphenyl)-2-phenyl-2-cyclopropylethene;
1-[4-(3-ethylmethylamino-2-hydroxypropoxy)phenyl]-1-(4-hydroxyphenyl)-2-phenyl-2-cyclopropylethene;
1-[4-(3-pyrrolidino-2-hydroxypropoxy)phenyl]-1-(4-hydroxyphenyl)-2-phenyl-2-cyclopropylethene;
1-[4-(3-piperidino-2-hydroxypropoxy)phenyl]-1-(4-hydroxyphenyl)-2-phenyl-2-cyclopropylethene;
1-[4-(3-cyclohexylmethylamino-2-hydroxypropoxy)-phenyl]-1-(4-hydroxyphenyl)-2-phenyl-2-cyclopropylethene;
1-[4-(3-dimethylamino-2-hydroxypropoxy)phenyl]-1-(4-cyclopropanecarbonyloxyphenyl)-2-phenyl-1-butene;
1-[4-(3-diethylamino-2-hydroxypropoxy)phenyl]-1-(4-cyclopropanecarbonyloxyphenyl)-2-phenyl-1-butene;
1-[4-(3-ethylmethylamino-2-hydroxypropoxy)phenyl]-1-(4-cyclopropanecarbonyloxyphenyl)-2-phenyl-1-butene;
1-[4-(3-pyrrolidino-2-hydroxypropoxy)phenyl]-1-(4-cyclopropanecarbonyloxyphenyl)-2-phenyl-1-butene;
1-[4-(3-piperidino-2-hydroxypropoxy)phenyl]-1-(4-cyclopropanecarbonyloxyphenyl)-2-phenyl-1-butene;
1-[4-(3-cyclohexylmethylamino-2-hydroxypropoxy)-phenyl]-1-(4-cyclopropanecarbonyloxyphenyl)-2-phenyl-1-butene;
1-[4-(3-methylamino-2-hydroxypropoxy)phenyl]-1-(4-hydroxyphenyl)-2-(3,4-methylenedioxyphenyl)-1-butene;
1-[4-(3-ethylamino-2-hydroxypropoxy)phenyl]-1-(4-hydroxyphenyl)-2-(3,4-methylenedioxyphenyl)-1-butene;
1-[4-(3-isopropylamino-2-hydroxypropoxy)phenyl]-1-(4-hydroxyphenyl)-2-(3,4-methylenedioxyphenyl)-1-butene;
1-[4-(3-cyclopentylamino-2-hydroxypropoxy)phenyl]-1-(4-hydroxyphenyl)-2-(3,4-methylenedioxyphenyl)-1-butene;
1-[4-(3-cyclohexylamino-2-hydroxypropoxy)phenyl]-1-(4-hydroxyphenyl)-2-(3,4-methylenedioxyphenyl)-1-butene;
1-[4-(3-methylamino-2-hydroxypropoxy)phenyl]-1-phenyl-2-(3,4-methylenedioxyphenyl)-1-butene;
1-[4-(3-ethylamino-2-hydroxypropoxy)phenyl]-1-phenyl-2-(3,4-methylenedioxyphenyl)-1-butene;
1-[4-(3-isopropylamino-2-hydroxypropoxy)phenyl]-1-phenyl-2-(3,4-methylenedioxyphenyl)-1-butene;
1-[4-(3-cyclopentylamino-2-hydroxypropoxy)phenyl]-1-phenyl-2-(3,4-methylenedioxyphenyl)-1-butene;
1-[4-(3-cyclohexylamino-2-hydroxypropoxy)phenyl]-1-phenyl-2-(3,4-methylenedioxyphenyl)-1-butene;
1-[4-(3-methylamino-2-hydroxypropoxy)phenyl]-1-(4-methoxymethoxyphenyl)-2-(3,4-methylenedioxyphenyl)-1-butene;
1-[4-(3-ethylamino-2-hydroxypropoxy)phenyl]-1-(4-methoxymethoxyphenyl)-2-(3,4-methylenedioxyphenyl)-1-butene;
1-[4-(3-isopropylamino-2-hydroxypropoxy)phenyl]-1-(4-methoxymethoxyphenyl)-2-(3,4-methylenedioxyphenyl)-1-butene;
1-[4-(3-cyclopentylamino-2-hydroxypropoxy)phenyl]-1-(4-methoxymethoxyphenyl)-2-(3,4-methylenedioxyphenyl)-1-butene;
1-[4-(3-cyclohexylamino-2-hydroxypropoxy)phenyl]-1-(4-methoxymethoxyphenyl)-2-(3,4-methylenedioxyphenyl)-1-butene;
1-[4-(3-methylamino-2-hydroxypropoxy)phenyl]-1-(4-acetoxyphenyl)-2-(3,4-methylenedioxyphenyl)-1-butene;
1-[4-(3-ethylamino-2-hydroxypropoxy)phenyl]-1-(4-acetoxyphenyl)-2-(3,4-methylenedioxyphenyl)-1-butene;

1-[4-(3-isopropylamino-2-hydroxypropoxy)phenyl]-1-(4-acetoxyphenyl)-2-(3,4-methylenedioxyphenyl)-1-butene;

1-[4-(3-cyclopentylamino-2-hydroxypropoxy)phenyl]-1-(4-acetoxyphenyl)-2-(3,4-methylenedioxyphenyl)-1-butene;

1-[4-(3-cyclohexylamino-2-hydroxypropoxy)phenyl]-1-(4-acetoxyphenyl)-2-(3,4-methylenedioxyphenyl)-1-butene;

1-[4-(3-methylamino-2-hydroxypropoxy)phenyl]-1-(4-dihydroxyphosphinooxyphenyl)-2-(3,4-methylenedioxyphenyl)-1-butene;

1-[4-(3-ethylamino-2-hydroxypropoxy)phenyl]-1-(4-dihydroxyphosphinooxyphenyl)-2-(3,4-methylenedioxyphenyl)-1-butene;

1-[4-(3-isopropylamineamino-2-hydroxypropoxy)phenyl]-1-(4-dihydroxyphosphinooxyphenyl)-2-(3,4-methylenedioxyphenyl)-1-butene;

1-[4-(3-cyclopentylamine-2-hydroxypropoxy)phenyl]-1-(4-dihydroxyphosphinooxyphenyl)-2-(3,4-methylenedioxyphenyl)-1-butene;

1-[4-(3-cyclohexylamino-2-hydroxypropoxy)phenyl]-1-(4-dihydroxyphosphinooxyphenyl)-2-(3,4-methylenedioxyphenyl)-1-butene;

1-[4-(3-methylamino-2-hydroxypropoxy)phenyl]-1-(4-benzoyloxyphenyl)-2-(3,4-methylenedioxyphenyl)-1-butene;

1-[4-(3-ethylamino-2-hydroxypropoxy)phenyl]-1-(4-benzoyloxyphenyl)-2-(3,4-methylenedioxyphenyl)-1-butene;

1-[4-(3-isopropylamino-2-hydroxypropoxy)phenyl]-1-(4-benzoyloxyphenyl)-2-(3,4-methylenedioxyphenyl)-1-butene;

1-[4-(3-cyclopentylamino-2-hydroxypropoxy)phenyl]-1-(4-benzoyloxyphenyl)-2-(3,4-methylenedioxyphenyl)-1-butene;

1-[4-(3-cyclohexylamino-2-hydroxypropoxy)phenyl]-1-(4-benzoyloxyphenyl)-2-(3,4-methylenedioxyphenyl)-1-butene;

1-[4-(3-methylamino-2-hydroxypropoxy)phenyl]-1-(4-pivaloyloxymethoxyphenyl)-2-(3,4-methylenedioxyphenyl)-1-butene;

1-[4-(3-ethylamino-2-hydroxypropoxy)phenyl]-1-(4-pivaloyloxymethoxyphenyl)-2-(3,4-methylenedioxyphenyl)-1-butene;

1-[4-(3-isopropylamino-2-hydroxypropoxy)phenyl]-1-(4-pivaloyloxymethoxyphenyl)-2-(3,4-methylenedioxyphenyl)-1-butene;

1-[4-(3-cyclopentylamino-2-hydroxypropoxy)phenyl]-1-(4-pivaloyloxymethoxyphenyl)-2-(3,4-methylenedioxyphenyl)-1-butene;

1-[4-(3-cyclohexylamino-2-hydroxypropoxy)phenyl]-1-(4-pivaloyloxymethoxyphenyl)-2-(3,4-methylenedioxyphenyl)-1-butene;

1-[4-(3-methylamino-2-hydroxypropoxy)phenyl]-1-(4-hydroxyphenyl)-2-(3,4-methylenedioxyphenyl)-2-cyclopropylethene;

1-[4-(3-ethylamino-2-hydroxypropoxy)phenyl]-1-(4-hydroxyphenyl)-2-(3,4-methylenedioxyphenyl)-2-cyclopropylethene;

1-[4-(3-isopropylamino-2-hydroxypropoxy)phenyl]-1-(4-hydroxyphenyl)-2-(3,4-methylenedioxyphenyl)-2-cyclopropylethene;

1-[4-(3-cyclopentylamino-2-hydroxypropoxy)phenyl]-1-(4-hydroxyphenyl)-2-(3,4-methylenedioxyphenyl)-2-cyclopropylethene;

1-[4-(3-cyclohexylamino-2-hydroxypropoxy)phenyl]-1-(4-hydroxyphenyl)-2-(3,4-methylenedioxyphenyl)-2-cyclopropylethene;

1-[4-(3-methylamino-2-hydroxypropoxy)phenyl]-1-(4-cyclopropanecarbonyloxyphenyl)-2-(3,4-methylenedioxyphenyl)-1-butene;

1-[4-(3-ethylamino-2-hydroxypropoxy)phenyl]-1-(4-cyclopropanecarbonyloxyphenyl)-2-(3,4-methylenedioxyphenyl)-1-butene;

1-[4-(3-isopropylamino-2-hydroxypropoxy)phenyl]-1-(4-cyclopropanecarbonyloxyphenyl)-2-(3,4-methylenedioxyphenyl)-1-butene;

1-[4-(3-cyclopentylamino-2-hydroxypropoxy)phenyl]-1-(4-cyclopropanecarbonyloxyphenyl)-2-(3,4-methylenedioxyphenyl)-1-butene;

1-[4-(3-cyclohexylamino-2-hydroxypropoxy)phenyl]-1-(4-cyclopropanecarbonyloxyphenyl)-2-(3,4-methylenedioxyphenyl)-1-butene;

1-[4-(3-methylamino-2-hydroxypropoxy)phenyl]-1-(4-hydroxyphenyl)-2-phenyl-1-butene;

1-[4-(3-ethylamino-2-hydroxypropoxy)phenyl]-1-(4-hydroxyphenyl)-2-phenyl-1-butene;

1-[4-(3-isopropylamino-2-hydroxypropoxy)phenyl]-1-(4-hydroxyphenyl)-2-phenyl-1-butene;
1-[4-(3-cyclopentylamino-2-hydroxypropoxy)phenyl]-1-(4-hydroxyphenyl)-2-phenyl-1-butene;
1-[4-(3-cyclohexylamino-2-hydroxypropoxy)phenyl]-1-(4-hydroxyphenyl)-2-phenyl-1-butene;
1-[4-(3-methylamino-2-hydroxypropoxy)phenyl]-1,2-diphenyl-1-butene;
1-[4-(3-ethylamino-2-hydroxypropoxy)phenyl]-1,2-diphenyl-1-butene;
1-[4-(3-isopropylamino-2-hydroxypropoxy)phenyl]-1,2-diphenyl-1-butene;
1-[4-(3-cyclopentylamino-2-hydroxypropoxy)phenyl]-1,2-diphenyl-1-butene;
1-[4-(3-cyclohexylamino-2-hydroxypropoxy)phenyl]-1,2-diphenyl-1-butene;
1-[4-(3-dimethylamino-2-acetoxypropoxy)phenyl]-1-(4-hydroxyphenyl)-2-(3,4-methylenedioxyphenyl)-1-butene;
1-[4-(3-diethylamino-2-acetoxypropoxy)phenyl]-1-(4-hydroxyphenyl)-2-(3,4-methylenedioxyphenyl)-1-butene;
1-[4-(3-ethylmethylamino-2-acetoxypropoxy)phenyl]-1-(4-hydroxyphenyl)-2-(3,4-methylenedioxyphenyl)-1-butene;
1-[4-(3-pyrrolidino-2-acetoxypropoxy)phenyl]-1-(4-hydroxyphenyl)-2-(3,4-methylenedioxyphenyl)-1-butene;
1-[4-(3-piperidino-2-acetoxypropoxy)phenyl]-1-(4-hydroxyphenyl)-2-(3,4-methylenedioxyphenyl)-1-butene;
1-[4-(3-cyclohexylmethylamino-2-acetoxypropoxy)-phenyl]-1-(4-hydroxyphenyl)-2-(3,4-methylenedioxyphenyl)-1-butene;
1-[4-(3-dimethylamino-2-acetoxypropoxy)phenyl]-1-phenyl-2-(3,4-methylenedioxyphenyl)-1-butene;
1-[4-(3-diethylamino-2-acetoxypropoxy)phenyl]-1-phenyl-2-(3,4-methylenedioxyphenyl)-1-butene;
1-[4-(3-ethylmethylamino-2-acetoxypropoxy)phenyl]-1-phenyl-2-(3,4-methylenedioxyphenyl)-1-butene;
1-[4-(3-pyrrolidino-2-acetoxypropoxy)phenyl]-1-phenyl-2-(3,4-methylenedioxyphenyl)-1-butene;
1-[4-(3-piperidino-2-acetoxypropoxy)phenyl]-1-phenyl-2-(3,4-methylenedioxyphenyl)-1-butene;
1-[4-(3-cyclohexylmethylamino-2-acetoxypropoxy)-phenyl]-1-phenyl-2-(3,4-methylenedioxyphenyl)-1-butene;
1-[4-(3-dimethylamino-2-acetoxypropoxy)phenyl]-1-(4-methoxymethoxyphenyl)-2-(3,4-methylenedioxyphenyl)-1-butene;
1-[4-(3-diethylamino-2-acetoxypropoxy)phenyl]-1-(4-methoxymethoxyphenyl)-2-(3,4-methylenedioxyphenyl)-1-butene; 1-[4-(3-ethylmethylamino-2-acetoxypropoxy)phenyl]-1-(4-methoxymethoxyphenyl)-2-(3,4-methylenedioxyphenyl)-1-butene;
1-[4-(3-pyrrolidino-2-acetoxypropoxy)phenyl]-1-(4-methoxymethoxyphenyl)-2-(3,4-methylenedioxyphenyl)-1-butene;
1-[4-(3-piperidino-2-acetoxypropoxy)phenyl]-1-(4-methoxymethoxyphenyl)-2-(3,4-methylenedioxyphenyl)-1-butene;
1-[4-(3-cyclohexylmethylamino-2-acetoxypropoxy)-phenyl]-1-(4-methoxymethoxyphenyl)-2-(3,4-methylenedioxyphenyl)-1-butene;
1-[4-(3-dimethylamino-2-acetoxypropoxy)phenyl]-1-(4-acetoxyphenyl)-2-(3,4-methylenedioxyphenyl)-1-butene;
1-[4-(3-diethylamino-2-acetoxypropoxy)phenyl]-1-(4-acetoxyphenyl)-2-(3,4-methylenedioxyphenyl)-1-butene;
1-[4-(3-ethylmethylamino-2-acetoxypropoxy)phenyl]-1-(4-acetoxyphenyl)-2-(3,4-methylenedioxyphenyl)-1-butene;
1-[4-(3-pyrrolidino-2-acetoxypropoxy)phenyl]-1-(4-acetoxyphenyl)-2-(3,4-methylenedioxyphenyl)-1-butene;
1-[4-(3-piperidino-2-acetoxypropoxy)phenyl]-1-(4-acetoxyphenyl)-2-(3,4-methylenedioxyphenyl)-1-butene;
1-[4-(3-cyclohexylmethylamino-2-acetoxypropoxy)-phenyl]-1-(4-acetoxyphenyl)-2-(3,4-methylenedioxyphenyl)-1-butene;
1-[4-(3-dimethylamino-2-acetoxypropoxy)phenyl]-1-(4-(dihydroxyphosphinooxyphenyl)-2-(3,4-methylenedioxyphenyl)-1-butene;
1-[4-(3-diethylamino-2-acetoxypropoxy)phenyl]-1-(4-dihydroxyphosphinooxyphenyl)-2-(3,4-methylenedioxyphenyl)-1-butene;
1-[4-(3-ethylmethylamino-2-acetoxypropoxy)phenyl]-1-(4-dihydroxyphosphinooxyphenyl)-2-(3,4-methylenedioxyphenyl)-1-butene;
1-[4-(3-pyrrolidino-2-acetoxypropoxy)phenyl]-1-(4-dihydroxyphosphinooxyphenyl)-2-(3,4-methylenedioxyphenyl)-1-butene;

1-[4-(3-piperidino-2-acetoxypropoxy)phenyl]-1-(4-dihydroxyphosphinooxyphenyl)-2-(3,4-methylenedioxyphenyl)-1-butene;

1-[4-(3-cyclohexylmethylamino-2-acetoxypropoxy)-phenyl]-1-(4-dihydroxyphosphinooxyphenyl)-2-(3,4-methylenedioxyphenyl)-1-butene;

1-[4-(3-dimethylamino-2-acetoxypropoxy)phenyl]-1-(4-benzoyloxyphenyl)-2-(3,4-methylenedioxyphenyl)-1-butene;

1-[4-(3-diethylamino-2-acetoxypropoxy)phenyl]-1-(4-benzoyloxyphenyl)-2-(3,4-methylenedioxyphenyl)-1-butene;

1-[4-(3-ethylmethylamino-2-acetoxypropoxy)phenyl]-1-(4-benzoyloxyphenyl)-2-(3,4-methylenedioxyphenyl)-1-butene;

1-[4-(3-pyrrolidino-2-acetoxypropoxy)phenyl]-1-(4-benzoyloxyphenyl)-2-(3,4-methylenedioxyphenyl)-1-butene;

1-[4-(3-piperidino-2-acetoxypropoxy)phenyl]-1-(4-benzoyloxyphenyl)-2-(3,4-methylenedioxyphenyl)-1-butene;

1-[4-(3-cyclohexylmethylamino-2-acetoxypropoxy)-phenyl]-1-(4-benzoyloxyphenyl)-2-(3,4-methylenedioxyphenyl)-1-butene;

1-[4-(3-dimethylamino-2-acetoxypropoxy)phenyl]-1-(4-pivaloyloxymethoxyphenyl)-2-(3,4-methylenedioxyphenyl)-1-butene;

1-[4-(3-diethylamino-2-acetoxypropoxy)phenyl]-1-(4-pivaloyloxymethoxyphenyl)-2-(3,4-methylenedioxyphenyl)-1-butene;

1-[4-(3-ethylmethylamino-2-acetoxypropoxy)phenyl]-1-(4-pivaloyloxymethoxyphenyl)-2-(3,4-methylenedioxyphenyl)-1-butene;

1-[4-(3-pyrrolidino-2-acetoxypropoxy)phenyl]-1-(4-pivaloyloxymethoxyphenyl)-2-(3,4-methylenedioxyphenyl)-1-butene;

1-[4-(3-piperidino-2-acetoxypropoxy)phenyl]-1-(4-pivaloyloxymethoxyphenyl)-2-(3,4-methylenedioxyphenyl)-1-butene;

1-[4-(3-cyclohexylmethylamino-2-acetoxypropoxy)-phenyl]-1-(4-pivaloyloxymethoxyphenyl)-2-(3,4-methylenedioxyphenyl)-1-butene;

1-[4-(3-dimethylamino-2-acetoxypropoxy)phenyl]-1-(4-hydroxyphenyl)-2-(3,4-methylenedioxyphenyl)-2-cyclopropylethene;

1-[4-(3-diethylamino-2-acetoxypropoxy)phenyl]-1-(4-hydroxyphenyl)-2-(3,4-methylenedioxyphenyl)-2-cyclopropylethene;

1-[4-(3-ethylmethylamino-2-acetoxypropoxy)phenyl]-1-(4-hydroxyphenyl)-2-(3,4-methylenedioxyphenyl)-2-cyclopropylethene;

1-[4-(3-pyrrolidino-2-acetoxypropoxy)phenyl]-1-(4-hydroxyphenyl)-2-(3,4-methylenedioxyphenyl)-2-cyclopropylethene;

1-[4-(3-piperidino-2-acetoxypropoxy)phenyl]-1-(4-hydroxyphenyl)-2-(3,4-methylenedioxyphenyl)-2-cyclopropylethene;

1-[4-(3-cyclohexylmethylamino-2-acetoxypropoxy)-phenyl]-1-(4-hydroxyphenyl)-2-(3,4-methylenedioxyphenyl)-2-cyclopropylethene;

1-[4-(3-dimethylamino-2-acetoxypropoxy)phenyl]-1-(4-cyclopropanecarbonyloxyphenyl)-2-(3,4-methylenedioxyphenyl)-1-butene;

1-[4-(3-diethylamino-2-acetoxypropoxy)phenyl]-1-(4-cyclopropanecarbonyloxyphenyl)-2-(3,4-methylenedioxyphenyl)-1-butene;

1-[4-(3-ethylmethylamino-2-acetoxypropoxy)phenyl]-1-(4-cyclopropanecarbonyloxyphenyl)-2-(3,4-methylenedioxyphenyl)-1-butene;

1-[4-(3-pyrrolidino-2-acetoxypropoxy)phenyl]-1-(4-cyclopropanecarbonyloxyphenyl)-2-(3,4-methylenedioxyphenyl)-1-butene;

1-[4-(3-piperidino-2-acetoxypropoxy)phenyl]-1-(4-cyclopropanecarbonyloxyphenyl)-2-(3,4-methylenedioxyphenyl)-1-butene;

1-[4-(3-cyclohexylmethylamino-2-acetoxypropoxy)-phenyl]-1-(4-cyclopropanecarbonyloxyphenyl)-2-(3,4-methylenedioxyphenyl)-1-butene;

1-[4-(3-dimethylamino-2-cyclopropanecarbonyloxypropoxy)phenyl]-1-(4-hydroxyphenyl)-2-(3,4-methylenedioxyphenyl)-1-butene;

1-[4-(3-diethylamino-2-cyclopropanecarbonyloxypropoxy)phenyl]-1-(4-hydroxyphenyl)-2-(3,4-methylenedioxyphenyl)-1-butene;

1-[4-(3-ethylmethylamino-2-cyclopropanecarbonyloxypropoxy)phenyl]-1-(4-hydroxyphenyl)-2-(3,4-methylenedioxyphenyl)-1-butene;

1-[4-(3-pyrrolidino-2-cyclopropanecarbonyloxypropoxy)phenyl]-1-(4-hydroxyphenyl)-2-(3,4-methylenedioxyphenyl)-1-butene;
1-[4-(3-piperidino-2-cyclopropanecarbonyloxypropoxy)-phenyl]-1-(4-hydroxyphenyl)-2-(3,4-methylenedioxyphenyl)-1-butene;
1-[4-(3-cyclohexylmethylamino-2-cyclopropanecarbonyloxypropoxy)phenyl]-1-(4-hydroxyphenyl)-2-(3,4-methylenedioxyphenyl)-1-butene;
1-[4-(3-dimethylamino-2-butanoyloxypropoxy)phenyl]-1-(4-hydroxyphenyl)-2-(3,4-methylenedioxyphenyl)-1-butene;
1-[4-(3-diethylamino-2-butanoyloxypropoxy)phenyl]-1-(4-hydroxyphenyl)-2-(3,4-methylenedioxyphenyl)-1-butene;
1-[4-(3-ethylmethylamino-2-butanoyloxypropoxy)-phenyl]-1-(4-hydroxyphenyl)-2-(3,4-methylenedioxyphenyl)-1-butene;
1-[4-(3-pyrrolidino-2-butanoyloxypropoxy)phenyl]-1-(4-hydroxyphenyl)-2-(3,4-methylenedioxyphenyl)-1-butene;
1-[4-(3-piperidino-2-butanoyloxypropoxy)phenyl]-1-(4-hydroxyphenyl)-2-(3,4-methylenedioxyphenyl)-1-butene;
1-[4-(3-cyclohexylmethylamino-2-butanoyloxypropoxy)-phenyl]-1-(4-hydroxyphenyl)-2-(3,4-methylenedioxyphenyl)-1-butene;
1-[4-(1,3-bisdimethylamino-2-propoxy)phenyl]-1-(4-hydroxyphenyl)-2-(3,4-methylenedioxyphenyl)-1-butene;
1-[4-(1,3-bisdiethylamino-2-propoxy)phenyl]-1-(4-hydroxyphenyl)-2-(3,4-methylenedioxyphenyl)-1-butene;
1-[4-(1,3-bisethylmethylamino-2-propoxy)phenyl]-1-(4-hydroxyphenyl)-2-(3,4-methylenedioxyphenyl)-1-butene;
1-[4-(1,3-bispyrrolidino-2-propoxy)phenyl]-1-(4-hydroxyphenyl)-2-(3,4-methylenedioxyphenyl)-1-butene;
1-[4-(1,3-bispiperidino-2-propoxy)phenyl]-1-(4-hydroxyphenyl)-2-(3,4-methylenedioxyphenyl)-1-butene;
1-[4-(1,3-bisdimethylamino-2-propoxy)phenyl]-1-phenyl-2-(3,4-methylenedioxyphenyl)-1-butene;
1-[4-(1,3-bisdiethylamino-2-propoxy)phenyl]-1-phenyl-2-(3,4-methylenedioxyphenyl)-1-butene;
1-[4-(1,3-bisethylmethylamino-2-propoxy)phenyl]-1-phenyl-2-(3,4-methylenedioxyphenyl)-1-butene;
1-[4-(1,3-bispyrrolidino-2-propoxy)phenyl]-1-phenyl-2-(3,4-methylenedioxyphenyl)-1-butene;
1-[4-(1,3-bispiperidino-2-propoxy)phenyl]-1-phenyl-2-(3,4-methylenedioxyphenyl)-1-butene;
1-[4-(1,3-bisdimethylamino-2-propoxy)phenyl]-1-(4-methoxymethoxyphenyl)-2-(3,4-methylenedioxyphenyl)-1-butene;
1-[4-(1,3-bisdiethylamino-2-propoxy)phenyl]-1-(4-methoxymethoxyphenyl)-2-(3,4-methylenedioxyphenyl)-1-butene;
1-[4-(1,3-bisethylmethylamino-2-propoxy)phenyl]-1-(4-methoxymethoxyphenyl)-2-(3,4-methylenedioxyphenyl)-1-butene;
1-[4-(1,3-bispyrrolidino-2-propoxy)phenyl]-1-(4-methoxymethoxyphenyl)-2-(3,4-methylenedioxyphenyl)-1-butene;
1-[4-(1,3-bispiperidino-2-propoxy)phenyl]-1-(4-methoxymethoxyphenyl)-2-(3,4-methylenedioxyphenyl)-1-butene;
1-[4-(1,3-bisdimethylamino-2-propoxy)phenyl]-1-(4-acetoxyphenyl)-2-(3,4-methylenedioxyphenyl)-1-butene;
1-[4-(1,3-bisdiethylamino-2-propoxy)phenyl]-1-(4-acetoxyphenyl)-2-(3,4-methylenedioxyphenyl)-1-butene;
1-[4-(1,3-bisethylmethylamino-2-propoxy)phenyl]-1-(4-acetoxyphenyl)-2-(3,4-methylenedioxyphenyl)-1-butene;
1-[4-(1,3-bispyrrolidino-2-propoxy)phenyl]-1-(4-acetoxyphenyl)-2-(3,4-methylenedioxyphenyl)-1-butene;
1-[4-(1,3-bispiperidino-2-propoxy)phenyl]-1-(4-acetoxyphenyl)-2-(3,4-methylenedioxyphenyl)-1-butene;
1-[4-(1,3-bisdimethylamino-2-propoxy)phenyl]-1-(4-phosphonoxyphenyl)-2-(3,4-methylenedioxyphenyl)-1-butene;
1-[4-(1,3-bisdiethylamino-2-propoxy)phenyl]-1-(4-phosphonoxyphenyl)-2-(3,4-methylenedioxyphenyl)-1-butene;
1-[4-(1,3-bisethylmethylamino-2-propoxy)phenyl]-1-(4-phosphonoxyphenyl)-2-(3,4-methylenedioxyphenyl)-1-butene;
1-[4-(1,3-bispyrrolidino-2-propoxy)phenyl]-1-(4-phosphonoxyphenyl)-2-(3,4-methylenedioxyphenyl)-1-butene;
1-[4-(1,3-bispiperidino-2-propoxy)phenyl]-1-(4-phosphonoxyphenyl)-2-(3,4-methylenedioxyphenyl)-1-butene;

1-[4-(1,3-bisdimethylamino-2-propoxy)phenyl]-1-(4-benzoyloxyphenyl)-2-(3,4-methylenedioxyphenyl)-1-butene;

1-[4-(1,3-bisdiethylamino-2-propoxy)phenyl]-1-(4-benzoyloxyphenyl)-2-(3,4-methylenedioxyphenyl)-1-butene;

1-[4-(1,3-bisethylmethylamino-2-propoxy)phenyl]-1-(4-benzoyloxyphenyl)-2-(3,4-methylenedioxyphenyl)-1-butene;

1-[4-(1,3-bispyrrolidino-2-propoxy)phenyl]-1-(4-benzoyloxyphenyl)-2-(3,4-methylenedioxyphenyl)-1-butene;

1-[4-(1,3-bispiperidino-2-propoxy)phenyl]-1-(4-benzoyloxyphenyl)-2-(3,4-methylenedioxyphenyl)-1-butene;

1-[4-(1,3-bisdimethylamino-2-propoxy)phenyl]-1-(4-pivaloyloxymethoxyphenyl)-2-(3,4-methylenedioxyphenyl)-1-butene;

1-[4-(1,3-bisdiethylamino-2-propoxy)phenyl]-1-(4-pivaloyloxymethoxyphenyl)-2-(3,4-methylenedioxyphenyl)-1-butene;

1-[4-(1,3-bisethylmethylamino-2-propoxy)phenyl]-1-(4-pivaloyloxymethoxyphenyl)-2-(3,4-methylenedioxyphenyl)-1-butene;

1-[4-(1,3-bispyrrolidino-2-propoxy)phenyl]-1-(4-pivaloyloxymethoxyphenyl)-2-(3,4-methylenedioxyphenyl)-1-butene;

1-[4-(1,3-bispiperidino-2-propoxy)phenyl]-1-(4-pivaloyloxymethoxyphenyl)-2-(3,4-methylenedioxyphenyl)-1-butene;

1-[4-(1,3-bisdimethylamino-2-propoxy)phenyl]-1-(4-hydroxyphenyl)-2-(3,4-methylenedioxyphenyl)-2-cyclopropylethene;

1-[4-(1,3-bisdiethylamino-2-propoxy)phenyl]-1-(4-hydroxyphenyl)-2-(3,4-methylenedioxyphenyl)-2-cyclopropylethene;

1-[4-(1,3-bisethylmethylamino-2-propoxy)phenyl]-1-(4-hydroxyphenyl)-2-(3,4-methylenedioxyphenyl)-2-cyclopropylethene;

1-[4-(1,3-bispyrrolidino-2-propoxy)phenyl]-1-(4-hydroxyphenyl)-2-(3,4-methylenedioxyphenyl)-2-cyclopropylethene;

1-[4-(1,3-bispiperidino-2-propoxy)phenyl]-1-(4-hydroxyphenyl)-2-(3,4-methylenedioxyphenyl)-2-cyclopropylethene;

1-[4-(1,3-bisdimethylamino-2-propoxy)phenyl]-1-(4-cyclopropanecarbonyloxyphenyl)-2-(3,4-methylenedioxyphenyl)-1-butene;

1-[4-(1,3-bisdiethylamino-2-propoxy)phenyl]-1-(4-cyclopropanecarbonyloxyphenyl)-2-(3,4-methylenedioxyphenyl)-1-butene;

1-[4-(1,3-bisethylmethylamino-2-propoxy)phenyl]-1-(4-cyclopropanecarbonyloxyphenyl)-2-(3,4-methylenedioxyphenyl)-1-butene;

1-[4-(1,3-bispyrrolidino-2-propoxy)phenyl]-1-(4-cyclopropanecarbonyloxyphenyl)-2-(3,4-methylenedioxyphenyl)-1-butene;

1-[4-(1,3-bispiperidino-2-propoxy)phenyl]-1-(4-cyclopropanecarbonyloxyphenyl)-2-(3,4-methylenedioxyphenyl)-1-butene;

1-[4-(1,3-bisdimethylamino-2-propoxy)phenyl]-1,2-diphenyl-1-butene;

1-[4-(1,3-bisdiethylamino-2-propoxy)phenyl]-1,2-diphenyl-1-butene;

1-[4-(1,3-bisethylmethylamino-2-propoxy)phenyl]-1,2-diphenyl-1-butene;

1-[4-(1,3-bispyrrolidino-2-propoxy)phenyl]-1,2-diphenyl-1-butene;

1-[4-(1,3-bispiperidino-2-propoxy)phenyl]-1,2-diphenyl-1-butene;

1-[4-(1,3-bisdimethylamino-2-propoxy)phenyl]-1-(4-hydroxyphenyl)-2-phenyl-1-butene;

1-[4-(1,3-bisdiethylamino-2-propoxy)phenyl]-1-(4-hydroxyphenyl)-2-phenyl-1-butene;

1-[4-(1,3-bisethylmethylamino-2-propoxy)phenyl]-1-(4-hydroxyphenyl)-2-phenyl-1-butene;

1-[4-(1,3-bispyrrolidino-2-propoxy)phenyl]-1-(4-hydroxyphenyl)-2-phenyl-1-butene;

1-[4-(1,3-bispiperidino-2-propoxy)phenyl]-1-(4-hydroxyphenyl)-2-phenyl-1-butene;

1-[4-(2-methylaminoethoxy)phenyl]-1-(4-hydroxyphenyl)-2-(3,4-methylenedioxyphenyl)-1-butene;

1-[4-(2-ethylaminoethoxy)phenyl]-1-(4-hydroxyphenyl)-2-(3,4-methylenedioxyphenyl)-1-butene;

1-[4-(2-isopropylaminoethoxy)phenyl]-1-(4-hydroxyphenyl)-2-(3,4-methylenedioxyphenyl)-1-butene;

1-[4-(2-cyclohexylaminoethoxy)phenyl]-1-(4-hydroxyphenyl)-2-(3,4-methylenedioxyphenyl)-1-butene;

1-[4-(2-dimethylaminoethoxy)phenyl]-1-(4-hydroxyphenyl)-2-(3,4-methylenedioxyphenyl)-1-butene;

1-[4-(2-diethylaminoethoxy)phenyl]-1-(4-hydroxyphenyl)-2-(3,4-methylenedioxyphenyl)-1-butene;

1-[4-(2-methylethylaminoethoxy)phenyl]-1-(4-hydroxyphenyl)-2-(3,4-methylenedioxyphenyl)-1-butene;

1-[4-(2-cyclohexylmethylaminoethoxy)phenyl]-1-(4-hydroxyphenyl)-2-(3,4-methylenedioxyphenyl)-1-butene;
1-[4-(2-pyrrolidinoethoxy)phenyl]-1-(4-hydroxyphenyl)-2-(3,4-methylenedioxyphenyl)-1-butene;
1-[4-(2-methylaminoethoxy)phenyl]-1-phenyl-2-(3,4-methylenedioxyphenyl)-1-butene;
1-[4-(2-ethylaminoethoxy)phenyl]-1-phenyl-2-(3,4-methylenedioxyphenyl)-1-butene;
1-[4-(2-isopropylaminoethoxy)phenyl]-1-phenyl-2-(3,4-methylenedioxyphenyl)-1-butene;
1-[4-(2-cyclohexylaminoethoxy)phenyl]-1-phenyl-2-(3,4-methylenedioxyphenyl)-1-butene;
1-[4-(2-dimethylaminoethoxy)phenyl]-1-phenyl-2-(3,4-methylenedioxyphenyl)-1-butene;
1-[4-(2-diethylaminoethoxy)phenyl]-1-phenyl-2-(3,4-methylenedioxyphenyl)-1-butene;
1-[4-(2-methylethylaminoethoxy)phenyl]-1-phenyl-2-(3,4-methylenedioxyphenyl)-1-butene;
1-[4-(2-cyclohexylmethylaminoethoxy)phenyl]-1-phenyl-2-(3,4-methylenedioxyphenyl)-1-butene;
1-[4-(2-pyrrolidinoethoxy)phenyl]-1-phenyl-2-(3,4-methylenedioxyphenyl)-1-butene;
1-[4-(2-methylaminoethoxy)phenyl]-1-(4-methoxymethoxyphenyl)-2-(3,4-methylenedioxyphenyl)-1-butene;
1-[4-(2-ethylaminoethoxy)phenyl]-1-(4-methoxymethoxyphenyl)-2-(3,4-methylenedioxyphenyl)-1-butene;
1-[4-(2-isopropylaminoethoxy)phenyl]-1-(4-methoxymethoxyphenyl)-2-(3,4-methylenedioxyphenyl)-1-butene;
1-[4-(2-cyclohexylaminoethoxy)phenyl]-1-(4-methoxymethoxyphenyl)-2-(3,4-methylenedioxyphenyl)-1-butene;
1-[4-(2-dimethylaminoethoxy)phenyl]-1-(4-methoxymethoxyphenyl)-2-(3,4-methylenedioxyphenyl)-1-butene;
1-[4-(2-diethylaminoethoxy)phenyl]-1-(4-methoxymethoxyphenyl)-2-(3,4-methylenedioxyphenyl)-1-butene;
1-[4-(2-methylethylaminoethoxy)phenyl]-1-(4-methoxymethoxyphenyl)-2-(3,4-methylenedioxyphenyl)-1-butene;
1-[4-(2-cyclohexylaminoethoxy)phenyl]-1-(4-methoxymethoxyphenyl)-2-(3,4-methylenedioxyphenyl)-1-butene;
1-[4-(2-pyrrolidinoethoxy)phenyl]-1-(4-methoxymethoxyphenyl)-2-(3,4-methylenedioxyphenyl)-1-butene;
1-[4-(2-methylaminoethoxy)phenyl]-1-(4-acetoxyphenyl)-2-(3,4-methylenedioxyphenyl)-1-butene;
1-[4-(2-ethylaminoethoxy)phenyl]-1-(4-acetoxyphenyl)-2-(3,4-methylenedioxyphenyl)-1-butene;
1-[4-(2-isopropylaminoethoxy)phenyl]-1-(4-acetoxyphenyl)-2-(3,4-methylenedioxyphenyl)-1-butene;
1-[4-(2-cyclohexylaminoethoxy)phenyl]-1-(4-acetoxyphenyl)-2-(3,4-methylenedioxyphenyl)-1-butene;
1-[4-(2-dimethylaminoethoxy)phenyl]-1-(4-acetoxyphenyl)-2-(3,4-methylenedioxyphenyl)-1-butene;
1-[4-(2-diethylaminoethoxy)phenyl]-1-(4-acetoxyphenyl)-2-(3,4-methylenedioxyphenyl)-1-butene;
1-[4-(2-methylethylaminoethoxy)phenyl]-1-(4-acetoxyphenyl)-2-(3,4-methylenedioxyphenyl)-1-butene;
1-[4-(2-cyclohexylmethylaminoethoxy)phenyl]-1-(4-acetoxyphenyl)-2-(3,4-methylenedioxyphenyl)-1-butene;
1-[4-(2-pyrrolidinoethoxy)phenyl]-1-(4-acetoxyphenyl)-2-(3,4-methylenedioxyphenyl)-1-butene;
1-[4-(2-methylaminoethoxy)phenyl]-1-(4-dihydroxyphosphinooxyphenyl)-2-(3,4-methylenedioxyphenyl)-1-butene;
1-[4-(2-ethylaminoethoxy)phenyl]-1-(4-dihydroxyphosphinooxyphenyl)-2-(3,4-methylenedioxyphenyl)-1-butene;
1-[4-(2-isopropylamineaminoethoxy)phenyl]-1-(4-dihydroxyphosphinooxyphenyl)-2-(3,4-methylenedioxyphenyl)-1-butene;
1-[4-(2-cyclohexylaminoethoxy)phenyl]-1-(4-dihydroxyphosphinooxyphenyl)-2-(3,4-methylenedioxyphenyl)-1-butene;
1-[4-(2-dimethylaminoethoxy)phenyl]-1-(4-dihydroxyphosphinooxyphenyl)-2-(3,4-methylenedioxyphenyl)-1-butene;
1-[4-(2-diethylaminoethoxy)phenyl]-1-(4-dihydroxyphosphinooxyphenyl)-2-(3,4-methylenedioxyphenyl)-1-butene;
1-[4-(2-methylethylaminoethoxy)phenyl]-1-(4-dihydroxyphosphinooxyphenyl)-2-(3,4-methylenedioxyphenyl)-1-butene;
1-[4-(2-cyclohexylmethylaminoethoxy)phenyl]-1-(4-dihydroxyphosphinooxyphenyl)-2-(3,4-methylenedioxyphenyl)-1-butene;
1-[4-(2-pyrrolidinoethoxy)phenyl]-1-(4-dihydroxyphosphinooxyphenyl)-2-(3,4-methylenedioxyphenyl)-1-butene;
1-[4-(2-methylaminoethoxy)phenyl]-1-(4-benzoyloxyphenyl)-2-(3,4-methylenedioxyphenyl)-1-butene;
1-[4-(2-ethylaminoethoxy)phenyl]-1-(4-benzoyloxyphenyl)-2-(3,4-methylenedioxyphenyl)-1-butene;

1-[4-(2-isopropylaminoethoxy)phenyl]-1-(4-benzoyloxyphenyl)-2-(3,4-methylenedioxyphenyl)-1-butene;

1-[4-(2-cyclohexylaminoethoxy)phenyl]-1-(4-benzoyloxyphenyl)-2-(3,4-methylenedioxyphenyl)-1-butene;

1-[4-(2-dimethylaminoethoxy)phenyl]-1-(4-benzoyloxyphenyl)-2-(3,4-methylenedioxyphenyl)-1-butene;

1-[4-(2-diethylaminoethoxy)phenyl]-1-(4-benzoyloxyphenyl)-2-(3,4-methylenedioxyphenyl)-1-butene;

1-[4-(2-methylethylaminoethoxy)phenyl]-1-(4-benzoyloxyphenyl)-2-(3,4-methylenedioxyphenyl)-1-butene;

1-[4-(2-cyclohexylmethylaminoethoxy)phenyl]-1-(4-benzoyloxyphenyl)-2-(3,4-methylenedioxyphenyl)-1-butene;

1-[4-(2-pyrrolidinoethoxy)phenyl]-1-(4-benzoyloxyphenyl)-2-(3,4-methylenedioxyphenyl)-1-butene;

1-[4-(2-methylaminoethoxy)phenyl]-1-(4-pivaloyloxymethoxyphenyl)-2-(3,4-methylenedioxyphenyl)-1-butene;

1-[4-(2-ethylaminoethoxy)phenyl]-1-(4-pivaloyloxymethoxyphenyl)-2-(3,4-methylenedioxyphenyl)-1-butene;

1-[4-(2-isopropylaminoethoxy)phenyl]-1-(4-pivaloyloxymethoxyphenyl)-2-(3,4-methylenedioxyphenyl)-1-butene;

1-[4-(2-cyclohexylaminoethoxy)phenyl]-1-(4-pivaloyloxymethoxyphenyl)-2-(3,4-methylenedioxyphenyl)-1-butene;

1-[4-(2-dimethylaminoethoxy)phenyl]-1-(4-pivaloyloxymethoxyphenyl)-2-(3,4-methylenedioxyphenyl)-1-butene;

1-[4-(2-diethylaminoethoxy)phenyl]-1-(4-pivaloyloxymethoxyphenyl)-2-(3,4-methylenedioxyphenyl)-1-butene;

1-[4-(2-methylethylaminoethoxy)phenyl]-1-(4-pivaloyloxymethoxyphenyl)-2-(3,4-methylenedioxyphenyl)-1-butene;

1-[4-(2-cyclohexylmethylaminoethoxy)phenyl]-1-(4-pivaloyloxymethoxyphenyl)-2-(3,4-methylenedioxyphenyl)-1-butene;

1-[4-(2-pyrrolidinoethoxy)phenyl]-1-(4-pivaloyloxymethoxyphenyl)-2-(3,4-methylenedioxyphenyl)-1-butene;

1-[4-(2-methylaminoethoxy)phenyl]-1-(4-hydroxyphenyl)-2-(3,4-methylenedioxyphenyl)-2-cyclopropylethene;

1-[4-(2-ethylaminoethoxy)phenyl]-1-(4-hydroxyphenyl)-2-(3,4-methylenedioxyphenyl)-2-cyclopropylethene;

1-[4-(2-isopropylaminoethoxy)phenyl]-1-(4-hydroxyphenyl)-2-(3,4-methylenedioxyphenyl)-2-cyclopropylethene;

1-[4-(2-cyclohexylaminoethoxy)phenyl]-1-(4-hydroxyphenyl)-2-(3,4-methylenedioxyphenyl)-2-cyclopropylethene;

1-[4-(2-dimethylaminoethoxy)phenyl]-1-(4-hydroxyphenyl)-2-(3,4-methylenedioxyphenyl)-2-cyclopropylethene;

1-[4-(2-diethylaminoethoxy)phenyl]-1-(4-hydroxyphenyl)-2-(3,4-methylenedioxyphenyl)-2-cyclopropylethene;

1-[4-(2-methylethylaminoethoxy)phenyl]-1-(4-hydroxyphenyl)-2-(3,4-methylenedioxyphenyl)-2-cyclopropylethene;

1-[4-(2-cyclohexylmethylaminoethoxy)phenyl]-1-(4-hydroxyphenyl)-2-(3,4-methylenedioxyphenyl)-2-cyclopropylethene;

1-[4-(2-pyrrolidinoethoxy)phenyl]-1-(4-hydroxyphenyl)-2-(3,4-methylenedioxyphenyl)-2-cyclopropylethene;

1-[4-(2-methylaminoethoxy)phenyl]-1-(4-cyclopropanecarbonyloxyphenyl)-2-(3,4-methylenedioxyphenyl)-1-butene;

1-[4-(2-ethylaminoethoxy)phenyl]-1-(4-cyclopropanecarbonyloxyphenyl)-2-(3,4-methylenedioxyphenyl)-1-butene;

1-[4-(2-isopropylaminoethoxy)phenyl]-1-(4-cyclopropanecarbonyloxyphenyl)-2-(3,4-methylenedioxyphenyl)-1-butene;

1-[4-(2-cyclohexylaminoethoxy)phenyl]-1-(4-cyclopropanecarbonyloxyphenyl)-2-(3,4-methylenedioxyphenyl)-1-butene;

1-[4-(2-dimethylaminoethoxy)phenyl]-1-(4-cyclopropanecarbonyloxyphenyl)-2-(3,4-methylenedioxyphenyl)-1-butene;

1-[4-(2-diethylaminoethoxy)phenyl]-1-(4-cyclopropanecarbonyloxyphenyl)-2-(3,4-methylenedioxyphenyl)-1-butene;

1-[4-(2-methylethylaminoethoxy)phenyl]-1-(4-cyclopropanecarbonyloxyphenyl)-2-(3,4-methylenedioxyphenyl)-1-butene;

1-[4-(2-cyclohexylmethylaminoethoxy)phenyl]-1-(4-cyclopropanecarbonyloxyphenyl)-2-(3,4-methylenedioxyphenyl)-1-butene;

1-[4-(2-pyrrolidinoethoxy)phenyl]-1-(4-cyclopropanecarbonyloxyphenyl)-2-(3,4-methylenedioxyphenyl)-1-butene;

1-[4-(dimethylaminoethoxy)phenyl]-1-phenyl-1-[1-(1,2,3,4-tetrahydronaphthylidene)]methane;

1-[4-(dimethylaminoethoxy)phenyl]-1-phenyl-1-[1-(4-methyl-1,2,3,4-tetrahydronaphthylidene)]methane;

1-[4-(dimethylaminoethoxy)phenyl]-1-phenyl-1-(1-indanylidene)methane;

1-[4-(dimethylaminoethoxy)phenyl]-1-phenyl-1-(5-benzocycloheptylidene)methane;

1-[4-(dimethylaminoethoxy)phenyl]-1-phenyl-1-(4-chromanylidene)methane;

1-[4-(dimethylaminoethoxy)phenyl]-1-phenyl-1-(4-thiochromanylidene)methane;

1-[4-(dimethylaminoethoxy)phenyl]-1-phenyl-1-(4-thiochromanylidene-1-oxido)methane;

1-[4-(dimethylaminoethoxy)phenyl]-1-phenyl-1-(4-thiochromanylidene-1,1-dioxido)methane;

1-[4-(dimethylaminoethoxy)phenyl]-1-phenyl-1-[2-(1,2,3,4-tetrahydronaphthylidene)]methane;

1-[4-(dimethylaminoethoxy)phenyl]-1-phenyl-1-[2-(1-methyl-1,2,3,4-tetrahydronaphthylidene)]methane;

1-[4-(dimethylaminoethoxy)phenyl]-1-phenyl-1-[1-(5-methoxy-1,2,3,4-tetrahydronaphthylidene)]methane;

1-[4-(dimethylaminoethoxy)phenyl]-1-phenyl-1-[4-(1,2,3,4-tetrahydroquinolinylidene)]methane;

1-[4-(diethylaminoethoxy)phenyl]-1-phenyl-1-[1-(1,2,3,4-tetrahydronaphthylidene)]methane;

1-[4-(methylethylaminoethoxy)phenyl]-1-phenyl-1-[1-(1,2,3,4-tetrahydronaphthylidene)]methane;

1-[4-(pyrrolidinoethoxy)phenyl]-1-phenyl-1-[1-(1,2,3,4-tetrahydronaphthylidene)]methane;

1-[4-(cyclohexylmethylaminoethoxy)phenyl]-1-phenyl-1-[1-(1,2,3,4-tetrahydronaphthylidene)]methane;

1-[4-(dimethylaminoethoxy)phenyl]-1-(4-hydroxyphenyl)-1-[1-(1,2,3,4-tetrahydronaphthylidene)]methane;

1-[4-(dimethylaminoethoxy)phenyl]-1-(4-hydroxyphenyl)-1-[1-(4-methyl-1,2,3,4-tetrahydronaphthylidene)-]-methane;

1-[4-(dimethylaminoethoxy)phenyl]-1-(4-hydroxyphenyl)-1-(1-indanylidene)methane;

1-[4-(dimethylaminoethoxy)phenyl]-1-(4-hydroxyphenyl)-1-(5-benzocycloheptylidene)methane;

1-[4-(dimethylaminoethoxy)phenyl]-1-(4-hydroxyphenyl)-1-(4-chromanylidene)methane;

1-[4-(dimethylaminoethoxy)phenyl]-1-(4-hydroxyphenyl)-1-(4-chromanylidene)methane;

1-[4-(dimethylaminoethoxy)phenyl]-1-(4-hydroxyphenyl)-1-(4-thiochromanylidene-1-oxido)methane;

1-[4-(dimethylaminoethoxy)phenyl]-1-(4-hydroxyphenyl)-1-(4-thiochromanylidene-1,1-dioxido)methane;

1-[4-(dimethylaminoethoxy)phenyl]-1-(4-hydroxyphenyl)-1-[2-(1,2,3,4-tetrahydronaphthylidene)]methane;

1-[4-(dimethylaminoethoxy)phenyl]-1-(4-hydroxyphenyl)-1-[2-(1-methyl-1,2,3,4-tetrahydronaphthylidene)-]-methane;

1-[4-(dimethylaminoethoxy)phenyl]-1-(4-hydroxyphenyl)-1-[1-(5-methoxy-1,2,3,4-tetrahydronaphthylidene)]-methane;

1-[4-(dimethylaminoethoxy)phenyl]-1-(4-hydroxyphenyl)-1-[4-(1,2,3,4-tetrahydroquinolinylidene)]-methane;

1-[4-(diethylaminoethoxy)phenyl]-1-(4-hydroxyphenyl)-1-[1-(1,2,3,4-tetrahydronaphthylidene)]methane;

1-[4-(methylethylaminoethoxy)phenyl]-1-(4-hydroxyphenyl)-1-[1-(1,2,3,4-tetrahydronaphthylidene)]-methane;

1-[4-(pyrrolidinoethoxy)phenyl]-1-(4-hydroxyphenyl)-1-[1-(1,2,3,4-tetrahydronaphthylidene)]methane;

1-[4-(cyclohexylmethylaminoethoxy)phenyl]-1-(4-hydroxyphenyl)-1-[1-(1,2,3,4-tetrahydronaphthylidene)]-methane;

1-[4-(dimethylaminoethoxy)phenyl]-1-(4-methoxymethoxyphenyl)-1-[1-(1,2,3,4-tetrahydronaphthylidene)]-methane;

1-[4-(dimethylaminoethoxy)phenyl]-1-(4-methoxymethoxyphenyl)-1-[1-(4-methyl-1,2,3,4-tetrahydronaphthylidene)]methane;

1-[4-(dimethylaminoethoxy)phenyl]-1-(4-methoxymethoxyphenyl)-1-(1-indanylidene)methane;

1-[4-(dimethylaminoethoxy)phenyl]-1-(4-methoxymethoxyphenyl)-1-(5-benzocycloheptylidene)methane;

1-[4-(dimethylaminoethoxy)phenyl]-1-(4-methoxymethoxyphenyl)-1-(4-chromanylidene)methane;

1-[4-(dimethylaminoethoxy)phenyl]-1-(4-methoxymethoxyphenyl)-1-(4-thiochromanylidene)methane;

1-[4-(dimethylaminoethoxy)phenyl]-1-(4-methoxymethoxyphenyl)-1-(4-thiochromanylidene-1-oxido)-methane;

1-[4-(dimethylaminoethoxy)phenyl]-1-(4-methoxymethoxyphenyl)-1-(4-thiochromanylidene-1,1-dioxido)-methane;

1-[4-(dimethylaminoethoxy)phenyl]-1-(4-methoxymethoxyphenyl)-1-[2-(1,2,3,4-tetrahydronaphthylidene)]-methane;

1-[4-(dimethylaminoethoxy)phenyl]-1-(4-methoxymethoxyphenyl)-1-[2-(1-methyl-1,2,3,4-tetrahydronaphthylidene)]methane;

1-[4-(dimethylaminoethoxy)phenyl]-1-(4-methoxymethoxyphenyl)-1-[1-(5-methoxy-1,2,3,4-tetrahydronaphthylidene)]methane;

1-[4-(dimethylaminoethoxy)phenyl]-1-(4-methoxymethoxyphenyl)-1-[4-(1,2,3,4-tetrahydroquinolinylidene)]-methane;

1-[4-(dimethylaminoethoxy)phenyl]-1-(4-methoxymethoxyphenyl)-1-[1-(1,2,3,4-tetrahydronaphthylidene)]-methane;

1-[4-(methylethylaminoethoxy)phenyl]-1-(4-methoxymethoxyphenyl)-1-[1-(1,2,3,4-tetrahydronaphthylidene)]-methane;

1-[4-(pyrrolidinoethoxy)phenyl]-1-(4-methoxymethoxyphenyl)-1-[1-(1,2,3,4-tetrahydronaphthylidene)]-methane;

1-[4-(cyclohexylmethylaminoethoxy)phenyl]-1-(4-methoxymethoxyphenyl)-1-[1-(1,2,3,4-tetrahydronaphthylidene)]methane;

1-[4-(dimethylaminoethoxy)phenyl]-1-(4-acetoxyphenyl)-1-[1-(1,2,3,4-tetrahydronaphthylidene)]methane;

1-[4-(dimethylaminoethoxy)phenyl]-1-(4-acetoxyphenyl)-1-[1-(4-methyl-1,2,3,4-tetrahydronaphthylidene)]-methane;

1-[4-(dimethylaminoethoxy)phenyl]-1-(4-acetoxyphenyl)-1-(1-indanylidene)methane;

1-[4-(dimethylaminoethoxy)phenyl]-1-(4-acetoxyphenyl)-1-(5-benzocycloheptylidene)methane;

1-[4-(dimethylaminoethoxy)phenyl]-1-(4-acetoxyphenyl)-1-(4-chromanylidene)methane;

1-[4-(dimethylaminoethoxy)phenyl]-1-(4-acetoxyphenyl)-1-(4-thiochromanylidene)methane;

1-[4-(dimethylaminoethoxy)phenyl]-1-(4-acetoxyphenyl)-1-(4-thiochromanylidene-1-oxido)methane;

1-[4-(dimethylaminoethoxy)phenyl]-1-(4-acetoxyphenyl)-1-(4-thiochromanylidene-1,1-dioxido)methane;

1-[4-(dimethylaminoethoxy)phenyl]-1-(4-acetoxyphenyl)-1-[2-(1,2,3,4-tetrahydronaphthylidene)]methane;

1-[4-(dimethylaminoethoxy)phenyl]-1-(4-acetoxyphenyl)-1-[2-(1-methyl-1,2,3,4-tetrahydronaphthylidene)]-methane;

1-[4-(dimethylaminoethoxy)phenyl]-1-(4-acetoxyphenyl)-1-[1-(5-methoxy-1,2,3,4-tetrahydronaphthylidene)]-methane;

1-[4-(dimethylaminoethoxy)phenyl]-1-(4-acetoxyphenyl)-1-[4-(1,2,3,4-tetrahydroquinolinylidene)]methane;

1-[4-(diethylaminoethoxy)phenyl]-1-(4-acetoxyphenyl)-1-[1-(1,2,3,4-tetrahydronaphthylidene)]methane;

1-[4-(methylethylaminoethoxy)phenyl]-1-(4-acetoxyphenyl)-1-[1-(1,2,3,4-tetrahydronaphthylidene)]-methane;

1-[4-(pyrrolidinoethoxy)phenyl]-1-(4-acetoxyphenyl)-1-[1-(1,2,3,4-tetrahydronaphthylidene)]methane;

1-[4-(cyclohexylmethylaminoethoxy)phenyl]-1-(4-acetoxyphenyl)-1-[1-(1,2,3,4-tetrahydronaphthylidene)]-methane;

1-[4-(dimethylaminoethoxy)phenyl]-1-(4-dihydroxyphosphinooxyphenyl)-1-[1-(1,2,3,4-tetrahydronaphthylidene)]-methane;

1-[4-(dimethylaminoethoxy)phenyl]-1-(4-dihydroxyphosphinooxyphenyl)-1-[1-(4-methyl-1,2,3,4-tetrahydronaphthylidene)]methane;

1-[4-(dimethylaminoethoxy)phenyl]-1-(4-dihydroxyphosphinooxyphenyl)-1-(1-indanylidene)methane;

1-[4-(dimethylaminoethoxy)phenyl]-1-(4-dihydroxyphosphinooxyphenyl)-1-(5-benzocycloheptylidene)-methane;

1-[4-(dimethylaminoethoxy)phenyl]-1-(4-dihydroxyphosphinooxyphenyl)-1-(4-chromanylidene)methane;

1-[4-(dimethylaminoethoxy)phenyl]-1-(4-dihydroxyphosphinooxyphenyl)-1-(4-thiochromanylidene)-methane;

1-[4-(dimethylaminoethoxy)phenyl]-1-(4-dihydroxyphosphinooxyphenyl)-1-(4-thiochromanylidene-1-oxido)methane;

1-[4-(dimethylaminoethoxy)phenyl]-1-(4-dihydroxyphosphinooxyphenyl)-1-(4-thiochromanylidene-1,1-dioxido)-methane;

1-[4-(dimethylaminoethoxy)phenyl]-1-(4-dihydroxyphosphinooxyphenyl)-1-[2-(1,2,3,4-tetrahydronaphthylidene)]-methane;

1-[4-(dimethylaminoethoxy)phenyl]-1-(4-dihydroxyphosphinooxyphenyl)-1-[2-(1-methyl-1,2,3,4-tetrahydronaphthylidene)]methane;

1-[4-(dimethylaminoethoxy)phenyl]-1-(4-dihydroxyphosphinooxyphenyl)-1-[1-(5-methoxy-1,2,3,4-tetrahydronaphthylidene)]methane;

1-[4-(dimethylaminoethoxy)phenyl]-1-(4-dihydroxyphosphinooxyphenyl)-1-[4-(1,2,3,4-tetrahydroquinolinylidene)]methane;

1-[4-(diethylaminoethoxy)phenyl]-1-(4-dihydroxyphosphinooxyphenyl)-1-[1-(1,2,3,4-tetrahydronaphthylidene)]-methane;

1-[4-(methylethylaminoethoxy)phenyl]-1-(4-dihydroxyphosphinooxyphenyl)-1-[1-(1,2,3,4-tetrahydronaphthylidene)]-methane;

1-[4-(pyrrolidinoethoxy)phenyl]-1-(4-dihydroxyphosphinooxyphenyl)-1-[1-(1,2,3,4-tetrahydronaphthylidene)]-methane;

1-[4-(cyclohexylmethylaminoethoxy)phenyl]-1-(4-dihydroxyphosphinooxyphenyl)-1-[1-(1,2,3,4-tetrahydronaphthylidene)]methane;

1-[4-(dimethylaminoethoxy)phenyl]-1-(4-benzoyloxyphenyl)-1-[1-(1,2,3,4-tetrahydronaphthylidene)]-methane;

1-[4-(dimethylaminoethoxy)phenyl]-1-(4-benzoyloxyphenyl)-1-[1-(4-methyl-1,2,3,4-tetrahydronaphthylidene)]-methane;

1-[4-(dimethylaminoethoxy)phenyl]-1-(4-benzoyloxyphenyl)-1-(1-indanylidene)methane;

1-[4-(dimethylaminoethoxy)phenyl]-1-(4-benzoyloxyphenyl)-1-(5-benzocycloheptylidene)methane;

1-[4-(dimethylaminoethoxy)phenyl]-1-(4-benzoyloxyphenyl)-1-(4-chromanylidene)methane;

1-[4-(dimethylaminoethoxy)phenyl]-1-(4-benzoyloxyphenyl)-1-(4-thiochromanylidene)methane;

1-[4-(dimethylaminoethoxy)phenyl]-1-(4-benzoyloxyphenyl)-1-(4-thiochromanylidene-1-oxido)methane;

1-[4-(dimethylaminoethoxy)phenyl]-1-(4-benzoyloxyphenyl)-1-(4-thiochromanylidene-1,1-dioxido)-methane;

1-[4-(dimethylaminoethoxy)phenyl]-1-(4-benzoyloxyphenyl)-1-[2-(1,2,3,4-tetrahydronaphthylidene)]-methane;

1-[4-(dimethylaminoethoxy)phenyl]-1-(4-benzoyloxyphenyl)-1-[2-(1-methyl-1,2,3,4-tetrahydronaphthylidene)]-methane;

1-[4-(dimethylaminoethoxy)phenyl]-1-(4-benzoyloxyphenyl)-1-[1-(5-methoxy-1,2,3,4-tetrahydronaphthylidene)]-methane;

1-[4-(dimethylaminoethoxy)phenyl]-1-(4-benzoyloxyphenyl)-1-[4-(1,2,3,4-tetrahydroquinolinylidene)]-methane;

1-[4-(diethylaminoethoxy)phenyl]-1-(4-benzoyloxyphenyl)-1-[1-(1,2,3,4-tetrahydronaphthylidene)]-methane;

1-[4-(methylethylaminoethoxy)phenyl]-1-(4-benzoyloxyphenyl)-1-[1-(1,2,3,4-tetrahydronaphthylidene)]-methane;

1-[4-(pyrrolidinoethoxy)phenyl]-1-(4-benzoyloxyphenyl)-1-[1-(1,2,3,4-tetrahydronaphthylidene)]methane;

1-[4-(cyclohexylmethylaminoethoxy)phenyl]-1-(4-benzoyloxyphenyl)-1-[1-(1,2,3,4-tetrahydronaphthylidene)]-methane;

1-[4-(dimethylaminoethoxy)phenyl]-1-(4-pivaloyloxymethoxyphenyl)-1-[1-(1,2,3,4-tetrahydronaphthylidene)]-methane;

1-[4-(dimethylaminoethoxy)phenyl]-1-(4-pivaloyloxymethoxyphenyl)-1-[1-(4-methyl-1,2,3,4-tetrahydronaphthylidene)]methane;

1-[4-(dimethylaminoethoxy)phenyl]-1-(4-pivaloyloxymethoxyphenyl)-1-(1-indanylidene)methane;

1-[4-(dimethylaminoethoxy)phenyl]-1-(4-pivaloyloxymethoxyphenyl)-1-(5-benzocycloheptylidene)-methane;

1-[4-(dimethylaminoethoxy)phenyl]-1-(4-pivaloyloxymethoxyphenyl)-1-(4-chromanylidene)methane;

1-[4-(dimethylaminoethoxy)phenyl]-1-(4-pivaloyloxymethoxyphenyl)-1-(4-thiochromanylidene)methane;

1-[4-(dimethylaminoethoxy)phenyl]-1-(4-pivaloyloxymethoxyphenyl)-1-(4-thiochromanylidene-1-oxido)-methane;

1-[4-(dimethylaminoethoxy)phenyl]-1-(4-pivaloyloxymethoxyphenyl)-1-(4-thiochromanylidene-1,1-dioxido)methane;

1-[4-(dimethylaminoethoxy)phenyl]-1-(4-pivaloyloxymethoxyphenyl)-1-[2-(1,2,3,4-tetrahydronaphthylidene)]-methane;

1-[4-(dimethylaminoethoxy)phenyl]-1-(4-pivaloyloxymethoxyphenyl)-1-[2-(1-methyl-1,2,3,4-tetrahydronaphthylidene)]methane;

1-[4-(dimethylaminoethoxy)phenyl]-1-(4-pivaloyloxymethoxyphenyl)-1-[1-(5-methoxy-1,2,3,4-tetrahydronaphthylidene)]methane;

1-[4-(dimethylaminoethoxy)phenyl]-1-(4-pivaloyloxymethoxyphenyl)-1-[4-(1,2,3,4-tetrahydroquinolinylidene)]-methane;

1-[4-(diethylaminoethoxy)phenyl]-1-(4-pivaloyloxymethoxyphenyl)-1-[1-(1,2,3,4-tetrahydronaphthylidene)-]-methane;

1-[4-(methylethylaminoethoxy)phenyl]-1-(4-pivaloyloxymethoxyphenyl)-1-[1-(1,2,3,4-tetrahydronaphthylidene)]methane;

1-[4-(pyrrolidinoethoxy)phenyl]-1-(4-pivaloyloxymethoxyphenyl)-1-[1-(1,2,3,4-tetrahydronaphthylidene)]-methane;

1-[4-(cyclohexylmethylaminoethoxy)phenyl]-1-(4-pivaloyloxymethoxyphenyl)-1-[1-(1,2,3,4-tetrahydronaphthylidene)]methane;

1-[4-(dimethylaminoethoxy)phenyl]-1-(4-cyclopropanecarbonyloxyphenyl)-1-[1-(1,2,3,4-tetrahydronaphthylidene)]-methane;

1-[4-(dimethylaminoethoxy)phenyl]-1-(4-cyclopropanecarbonyloxyphenyl)-1-[1-(4-methyl-1,2,3,4-tetrahydronaphthylidene)]methane;

1-[4-(dimethylaminoethoxy)phenyl]-1-(4-cyclopropanecarbonyloxyphenyl)-1-(1-indanylidene)methane;

1-[4-(dimethylaminoethoxy)phenyl]-1-(4-cyclopropanecarbonyloxyphenyl)-1-(5-benzocycloheptylidene)-methane;

1-[4-(dimethylaminoethoxy)phenyl]-1-(4-cyclopropanecarbonyloxyphenyl)-1-(4-chromanylidene)methane;

1-[4-(dimethylaminoethoxy)phenyl]-1-(4-cyclopropanecarbonyloxyphenyl)-1-(4-thiochromanylidene)-methane;

1-[4-(dimethylaminoethoxy)phenyl]-1-(4-cyclopropanecarbonyloxyphenyl)-1-(4-thiochromanylidene-1-oxido)methane;

1-[4-(dimethylaminoethoxy)phenyl]-1-(4-cyclopropanecarbonyloxyphenyl)-1-(4-thiochromanylidene-1,1-dioxido)-methane;

1-[4-(dimethylaminoethoxy)phenyl]-1-(4-cyclopropanecarbonyloxyphenyl)-1-[2-(1,2,3,4-tetrahydronaphthylidene)]-methane;

1-[4-(dimethylaminoethoxy)phenyl]-1-(4-cyclopropanecarbonyloxyphenyl)-1-[2-(1-methyl-1,2,3,4-tetrahydronaphthylidene)]methane;

1-[4-(dimethylaminoethoxy)phenyl]-1-(4-cyclopropanecarbonyloxyphenyl)-1-[1-(5-methoxy-1,2,3,4-tetrahydronaphthylidene)]methane;

1-[4-(dimethylaminoethoxy)phenyl]-1-(4-cyclopropanecarbonyloxyphenyl)-1-[4-(1,2,3,4-tetrahydroquinolinylidene)]-methane;

1-[4-(diethylaminoethoxy)phenyl]-1-(4-cyclopropanecarbonyloxyphenyl)-1-[1-(1,2,3,4-tetrahydronaphthylidene)]-methane;

1-[4-(methylethylaminoethoxy)phenyl]-1-(4-cyclopropanecarbonyloxyphenyl)-1-[1-(1,2,3,4-tetrahydronaphthylidene)]methane;

1-[4-(pyrrolidinoethoxy)phenyl]-1-(4-cyclopropanecarbonyloxyphenyl)-1-[1-(1,2,3,4-tetrahydronaphthylidene)]-methane;

1-[4-(cyclohexylmethylaminoethoxy)phenyl]-1-(4-cyclopropanecarbonyloxyphenyl)-1-[1-(1,2,3,4-tetrahydronaphthylidene)]methane;

2-[4-[1-(4-hydroxyiminomethylphenyl)-2-phenyl-1-butenyl]phenoxy]-N,N-dimethylethylamine;

2-[4-[1-(3-hydroxyiminomethylphenyl)-2-phenyl-1-butenyl]phenoxy]-N,N-dimethylethylamine;

1-[2-[1-(4-methoxyiminomethylphenyl)-2-phenyl-1-butenyl]phenoxy]-N,N-dimethylethylamine;

1-[2-[1-(3-methoxyiminomethylphenyl)-2-phenyl-1-butenyl]phenoxy]-N,N-dimethylethylamine;

1-[2-[1-(4-ethoxycarbonylmethoxyiminophenyl)-2-phenyl-1-butenyl]phenoxy]-N,N-dimethylethylamine;

2-[4-[1-(3-ethoxycarbonylmethoxyphenyl)-2-phenyl-1-butenyl]phenoxy]-N,N-dimethylethylamine;

2-[4-[1-(4-phenylmethoxyiminomethylphenyl)-2-phenyl-1-butenyl]phenoxy]-N,N-di-n-methylethylamine; and

2-[4-[1-(3-phenylmethoxyiminomethylphenyl)-2-phenyl-1-butenyl]phenoxy]-N,N-dimethylethylamine.

The triphenylethylene derivative represented by the general formula (1) of the present invention in which the formula (2) is selected as the group $R_1$ may be obtained by: allowing a benzophenone derivative represented by the following formula (5):

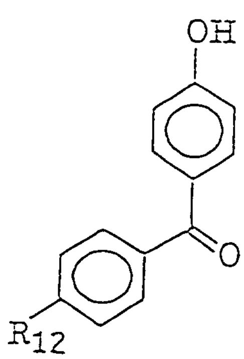

(5)

wherein $R_{12}$ represents a hydrogen atom or a hydroxyl group, to react with a ketone compound represented by the following formula (6):

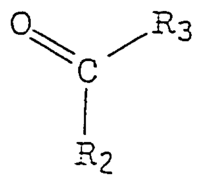

(6)

wherein $R_2$ and $R_3$ are the same meanings as defined above, in a medium containing a reducing agent which is effective to generate a reductive titanium compound and titanium in the state of substantially zero valency in a substantially dry inert atmosphere;
purifying a phenol derivative represented by the following formula (7):

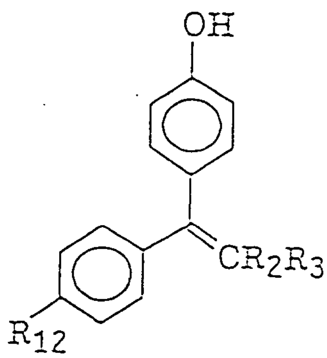

(7)

wherein $R_2$, $R_3$ and $R_{12}$ are the same meanings as defined above, from the thus obtained reaction mixture by column chromatography or the like means;
converting the thus purified phenol derivative into phenoxide and allowing the converted phenoxide to react with an oxirane derivative to obtain an epoxy derivative represented by the following formula (8):

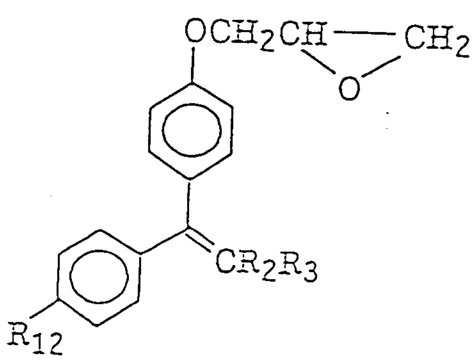

(8)

wherein $R_2$, $R_3$ and $R_{12}$ are the same meanings as defined above; allowing the thus obtained epoxy derivative to react with an appropriate amine to obtain an amine derivative represented by the following formula (9):

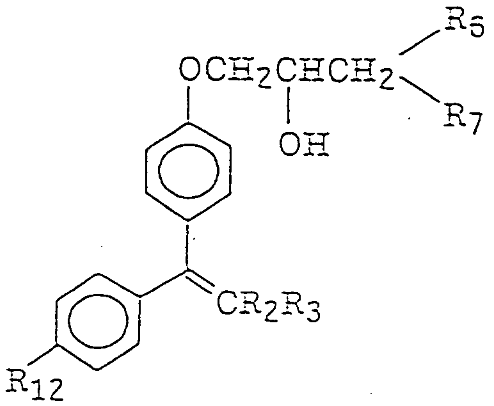

(9)

wherein $R_2$, $R_3$, $R_6$, $R_7$ and $R_{12}$ are the same meanings as defined above; and allowing the thus obtained amine derivative to react with an acid anhydride and acid halide in the presence of an appropriate base or a phase transfer catalyst. In addition, a similar derivative having a substituted hydroxyl group ($R_9C(O)O$-, $R_{10}OCH_2O$- or $-OPO(OH)_2$) as the group $R_4$ may be obtained by introducing the substituted hydroxyl group into a compound of the formula (7) in which $R_{12}$ is a hydroxyl group and then following the above procedure.

A triphenylethylene derivative of the general formula (1) in which the formula (3) is selected as the group $R_1$ may be obtained by allowing the phenol derivative represented by the formula (7) to react with an alcohol derivative represented by the following formula (10):

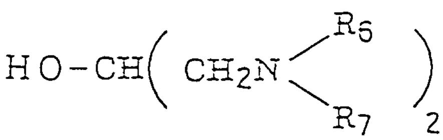

(10)

wherein $R_6$ and $R_7$ are the same meanings as defined above, in the presence of a dehydration condensation agent such as dicyclohexylcarbodiimide or the like and copper iodide.

A triphenylethylene derivative of the general formula (1) in which the formula (4) is selected as the group $R_1$ may be obtained by converting the phenol derivative of the formula (7) into phenoxide, allowing the phenoxide to react with a dihaloethane to obtain a halogenated derivative represented by the following formula (11):

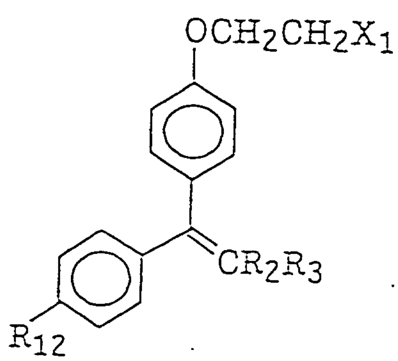

(11)

wherein $R_2$, $R_3$ and $R_{12}$ are the same meanings as defined above; and $X_1$ represents a halogen atom, and then allowing the thus obtained halogenated derivative to react with an appropriate amine.

Among the triphenylethylene derivatives of the general formula (1), compounds having a $-CH=NOR_{11}$ group as $R_4$ or $R_5$ may be obtained, for example, by:
allowing a ketone derivative represented by the following formula (12):

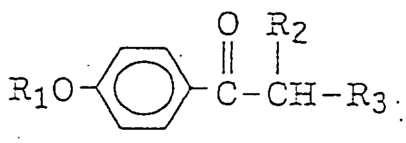

(12)

wherein $R_1$, $R_2$ and $R_3$ are the same meanings as defined above, to react with a phenyldioxorane derivative represented by the following formula (13):

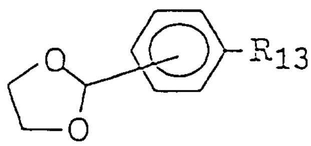

(13)

wherein $R_{13}$ represents $MgX_2$ or lithium and $X_2$ represents chlorine or bromine atom, thereby obtaining an alcohol derivative represented by the following formula (14):

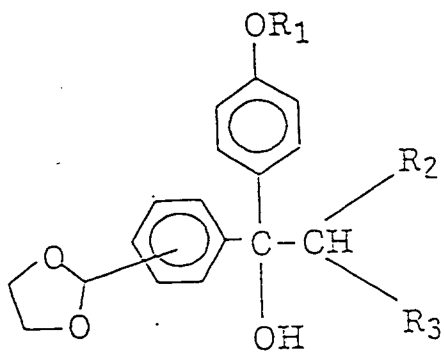

(14)

wherein $R_1$, $R_2$ and $R_3$ are the same meanings as defined above; dehydrating the thus obtained alcohol derivative in the presence of a mineral acid, simultaneously effecting acetal decomposition, thereby obtaining an aldehyde derivative represented by the following formula (15):

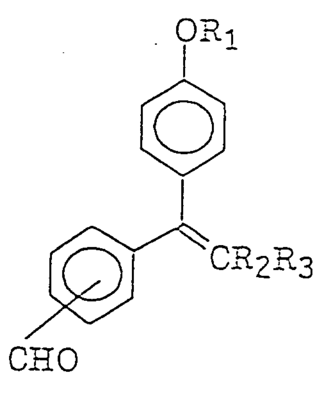

(15)

wherein $R_1$, $R_2$ and $R_3$ are the same meanings as defined above; and
allowing the thus obtained aldehyde derivative to react with an O-substituted hydroxylamine. In this instance, the dehydration reaction of the formula (14) may be effected by heating the compound in the presence of a mineral acid such as concentrated hydrochloric acid or the like.

Though the triphenylethylene derivative of the general formula (1) thus obtained in the aforementioned manner is a mixture of E and Z forms which are isomers against carbon-carbon double bond, these isomers show separate peaks on a high performance liquid chromatograph and therefore can be isolated as single isomers by fractionating them from each other. These isomers can also be isolated by means of

recrystallization after making them into mineral acid salts. When the aforementioned formula (2) is selected in the triphenylethylene derivative of the general formula (1), each of the resulting compounds becomes a mixture of R and S forms as optical isomers because of the presence of asymmetric carbon atom at the base of the hydroxyl group of the aminoalkyl side chain. These optical isomers can be separated into homologous forms by high performance liquid chromatography using an optical isomer separation column. It is also possible to separate and purify them by forming salts with an optically active acid. Also, an optically active triphenylethylene derivative can be obtained by allowing the phenol derivative of formula (7) to react with an optically active oxirane derivative to obtain an optically active epoxy compound and then allowing the thus obtained compound to react with an appropriate amine compound. In addition, the compound of the present invention represented by the general formula (1) can be made into a pharmacologically acceptable acid addition salt by treating it with an inorganic or organic acid. Illustrative examples of the inorganic acid include hydrochloric acid, hydrobromic acid, sulfuric acid, phosphoric acid and the like, and those of the organic acid include citric acid, maleic acid, malic acid, fumaric acid, succinic acid, methanesulfonic acid, p-toluenesulfonic acid, oxalic acid and the like.

The compound of the present invention represented by the general formula (1) and its pharmacologically acceptable acid addition salt have an excellent antiestrogen activity and are useful especially for the treatment of breast tumor. In addition, because of the antiestrogen function, they are also useful as a therapeutic agent for use in the treatment of osteoporosis.

The compound of the present invention may be administered in various dosage forms including oral preparations such as tablets, capsules, granules, powders, solutions and the like, as well as injections, suppositories and the like, of which oral preparations are generally preferred. When tablets, capsules, granules or powders are produced, various additives may be used optionally which include for instance: fillers such as lactose, sucrose, starch, talc, magnesium stearate, crystalline cellulose, methyl cellulose, glycerol, sodium alginate, gum arabic, corn starch, glucose, sorbitol, silicon dioxide and the like; binders such as polyvinyl alcohol, polyvinyl ether, ethyl cellulose, gum arabic, shellac, sucrose, tragacanth, gelatin, hydroxypropyl cellulose, hydroxypropyl starch, polyvinyl pyrrolidone and the like; lubricants such as magnesium stearate, talc and the like; and other known additives such as coloring agents, disintegrating agents and the like. In this instance, tablets may be subjected to coating in conventional manners. Liquid preparations may include suspensions, solutions, syrups, elixirs and the like in the aqueous or oily form, which can be prepared in the usual way using generally used additives.

When the compound of the present invention is orally administered to a patient, dose of the compound may vary depending on the condition, weight, age and the like of, the patient, but it may be administered generally in an approximate amount of from 1 to 500 mg per adult, preferably dividing the necessary dose into 1 to 4 times. In that case, the content of effective ingredient compound in one dose may be in the range of preferably from about 0.5 to 50 mg.

## BEST MODE OF CARRYING OUT THE INVENTION

The following Examples and Preparation Example are provided to further illustrate the present invention. It is to be understood, however, that the present invention is not restricted by the examples. In the following examples, $^1$H-NMR spectrum analysis was carried out using PMX-60SI or GX-400 manufactured by JEOL LTD., with TMS as an internal standard, and chemical shift was expressed as $\delta$ value (ppm).

### EXAMPLE 1

Synthesis of (E•Z)-1-[4-(3-dimethylamino-2-hydroxypropoxy)-phenyl]-1-(4-hydroxyphenyl)-2-(3,4-methylenedioxyphenyl)-1-butene (compound 1):

In a stream of argon, 12.6 ml of titanium tetrachloride was added dropwise to 240 ml of anhydrous tetrahydrofuran while cooling with ice. The resulting mixture was returned to room temperature, stirred for 15 minutes and then subjected to 1.5 hours of reflux in the presence of 12 g of zinc powder. After cooling down to room temperature, the resulting reaction solution was mixed with 3.84 g of 4,4'-dihydroxybenzophenone and 3.22 g of 3,4-methylenedioxypropiophenone, and the mixture was subjected to 2 hours of reflux. After cooling, the thus obtained reaction solution was added to 200 ml of water, followed by extraction with ether. The resulting organic layer was washed with water, dried over sodium sulfate and then treated *in vacuo* to remove the solvent. The resulting oily residue was subjected to silica gel column chromatography (developing solvent: chloroform/methanol) to obtain 3.73 g of 1,1-bis(4-hydroxyphenyl)-2-(3,4-methylenedioxyphenyl)-1-butene in the form of crystals. The thus obtained compound was dissolved in

22 ml of 0.5 N KOH (ethanol solution), and the solvent was subsequently removed *in vacuo*, thereby obtaining a phenoxide of 1,1-bis(4-hydroxyphenyl)-2-(3,4-methylenedioxyphenyl)-1-butene in the form of an oily material. The thus obtained compound was stirred at room temperature for 4 hours in 45 ml of DMF solvent together with 2.6 ml of epibromohydrin. The resulting reaction solution was added to 100 ml of water and then extracted with ether. The resulting organic layer was washed with water, dried over sodium sulfate and then treated *in vacuo* to remove the solvent, thereby obtaining a mixture containing 1-[4-(2,3-epoxypropoxy)phenyl]-1-(4-hydroxyphenyl)-2-(3,4-methylenedioxyphenyl)-1-butene. The thus obtained mixture was dissolved in 30 ml of ethanol, mixed with 3 ml of dimethylamine (50% aqueous solution) and then stirred at room temperature for 4 hours. After completion of the reaction, the solvent was removed *in vacuo*, and the resulting residue was purified by subjecting it to silica gel column chromatography (developing solvent: chloroform/methanol). In this way, 1.95 g of 1-[4-(3-dimethylamino-2-hydroxypropoxy)-phenyl]-1-(4-hydroxyphenyl)-2-(3,4-methylenedioxyphenyl)-1-butene was obtained as the compound of interest, in the form of a pale yellow oily material (E•Z mixture).

$^1$H-NMR spectrum, δ ($CDCl_3$):
0.90 (3H, t, $CH_2CH_3$, $CH_2N$)
3.84, 3.96 (2H, t, $OCH_2CH(OH)CH_2N$)
4.04 - 4.11, 4.10 - 4.19 (1H, m, $OCH_2CH(OH)CH_2N$)
4.64 (2H, bs, ph-OH, $OCH_2CH(OH)CH_2N$)
5.88 (2H, s, $OCH_2O$)
6.47 - 7.15 (11H, m, aromatic proton)

EXAMPLE 2

Synthesis of (E•Z)-1-[4-(3-diethylamino-2-hydroxypropoxy)-phenyl]-1-(4-hydroxyphenyl)-2-(3,4-methylenedioxyphenyl)-1-butene (compound 2):

The process of Example 1 was repeated except that 1-[4-(2,3-epoxypropoxy)phenyl]-1-(4-hydroxyphenyl)-2-(3,4-methylenedioxyphenyl)-1-butene of Example 1 was allowed to react with diethylamine, thereby obtaining 2.04 g of the title compound (E•Z mixture) in the form of a pale yellow oily material.

$^1$H-NMR spectrum, δ ($CDCl_3$):
1.09, 1.10 (6H, 2t, $N(CH_2CH_3)_2$)
2.30 - 2.45 (2H, m, $CH_2CH_3$)
2.51 - 2.82 (6H, m, $OCH_2CH(OH)CH_2N$, $N(CH_2CH_3)_2$)
3.70 - 4.37 (5H, m, ph-OH, $OCH_2CH(OH)CH_2N$)
5.90 (2H, s, $OCH_2O$)

EXAMPLE 3

Synthesis of (E•Z)-1-[4-(3-ethylmethylamino-2-hydroxypropoxy)phenyl]-1-(4-hydroxyphenyl)-2-(3,4-methylenedioxyphenyl)-1-butene (compound 3):

The process of Example 1 was repeated except that 1-[4-(2,3-epoxypropoxy)phenyl]-1-(4-hydroxyphenyl)-2-(3,4-methylenedioxyphenyl)-1-butene of Example 1 was allowed to react with methylethylamine, thereby obtaining 1.98 g of the title compound (E•Z mixture) in the form of a pale yellow oily material.

$^1$H-NMR spectrum, δ ($CDCl_3$):
0.92 (3H, t, $CH_2CH_3$)
1.10, 1.12 (3H, t, $NCH_2CH_3$)
2.30 - 2.48 (5H, m, $NCH_3$, $CH_2CH_3$)
2.48 - 2.73 (4H, m, $OCH_2CH(OH)CH_2NCH_2CH_3$)
3.81 - 3.93, 3.95 - 4.03 (2H, m, $OCH_2CH(OH)CH_2N$)
6.48 - 7.18 (11H, m, aromatic proton)

EXAMPLE 4

Synthesis of (E•Z)-1-[4-(3-cyclohexylmethylamino-2-hydroxypropoxy)phenyl]-1-(4-hydroxyphenyl)-2-(3,4-methylenedioxyphenyl)-1-butene (compound 4):

The process of Example 1 was repeated except that 1-[4-(2,3-epoxypropoxy)phenyl]-1-(4-hydroxyphenyl)-2-(3,4-methylenedioxyphenyl)-1-butene of Example 1 was allowed to react with cyclohexylmethylamine, thereby obtaining 2.20 g of the title compound (E•Z mixture) in the form of a pale yellow oily material.

$^1$H-NMR spectrum, $\delta$ ($CDCl_3$):
0.92 (3H, t, $CH_2C\underline{H}_3$)
1.01 - 2.20 (10H, m, methylene proton of cyclohexane ring)
2.31 - 2.48 (2H, m, $C\underline{H}_2CH_3$)
2.73 - 2.91 (3H, d, $NC\underline{H}_3$)
2.92 - 3.53 (3H, m, $OCH_2CH(OH)C\underline{H}_2N$, methine proton of cyclohexane ring)
3.69 - 4.18 (2H, m, $OC\underline{H}_2CH(OH)CH_2N$)
4.27 - 4.57 (1H, m, $OCH_2C\underline{H}(OH)CH_2N$)
5.88 (2H, s, $OC\underline{H}_2O$)
6.48 - 7.13 (11H, m, aromatic proton)

EXAMPLE 5

Synthesis of (E)-1-[4-(3-dimethylamino-2-hydroxypropoxy)-phenyl]-1-phenyl-2-(3,4-methylenedioxyphenyl)-1-butene (compound 5) and (Z)-1-[4-(3-dimethylamino-2-hydroxypropoxy)-phenyl]-1-phenyl-2-(3,4-methylenedioxyphenyl)-1-butene (compound 6):

In a stream of argon, 12.6 ml of titanium tetrachloride was added dropwise to 240 ml of anhydrous tetrahydrofuran while cooling with ice. The resulting mixture was returned to room temperature, stirred for 15 minutes and then subjected to 1.5 hours of reflux in the presence of 12 g of zinc powder. After cooling down to room temperature, the resulting reaction solution was mixed with 3.56 g of 4-hydroxybenzophenone and 3.22 g of 3,4-methylenedioxypropiophenone, and the mixture was subjected to 2 hours of reflux. After cooling, the thus obtained reaction solution was added to 200 ml of water, followed by extraction with ether. The resulting organic layer was washed with water, dried over sodium sulfate and then treated *in vacuo* to remove the solvent. The resulting oily residue was subjected to silica gel column chromatography (developing solvent: chloroform/methanol) to obtain 3.46 g of 1-(4-hydroxyphenyl)-1-phenyl-2-(3,4-methylenedioxyphenyl)-1-butene in the form of crystals. The thus obtained compound was dissolved in 22 ml of 0.5 N KOH (ethanol solution) to obtain a phenoxide of 1-(4-hydroxyphenyl)-1-phenyl-2-(3,4-methylenedioxyphenyl)-1-butene in the form of an oily material. The thus obtained compound was stirred at room temperature for 4 hours in 45 ml of DMF solvent together with 2.6 ml of epibromohydrin. The resulting reaction solution was added to 100 ml of water and then extracted with ether. The resulting organic layer was washed with water, dried over sodium sulfate and then treated *in vacuo* to remove the solvent, thereby obtaining a mixture containing 1-[4-(2,3-epoxypropoxy)phenyl]-1-phenyl-2-(3,4)-methylenedioxyphenyl)-1-butene. The thus obtained mixture was dissolved in 30 ml of ethanol, mixed with 3 ml of dimethylamine (50% aqueous solution) and then stirred at room temperature for 4 hours. After completion of the reaction, the solvent was removed *in vacuo*, and the resulting residue was purified by subjecting it to silica gel column chromatography (developing solvent: chloroform/methanol) to obtain 1.78 g of (E•Z)-1-[4-(3-dimethylamino-2-hydroxypropoxy)phenyl]-1-phenyl-2-(3,4-methylenedioxyphenyl)-1-butene in the form of a pale yellow oily material. Thereafter, a 240 mg portion of the thus obtained product was subjected to high performance liquid chromatography equipped with a column of PREPPAX CARTRIDGE DELTA-PAK C18 (Waters Associates, Inc.; 47 mm in inner diameter x 30 cm in length), using a solvent system of water:methanol = 37:63 (containing 0.1% trifluoroacetic acid) at a flow rate of 70 ml/minutes, thereby obtaining 70 mg of (E)-1-[4-(3-dimethylamino-2-hydroxypropoxy)phenyl]-1-phenyl-2-(3,4-methylenedioxyphenyl)-1-butene and 60 mg of (Z)-1-[4-(3-dimethylamino-2-hydroxypropoxy)phenyl]-1-phenyl-2-(3,4-methylenedioxyphenyl)-1-butene as the compounds of interest.

$^1$H-NMR spectrum, $\delta$ ($CDCl_3$):

Compound 5

0.92 (3H, t, $CH_2C\underline{H}_3$)
2.09 - 2.67 (10H, m, $N(CH_3)_2$, $CH_2CH_3$, $CH_2N$)
3.70 - 4.27 (3H, m, $OC\underline{H}_2C\underline{H}(OH)CH_2N$)
5.80 (2H, s, $OC\underline{H}_2O$)
6.46 - 7.33 (12H, m, aromatic proton)

Compound 6

0.90 (3H, t, $CH_2C\underline{H}_3$)
2.10 - 2.83 (10H, m, $N(C\underline{H}_3)_2$, $C\underline{H}_2CH_3$, $C\underline{H}_2N$)
3.27 (1H, bs, $OCH_2CH(O\underline{H})CH_2N$)
3.67 - 4.27 (3H, m, $OC\underline{H}_2C\underline{H}(OH)CH_2N$)
5.83 (2H, s, $OC\underline{H}_2O$)
6.33 - 7.53 (12H, m, aromatic proton)

EXAMPLE 6

Synthesis of (E•Z)-1-[4-(3-dimethylamino-2-hydroxypropoxy)-phenyl]-1-(4-hydroxyphenyl)-2-phenyl-1-butene (compound 7):

In a stream of argon, 16 ml of titanium tetrachloride was added dropwise to 240 ml of anhydrous tetrahydrofuran while cooling with ice. The resulting mixture was returned to room temperature, stirred for about 20 minutes and then subjected to 2 hours of reflux in the presence of 14.3 g of zinc powder. After cooling down to room temperature, the resulting reaction solution was mixed with 5.46 g of 4,4'-dihydroxybenzophenone and 3.42 g of propiophenone, and the mixture was subjected to 4 hours of reflux. After cooling, the thus obtained reaction solution was added to 200 ml of water, followed by extraction with ether. The resulting organic layer was washed with water, dried over sodium sulfate and then treated *in vacuo* to remove the solvent. The resulting oily residue was subjected to silica gel column chromatography (developing solvent: hexane/ethyl acetate) to obtain about 8 g of crystals which was then recrystallized from toluene, thereby obtaining 6.00 g of 1,1-bis(4-hydroxyphenyl)-2-phenyl-1-butene in the form of white crystals. A 2.0 g portion of the thus obtained crystals was dissolved in 9.7 ml of 0.5 N potassium hydroxide (ethanol solution), and the solvent was subsequently removed *in vacuo*, thereby obtaining a phenoxide of 1,1-bis(4-hydroxyphenyl)-2-phenyl-1-butene in the form of an oily material. The thus obtained compound was stirred at room temperature for 4 hours in 30 ml of dimethylformamide solvent together with 1.5 ml of epibromohydrin. The resulting reaction solution was added to 100 ml of water and then extracted with ether. The resulting organic layer was washed with water, dried over sodium sulfate and then treated *in vacuo* to remove the solvent, thereby obtaining a mixture containing 1-[4-(2,3-epoxypropoxy)phenyl]-1-(4-hydroxyphenyl)-2-phenyl-1-butene. A 800 mg portion of the thus obtained mixture was dissolved in 12 ml of ethanol, mixed with 0.678 ml of 50% dimethylamine aqueous solution and then stirred overnight at room temperature. After completion of the reaction, the solvent was removed *in vacuo*, and the resulting residue was purified by subjecting it to thin layer chromatography (developing solvent: chloroform/methanol = 5/1). In this way, 125 mg of 1-[4-(3-dimethylamino-2-hydroxypropoxy)phenyl]-1-(4-hydroxyphenyl)-2-phenyl-1-butene was obtained as the compound of interest in the form of a pale yellow oily material (E•Z mixture).
$^1$H-NMR spectrum, $\delta$ ($CDCl_3$):
0.89 (3H, t, $CH_2C\underline{H}_3$)
2.33, 2.38 (3H, s, $N(C\underline{H}_3)_2$)
2.33 - 2.70 (4H, m, $C\underline{H}_2CH_3$), $OCH_2CH(OH)C\underline{H}_2N$)
3.67 - 4.33 (3H, m, $OC\underline{H}_2C\underline{H}(OH)CH_2N$)
5.40 - 5.67 (2H, m, $OCH_2CH(O\underline{H})CH_2N$, Ar-O$\underline{H}$)
6.27 - 7.27 (13H, m, aromatic proton)

EXAMPLE 7

Synthesis of (E•Z)-1-[4-(3-cyclohexylamino-2-hydroxypropoxy)-phenyl]-1-(4-hydroxyphenyl)-2-phenyl-1-butene (compound 8):

The process of Example 6 was repeated except that cyclohexylamine was used instead of dimethylamine, thereby obtaining 170 mg of the title compound (E•Z mixture) in the form of a pale yellow oily material.

$^1$H-NMR spectrum, δ ($CDCl_3$):
0.91 (3H, t, $CH_2C\underline{H}_3$)
1.45 - 2.17 (10H, m, methylene hydrogen of cyclohexane ring)
2.41 - 2.50 (2H, q, $C\underline{H}_2CH_3$)
2.93 - 3.11 (1H, m, methine hydrogen of cyclohexane ring)
3.11 - 3.37 (2H, m, $OCH_2CH(OH)C\underline{H}_2N$)
3.75 - 3.90, 3.93 - 4.08 (2H, m, $OC\underline{H}_2CH(OH)CH_2N$)
4.26 - 4.50 (1H, m, $OCH_2C\underline{H}(OH)CH_2N$)
6.39 (1H, bs, $OCH_2CH(O\underline{H})CH_2N$)
6.45 - 7.17 (13H, m, aromatic proton)
7.60 - 7.82, 8.83 - 9.09 (2H, m, N$\underline{H}$, Ar-O$\underline{H}$)

EXAMPLE 8

Synthesis of (E•Z)-1-[4-(3-cyclohexylamino-2-hydroxypropoxy)-phenyl]-1-(4-hydroxyphenyl)-2-(3,4-,methylenedioxyphenyl)-1-butene (compound 9):

The process of Example 1 was repeated except that cyclohexylamine was used instead of dimethylamine, thereby obtaining 1.95 g of the title compound (E•Z mixture) in the from of a pale yellow oily material.

$^1$H-NMR spectrum, δ ($CDCl_3$):
1.18 - 2.18 (10H, m, methylene hydrogen of cyclohexane ring)
2.38 (2H, q, $C\underline{H}_2CH_3$)
2.91 - 3.12 (1H, m, methine hydrogen of cyclohexane ring)
3.07 - 3.31 (2H, m, $OCH_2CH(OH)C\underline{H}_2N$)
3.80 - 4.07 (2H, m, $OC\underline{H}_2CH(OH)CH_2N$)
3.96 - 4.29 (1H, m, $OCH_2CH(O\underline{H})CH_2N$)
4.18 - 4.55 (1H, m, $OCH_2C\underline{H}(OH)CH_2N$)
5.88, 5.89 (2H, s, $OC\underline{H}_2O$)
6.47 - 7.07 (11H, m, aromatic proton)
7.93 - 8.19 (1H, m, N$\underline{H}$)
9.23 - 9.42 (1H, bs, Ar-O$\underline{H}$)

EXAMPLE 9

Synthesis of (E•Z)-1-[4-(3-methylamino-2-hydroxypropoxy)-phenyl]-1-(4-hydroxyphenyl)-2-(3,4-,methylenedioxyphenyl)-1-butene (compound 10):

The process of Example 1 was repeated except that methylamine was used instead of dimethylamine, thereby obtaining 1.6 g of the title compound (E•Z mixture) in the form of a pale yellow oily material.

$^1$H-NMR spectrum, δ ($CDCl_3$, $CD_3OD$, $D_2O$):
0.90 (3H, t, $C\underline{H}_3$)
2.07 - 2.70 (5H, m, $-NHC\underline{H}_3$, $-C\underline{H}_3CH_3$)
2.70 - 3.10 (2H, m, $OCH_2CH(OH)C\underline{H}_2N$)
3.67 - 4.40 (3H, m, $OC\underline{H}_2C\underline{H}(OH)CH_2N$)
5.78 (2H, s, $OC\underline{H}_2O$)
6.47 - 7.07 (11H, m, aromatic proton)

EXAMPLE 10

Synthesis of (E•Z)-1-[4-(3-ethylamino-2-hydroxypropoxy)-phenyl]-1-(4-hydroxyphenyl)-2-(3,4-methylenedioxyphenyl)-1-butene (compound 11):

The process of Example 1 was repeated except that ethylamine was used instead of dimethylamine, thereby obtaining 1.5 g of the title compound (E•Z mixture) in the form of a pale yellow oily material.
$^1$H-NMR spectrum, δ ($CDCl_3$):
0.90, 1.13 (6H, t × 2, $NHCH_2C\underline{H}_3$, $CH_2C\underline{H}_3$)
2.10 - 3.10 (6H, m, $OCH_2CH(OH)C\underline{H}_2NC\underline{H}_2CH_3$, $C\underline{H}_2CH_3$)
3.63 - 4.53 (6H, m, $OC\underline{H}_2C\underline{H}(O\underline{H})CH_2N\underline{H}$, $\underline{H}OPh$)
5.78 (2H, s, $OC\underline{H}_2O$)
6.26 - 7.23 (11H, m, aromatic proton)

EXAMPLE 11

Synthesis of (Z)-1-[4-(3-cyclohexylamino-2-hydroxypropoxy)-phenyl]-1,2-diphenyl-1-butene (compound 12):

In a stream of argon, 16 ml of titanium tetrachloride was added dropwise to 240 ml of anhydrous tetrahydrofuran while cooling with ice. The resulting mixture was returned to room temperature, stirred for about 20 minutes and then subjected to 2 hours of reflux in the presence of 14.3 g of zinc powder. After cooling down to room temperature, the resulting reaction solution was mixed with 4.76 g of 4-hydroxybenzophenone and 3.22 g of propiophenone, and the mixture was subjected to 2 hours of reflux. After cooling, the thus obtained reaction solution was added to 200 ml of water, followed by extraction with ether. The resulting organic layer was washed with water, dried over sodium sulfate and then treated *in vacuo* to remove the solvent, thereby obtaining 9.88 g of crude crystals containing 1-(4-hydroxyphenyl)-1,2-diphenyl-1-butene. The thus obtained oily residue was dissolved in 66 ml of 0.5 N potassium hydroxide (ethanol solution), and the solvent was subsequently removed *in vacuo*, thereby obtaining a phenoxide of 1-(4-hydroxyphenyl)-1,2-diphenyl-1-butene in the form of an oily material. The thus obtained compound was stirred at room temperature for 3 hours in 150 ml of DMF solvent together with 6.2 ml of epibromohydrin. The resulting reaction solution was added to 200 ml of ice water and then extracted with ether. The resulting organic layer was washed with water, dried over sodium sulfate and then treated *in vacuo* to remove the solvent, thereby obtaining 10.3 g of a mixture containing 1-[4-(2,3-epoxypropoxy)phenyl]-1,2-diphenyl-1-butene. A 500 mg portion of the thus obtained mixture was dissolved in 7.5 ml of ethanol, mixed with 0.403 ml of cyclohexylamine and then stirred at room temperature for 4 hours. After completion of the reaction, the solvent was removed *in vacuo*, and the resulting residue was purified by subjecting it to thin layer chromatography (developing solvent: chloroform/methanol = 14/1). By recrystallizing the thus purified product from ether, 127 mg of 1-[4-(3-cyclohexylamino-2-hydroxypropoxy)phenyl]-1,2-diphenyl-1-butene was obtained as the compound of interest in the form of white crystals.
$^1$H-NMR spectrum, δ ($CDCl_3$):
0.92 (3H, t, $CH_2C\underline{H}_3$)
0.83 - 2.13 (10H, m, methylene hydrogen of cyclohexane ring)
2.47 (2H, q, $C\underline{H}_2CH_3$)
2.73 - 3.23 (3H, m, $OCH_2CH(OH)C\underline{H}_2N$, methine hydrogen of cyclohexane ring)
3.70 - 3.93 (2H, m, $OC\underline{H}_2CH(OH)C\underline{H}_2N$)
4.01 - 4.57 (1H, m, $OCH_2C\underline{H}(OH)CH_2N$)
5.18 (2H, bs, $N\underline{H}$, $OCH_2CH(O\underline{H})CH_2N$)
6.38 - 7.42 (14H, m, aromatic proton)

EXAMPLE 12

Synthesis of (Z)-1-[4-(3-ethylamino-2-hydroxypropoxy)phenyl]-1,2-diphenyl-1-butene (compound 13):

The process of Example 1 was repeated except that 500 mg of 1-[4-(2,3-epoxypropoxy)phenyl]-1,2-diphenyl-1-butene obtained in Example 11 was allowed to react with 0.5 ml of ethylamine (33% aqueous solution), thereby obtaining 181 mg of the title compound in the form of white crystals.
$^1$H-NMR spectrum, δ ($CDCl_3$):
0.90 (3H, t, $CCH_2C\underline{H}_3$)

1.33 (3H, t, $NCH_2CH_3$)
2.45 (2H, q, $CCH_2CH_3$)
2.73 - 3.37 (4H, m, $NCH_2CH_3$, $OCH_2CH(OH)CH_2N$)
3.67 - 3.96 (2H, m, $OCH_2CH(OH)CH_2N$)
4.23 - 4.77 (1H, m, $OCH_2CH(OH)CH_2N$)
5.82 (2H, bs, NH, $OCH_2CH(OH)CH_2N$)
6.30 - 7.33 (14H, m, aromatic proton)

EXAMPLE 13

Synthesis of (Z)-1-[4-(3-isopropylamino-2-hydroxypropoxy)-phenyl]-1,2-diphenyl-1-butene (compound 14):

The process of Example 1 was repeated except that 500 mg of 1-[4-(2,3-epoxypropoxy)phenyl]-1,2-diphenyl-1-butene obtained in Example 11 was allowed to react with 0.3 ml of isopropylamine, thereby obtaining 230 mg of the title compound in the form of white crystals.
$^1$H-NMR spectrum, $\delta$ ($CDCl_3$):
0.91 (3H, t, $CCH_2CH_3$)
1.25, 1.37 (6H, m, $NCH(CH_3)_2$)
2.43 (2H, q, $CCH_2CH_3$)
2.83 - 3.42 (3H, m, $NCHC(CH_3)_2$, $CH(OH)CH_2N$)
3.61 - 4.00 (2H, m, $OCH_2CH(OH)CH_2N$)
4.08 - 4.67 (1H, m, $OCH_2CH(OH)CH_2N$)
5.81 (2H, bs, NH, $OCH_2CH(OH)CH_2N$)
6.26 - 7.31 (14H, m, aromatic proton)

EXAMPLE 14

Synthesis of (E•Z)-1-[4-(3-dimethylamino-2-acetoxypropoxy)-phenyl]-1-(4-acetoxyphenyl)-2-(3,4-methylenedioxyphenyl)-1-butene (compound 15):

A 1,500 mg portion of a corresponding compound was dissolved in 7.5 ml of pyridine, and the resulting solution was mixed with 1.0 ml of acetic anhydride and stirred at room temperature for 5 hours. After concentrating the reaction system under a reduced pressure, the resulting residue was purified by subjecting it to silica gel column chromatography (developing solvent: chloroform/methanol), thereby obtaining 200 mg of 1-[4-(3-dimethylamino-2-acetoxypropoxy)phenyl]-1-(4-acetoxyphenyl)-2-(3,4-methylenedioxyphenyl)-1-butene (compound 15) as the compound of interest in the form of a pale yellow oily material (E•Z mixture).
$^1$H-NMR spectrum, $\delta$ ($CDCl_3$):
0.92 (3H, t, $CH_2CH_3$)
2.04, 2.09, 2.16, 2.21 (6H, 2s, 2OAc)
2.25, 2.30 (6H, s, $N(CH_3)_2$)
2.44 (2H, q, $CH_2CH_3$)
2.53, 2.61 (2H, t, $OCH_2CH(OAc)CH_2N$)
3.95 - 4.18 (2H, m, $OCH_2CH(OAc)CH_2N$)
5.20 - 5.32 (1H, m, $OCH_2CH(OAc)CH_2N$)
5.88 (2H, s, $OCH_2O$)

EXAMPLE 15

Synthesis of (E•Z)-1-[4-(3-dimethylamino-2-acetoxypropoxy)-phenyl]-1-(4-hydroxyphenyl)-2-(3,4-methylenedioxyphenyl)-1-butene (compound 16):

A 500 mg portion of 1-[4-(3-dimethylamino-2-hydroxypropoxy)phenyl]-1-(4-hydroxyphenyl)-2-(3,4-methylenedioxyphenyl)-1-butene obtained in Example 1 was dissolved in 3 ml of dioxane, followed by the addition of 100 mg of powdery sodium hydroxide and 2 mg of tetra-n-butyl-ammonium hydrogensulfate. While stirring the resulting mixture, 97 mg of acetyl chloride dissolved in 1 ml of, dioxane was added dropwise over 30 minutes or more. After additional 3 hours or more of stirring at room temperature, the reaction system was neutralized with 2 N hydrochloric acid aqueous solution, the thus formed precipitate

was removed by filtration, and then the resulting filtrate was concentrated under a reduced pressure. Thereafter, the thus concentrated product was purified by subjecting it to thin layer chromatography (developing solvent: chloroform/methanol), thereby obtaining 90 mg of 1-[4-(3-dimethylamino-2-acetoxypropoxy)phenyl]-1-(4-hydroxyphenyl)-2-(3,4-methylenedioxyphenyl)-1-butene in the form of a pale yellow oily material.

$^1$H-NMR spectrum, δ ($CDCl_3$):
0.92 (3H, t, $CH_2\underline{CH_3}$)
2.00, 2.05 (3H, s, $\underline{OAc}$)
2.29, 2.33 (6H, s, $N(\underline{CH_3})_2$)
2.39, 2.40 (2H, q, $\underline{CH_2}CH_3$)
2.52 - 2.75 (2H, m, $OCH_2CH(OAc)\underline{CH_2}N$)
3.93 - 4.16 (2H, m, $O\underline{CH_2}CH(OAc)CH_2N$)
5.22 - 5.35 (1H, m, $OCH_2\underline{CH}(OAc)CH_2N$)
5.89, 5.90 (2H, s, $OCH_2O$)

EXAMPLE 16

Synthesis of (E•Z)-1-[4-(3-dimethylamino-2-acetoxypropoxy)-phenyl]-1-(4-methoxymethoxyphenyl)-2-(3,4-methylenedioxyphenyl)-1-butene (compound 17):

A 4 g portion of 1,1-bis(4-hydroxyphenyl)-2-(3,4-methylenedioxyphenyl)-1-butene obtained in Example 1 was dissolved in 22 ml of 0.5 N potassium hydroxide (ethanol solution), and the solvent was subsequently removed *in vacuo*, thereby obtaining a phenoxide of 1,1-bis(4-hydroxyphenyl)-2-(3,4-methylenedioxyphenyl)-1-butene in an oily form. In a stream of argon, to this were added 326 mg of 18-crown-6-ether and 50 ml of acetonitrile, followed by 30 minutes of stirring at room temperature. After adding 0.837 ml of chloromethyl methyl ether at -20°C, the resulting mixture was stirred for 1 hour and 30 minutes while gradually warming up the solution to room temperature, and the thus prepared reaction solution was neutralized with saturated sodium bicarbonate aqueous solution, followed by extraction with ether. The resulting organic layer was washed with water, dried over anhydrous sodium sulfate and then treated *in vacuo* to remove the solvent, thereby obtaining 1.8 g of 1-(4-methoxymethoxyphenyl)-1-(4-hydroxyphenyl)-2-(3,4-methylenedioxyphenyl)-1-butene and 1.6 g of 1,1-bis-(4-methoxymethoxyphenyl)-2-(3,4-methylenedioxyphenyl)-1-butene and recovering 1.1 g of the starting material. A 1.8 g portion of the thus obtained 1-(4-methoxymethoxyphenyl)-1-(4-hydroxyphenyl)-2-(3,4-methylenedioxyphenyl)-1-butene was dissolved in 9.2 ml of 0.5 N potassium hydroxide (ethanol solution), and the solvent was subsequently removed *in vacuo*, thereby obtaining a phenoxide in the form of an oily material. The thus obtained compound was stirred at room temperature for 4 hours in 25 ml of DMF solvent together with 0.4 ml of epibromohydrin. The resulting reaction solution was added to 50 ml of water to obtain 1-[4-(2,3-epoxypropoxy)phenyl]-1-(4-methoxymethoxyphenyl)-2-(3,4-methylenedioxyphenyl)-1-butene. The thus obtained compound was dissolved, without purifying it, in 25 ml of ethanol, mixed with 0.5 ml of dimethylamine (50% aqueous solution) and then stirred overnight at room temperature. After completion of the reaction, the solvent was removed *in vacuo*, and the resulting residue was purified by subjecting it to silica gel column chromatography (developing solvent: toluene/ethanol, containing 0.1% diethylamine), thereby obtaining 2.2 g of 1-[4-(3-dimethylamino-2-hydroxypropoxy)phenyl]-1-(4-methoxymethoxyphenyl)-2-(3,4-methylenedioxyphenyl)-1-butene in the form of a colorless transparent oily material. A 170 mg portion of the thus obtained 1-[4-(3-dimethylamino-2-hydroxypropoxy)phenyl]-1-(4-methoxymethoxyphenyl)-2-(3,4-methylenedioxyphenyl)-1-butene was dissolved in 3 ml of pyridine, and the resulting solution was mixed with 3 ml of acetic anhydride and stirred at room temperature for 4 hours. After completion of the reaction, the solvent was removed *in vacuo*, and the resulting residue was purified by subjecting it to silica gel column chromatography (developing solvent: toluene/ethanol, containing 0.1% diethylamine), thereby obtaining 168 mg of 1-[4-(3-dimethylamino-2-acetoxypropoxy)phenyl]-1-(4-methoxymethoxyphenyl)-2-(3,4-methylenedioxyphenyl)-1-butene as the compound of interest in the form of a pale yellow oily material.

$^1$H-NMR spectrum, δ ($CDCl_3$):
0.90 (3H, t, $CH_2\underline{CH_3}$)
1.97, 2.03 (3H, s, $\underline{OAc}$)
2.20, 2.26 (6H, s, $N(\underline{CH_3})_2$)
2.27 - 2.70 (4H, m, $\underline{CH_2}CH_3$, $OCH_2CH(OAc)\underline{CH_2}N$)
3.36, 3.45 (3H, s, $OCH_2O\underline{CH_3}$)
3.83 - 4.16 (2H, m, $O\underline{CH_2}CH(OAc)CH_2N$)

5.00, 5.11 (2H, s, $OC\underline{H_2}OCH_3$)
5.20 - 5.43 (1H, m, $OCH_2C\underline{H}(OAc)CH_2N$)
5.80 (2H, s, $OCH_2O$)

EXAMPLE 17

Synthesis of (E•Z)-1-[4-(1,3-bisdimethylamino-2-propoxy)-phenyl]-1-(4-hydroxyphenyl)-2-(3,4-methylenedioxyphenyl)-1-butene (compound 18):

A 0.949 g portion of crystals of 1,1-bis(4-hydroxyphenyl)-2-(3,4-methylenedioxyphenyl)-1-butene obtained in Example 1 was added to a mixture consisting of 0.658 g of 1,3-bisdimethylamino-2-propanol, 1.05 g of dicyclohexylcarbodiimide and 40 mg of copper iodide (stirred at 60°C for 2 hours in advance), and the thus prepared mixture was stirred at 60°C for 2 hours. The reaction solution was mixed with 50 ml of ether, the thus formed solid materials were removed by filtration, and the resulting filtrate was concentrated. Thereafter, the thus concentrated oily residue was purified by subjecting it to thin layer chromatography (developing solvent: toluene/methanol), thereby obtaining 0.402 g of (E•Z)-1-[4-(1,3-bisdimethylamino-2-propoxy)-phenyl]-1-(4-hydroxyphenyl)-2-(3,4-methylenedioxyphenyl)-1-butene as the compound of interest.
$^1$H-NMR spectrum, δ ($CDCl_3$):
0.90 - 0.93 (3H, t, $CH_2C\underline{H_3}$)
2.11 - 2.66 (18H, m, $N(C\underline{H_3})_2$, $(CH_3)_2N$, $NC\underline{H_2}CH(OAr)C\underline{H_2}N$, $CH_2CH_3$)
3.95 - 4.09 (1H, m, $NCH_2CH(OAr)CH_2N$)
5.87 (2H, s, $OC\underline{H_2}O$)
6.49 - 6.81 (10H, m, aromatic proton, $\underline{H}O$-ph)
7.01 - 7.10 (2H, m, aromatic proton)

EXAMPLE 18

Synthesis of (E•Z)-1-[4-(2-cyclohexylaminoethoxy)phenyl]-1-(4-hydroxyphenyl)-2-(3,4-methylenedioxyphenyl)-1-butene (compound 19):

A 3.0 g portion of the 6.15 g of white crystalline 1,1-bis(4-hydroxyphenyl)-2-phenyl-1-butene obtained in Example 6 was dissolved in 14.3 ml of 1,2-dibromoethane, and the resulting solution was mixed with 0.513 g of powdery potassium hydroxide and subjected to 2 days of reflux. Since the reaction stopped when about 20% of the reaction was completed, the following treatment was carried out at this stage. That is, the reaction system was diluted with 200 ml of methylene chloride and washed with 2 N hydrochloric acid aqueous solution, and the resulting organic layer was dried over anhydrous sodium sulfate. After removing anhydrous sodium sulfate by filtration, the resulting filtrate was concentrated under a reduced pressure to purify the thus obtained residue by silica gel column chromatography (chloroform/methanol), thereby obtaining 0.680 g of a bromo-form compound as a light yellow viscous liquid and recovering 2 g of the diphenol-form starting material. The bromo-form compound showed a positive Beilstein reaction. A 0.2 g portion of the bromo-form compound was dissolved in 0.5 ml of ethanol, mixed with 0.5 ml of cyclohexylamine and then stirred overnight at about 60°C. Since the reaction stopped when about 50% of the reaction was completed, the following treatment was carried out at this stage. That is, the reaction system was concentrated under a reduced pressure, and the resulting residue was purified by subjecting it to thin layer chromatography (chloroform:methanol = 8:1), thereby obtaining 45 mg of 1-[4-(2-cyclohexylaminoethoxy)-phenyl]-1-(4-hydroxyphenyl)-2-(3,4-methylenedioxyphenyl)-1-butene as the compound of interest in the form of white crystals.
$^1$H-NMR spectrum, δ ($CDCl_3$):
0.92 (3H, t, $CH_2C\underline{H_3}$)
1.13 - 2.05 (11H, m, methylene hydrogen of cyclohexane ring, $N\underline{H}$)
2.38 - 2.43 (2H, m, $C\underline{H_2}CH_3$)
2.44 - 2.60 (1H, m, methine hydrogen of cyclohexane ring)
2.99 - 3.09 (2H, m, $OCH_2C\underline{H_2}N$)
3.90 - 4.52 (3H, m, $OC\underline{H_2}CH_2N$, $O\underline{H}$)
5.88 - 5.89 (2H, s, $OC\underline{H_2}O$)
6.44 - 7.18 (11H, m, aromatic proton)

EXAMPLE 19

Synthesis of (E•Z)-1-[4-(2-dimethylaminoethoxy)phenyl]-1-(4-hydroxyphenyl)-2-(3,4-methylenedioxyphenyl)-1-butene (compound 20):

A 1.62 g portion of the bromo-form compound obtained in Example 18 was dissolved in 24 ml of ethanol, mixed with 1.00 ml of 50% dimethylamine aqueous solution and then stirred at about 45°C for 6 hours, followed by further addition of 1.00 ml 50% dimethylamine aqueous solution and by additional stirring at about 45°C for 6 hours. Since the reaction stopped when about 80% of the reaction was completed, the following treatment was carried out at this stage. That is, the reaction system was concentrated under a reduced pressure, and the resulting residue was purified by subjecting it to silica gel column chromatography (ethanol/toluene), thereby obtaining 0.818 mg of 1-[4-(2-dimethylaminoethoxy)-phenyl]-1-(4-hydroxyphenyl)-2-(3,4-methylenedioxyphenyl)-1-butene as the compound of interest in the form of white crystals.

$^1$H-NMR spectrum, δ ($CDCl_3$):
0.82 (3H, t, $CH_2\underline{CH_3}$)
2.20 - 2.45 (8H, m, $\underline{CH_2}CH_3$, $N(\underline{CH_3})_2$)
2.78 - 2.87 (2H, m, $OCH_2\underline{CH_2}N$)
3.85 - 4.00 (2H, m, $O\underline{CH_2}CH_2N$)
5.77, 5.80 (2H, s, $O\underline{CH_2}O$)
6.22 - 7.02 (11H, m, aromatic proton)
8.00 - 8.06 (1H, m, O$\underline{H}$)

EXAMPLE 20

Synthesis of 2-[4-[1-(4-hydroxyiminomethylphenyl)-2-phenyl-1-butenyl]phenoxy]-N,N-dimethylethylamine (compound 21):

In a stream of argon, an appropriate amount of 1-[4'-(2-dimethylaminoethoxy)phenyl]-2-phenyl-n-butane-1-one dissolved in 10 ml of anhydrous tetrahydrofuran was added to 20 ml anhydrous tetrahydrofuran solution of Grignard's reagent which has been prepared from 25 g of 4-bromophenyl-1,3-dioxorane and 2.6 g of metallic magnesium, and the resulting mixture was subjected to 2 hours of reflux. After cooling, the resulting reaction solution was mixed with 100 ml of saturated ammonium chloride solution, followed by extraction with ether. The resulting organic layer was washed with water, dried over sodium sulfate and then treated ***in vacuo*** to remove the solvent. The thus obtained oily residue was suspended in 100 ml of concentrated hydrochloric acid and subjected to 2 hours of reflux. After cooling, the reaction solution was adjusted to an alkaline pH level with an aqueous solution of sodium hydroxide and then extracted with chloroform. The resulting organic layer was washed with water, dried over sodium sulfate and then treated ***in vacuo*** to remove the solvent. The thus obtained brown oily material was purified by subjecting it to silica gel column chromatography (developing solvent: chloroform/methanol) to obtain 13 g of 2-[4-[1-(4-formylphenyl)-2-phenyl-1-butenyl]phenoxy]-N,N-dimethylethylamine in the form of a pale yellow oily material. A 1 g portion of the thus obtained compound was dissolved in 20 ml of ethanol, and the solution was mixed with 300 mg of hydroxylamine hydrochloride and 600 mg of potassium carbonate, followed by 5 hours of reflux. After completion of the reaction, the solvent was removed by distillation, and the resulting residue was dissolved in ether, washed with water and then dried on sodium sulfate, followed by distillation to remove the solvent. Thereafter, the thus obtained oily material was purified by subjecting it to silica gel column chromatography (developing solvent: chloroform/methanol). In this way, 0.5 g of 2-[4-[1-(4-hydroxyiminomethylphenyl)-2-phenyl-1-butenyl]phenoxy]-N,N-dimethylethylamine was obtained as the compound of interest in the form of a pale yellow oily material.

$^1$H-NMR spectrum, δ ($CDCl_3$):
2.28 - 2.57 (8H, m, $N(\underline{CH_3})_2$, $\underline{CH_2}CH_3$)
2.71, 2.82 (2H, t, $OCH_2\underline{CH_2}N$)
4.01, 4.17 (2H, t, $O\underline{CH_2}CH_2N$)
6.50 - 7.62 (13H, m, aromatic proton)
7.93, 8.12 (1H, s, C$\underline{H}$=N)

EXAMPLE 21

Synthesis of 2-[4-[1-(4-methoxyiminomethylphenyl)-2-phenyl-1-butenyl]phenoxy]-N,N-dimethylamine (compound 22):

The process of Example 20 was repeated except that 2-[4-[1-(4-formylphenyl)-2-phenyl-1-butenyl]-phenoxy]-N,N-dimethylethylamine of Example 20 was allowed to react with o-methylhydroxylamine, thereby obtaining 0.6 g of the compound of interest in the form of a pale yellow oily material.
$^1$H-NMR spectrum, δ ($CDCl_3$):
0.90 (3H, t, $CH_2C\underline{H}_3$)
2.22 - 2.77 (10H, m, $N(CH_3)_2$, $CH_2CH_3$, $CH_2CH_2N$)
3.84 - 4.10 (5H, m, $OC\underline{H}_2CH_2N$, $NOC\underline{H}_3$)
6.48 - 7.59 (13H, m, aromatic proton)
7.88, 8.06 (1H, s, $C\underline{H}=N$)

EXAMPLE 22

Synthesis of 2-[4-[1-(3-hydroxyiminomethylphenyl)-2-phenyl-1-butenyl]phenoxy]-N,N-dimethylamine (compound 23):

The process of Example 20 was repeated except that 3-bromophenyl-1,3-dioxorane was used instead of 4-bromophenyl-1,3-dioxorane to obtain 2-[4-[1-(3-formylphenyl)-2-phenyl-1-butenyl]phenoxy]-N,N-dimethylethylamine, and the thus obtained compound was allowed to react with hydroxylamine in the same manner as described in Example 20, thereby obtaining 0.4 g of the compound of interest in the form of a pale yellow oily material.
$^1$H-NMR spectrum, δ ($CDCl_3$):
0.90 (3H, t, $CH_2C\underline{H}_3$)
2.27 - 2.50 (8H, m, $C\underline{H}_2CH_3$, $N(C\underline{H}_3)_2$)
2.71, 2.80 (2H, t, $OCH_2C\underline{H}_2N$)
3.97, 4.12 (2H, t, $OC\underline{H}_2$)
6.50 - 7.52 (13H, m, aromatic proton)
7.84, 8.12 (1H, s, $C\underline{H}=N$)

EXAMPLE 23

1-[2-[1-(3-methoxyiminomethylphenyl)-2-phenyl-1-butenyl]-phenoxy]-N,N-dimethylamine (compound 24):

The process of Example 20 was repeated except that 2-[4-[1-(3-formylphenyl)-2-phenyl-1-butenyl]-phenoxy]-N,N-dimethylethylamine of Example 22 was allowed to react with o-methylhydroxylamine, thereby obtaining 0.6 g of the compound of interest in the form of a pale yellow oily material.
$^1$H-NMR spectrum, δ ($CDCl_3$):
2.17 - 2.82 (10H, m, $N(CH_3)_2$, $CH_2CH_3$, $CH_2C\underline{H}_2N$)
3.85 - 4.13 (5H, m, $OC\underline{H}_2CH_2N$, $NOC\underline{H}_3$)
6.52 - 8.08 (14H, m, aromatic proton, $C\underline{H}=N$)

EXAMPLE 24

1-[2-[1-(4-ethoxycarbonylmethoxyiminomethylphenyl)-2-phenyl-1-butenyl]phenoxy]-N,N-dimethylamine (compound 25):

The process of Example 20 was repeated except that 2-[4-[1-(4-formylphenyl)-2-phenyl-1-butenyl]-phenoxy]-N,N-dimethylethylamine of Example 20 was allowed to react with ethoxycarbonylmethoxyamine, thereby obtaining 0.7 g of the compound of interest in the form of a pale yellow oily material.
$^1$H-NMR spectrum, δ ($CDCl_3$):
0.90 (3H, t, $=CCH_2C\underline{H}_3$)
1.25, 1.28 (3H, t, $COOCH_2C\underline{H}_3$)
2.25 - 2.55 (8H, m, $N(C\underline{H}_3)_2$, $C\underline{H}_2CH_3$)
2.63, 2.72 (2H, t, $CH_2C\underline{H}_2N$)

3.91, 4.08 (2H, t, $OC\underline{H_2}CH_2N$)
4.21, 4.25 (2H, q, $COOC\underline{H_2}$)
4.63, 4.70 (2H, s, $NOC\underline{H_2}COO$)
6.52 - 7.60 (13H, m, aromatic proton)
8.04, 8.22 (1H, s, $C\underline{H}=N$)

EXAMPLE 25

2-[4-[1-(3-ethoxycarbonylmethoxyiminomethylphenyl)-2-phenyl-1-butenyl]phenoxy]-N,N-dimethylamine (compound 26):

The process of Example 20 was repeated except that 2-[4-[1-(3-formylphenyl)-2-phenyl-1-butenyl]-phenoxy]-N,N-dimethylethylamine of Example 22 was allowed to react with ethoxycarbonylmethoxyamine, thereby obtaining 0.7 g of the compound of interest in the form of a pale yellow oily material.
$^1$H-NMR spectrum, $\delta$ ($CDCl_3$):
0.89, 0.93 (3H, t, $=CCH_2CH_3$)
1.25 (3H, t, $COOCH_2C\underline{H_3}$)
2.24 - 2.60 (8H, m, $N(C\underline{H_3})_2$, $C\underline{H_2}CH_3$)
2.62, 2.71 (2H, t, $CH_2C\underline{H_2}N$)
3.90, 4.07 (2H, t, $OC\underline{H_2}CH_2N$)
4.21 (2H, q, $COOCH_2$)
6.50 - 7.62 (13H, m, aromatic proton)
7.92, 8.20 (1H, s, $C\underline{H}=N$)

EXAMPLE 26

2-[4-[1-(3-benzyloxyiminomethylphenyl)-2-phenyl-1-butenyl]-phenoxy]-N,N-dimethylamine (compound 27):

The process of Example 20 was repeated except that 2-[4-[1-(3-formylphenyl)-2-phenyl-1-butenyl]-phenoxy]-N,N-dimethylethylamine of Example 22 was allowed to react with o-benzylamine, thereby obtaining 0.7 g of the compound of interest in the form of a pale yellow oily material.
$^1$H-NMR spectrum, $\delta$ ($CDCl_3$):
0.90, 0.94 (3H, t, $CH_2C\underline{H_3}$)
2.20 - 2.60 (8H, m, $N(C\underline{H_3})_2$, $C\underline{H_2}CH_3$)
2.63, 2.71 (2H, t, $CH_2C\underline{H_2}N$)
3.90, 4.05 (2H, t, $OC\underline{H_2}CH_2N$)
5.13, 5.19 (2H, s, $NOCH_2$-ø)
6.50 - 7.60 (13H, m, aromatic proton)
7.85, 8.12 (1H, s, $C\underline{H}=N$)

EXAMPLE 27

Synthesis of (E)-1-[4-(3-dimethylamino-2-hydroxypropoxy)-phenyl]-1-(4-methoxymethoxyphenyl)-2-(3,4-methylenedioxyphenyl)-1-butene (compound 28) and (Z)-1-[4-(3-dimethylamino-2-hydroxypropoxy)phenyl]-1-(4-methoxymethoxyphenyl)-2-(3,4-methylenedioxyphenyl)-1-butene (compound 29):

A 4.00 g portion of crystals of 1-bis(4-hydroxyphenyl)-2-(3,4-methylenedioxyphenyl)-1-butene used in Example 1 was dissolved in 22 ml of 0.5 N KOH (ethanol solution), and the solvent was subsequently removed *in vacuo* to obtain K salt of the compound. In an atmosphere of argon, the thus obtained potassium salt was stirred together with 330 mg of 18-crown-6 ether in 50 ml of acetonitrile solvent at room temperature for 30 minutes. A 0.84 ml portion of chloromethyl methyl ether was added gradually to the above mixture which was cooled at -20 °C, and the resulting mixture was warmed up gradually to room temperature with stirring over 1 hour. The thus obtained reaction solution was neutralized with saturated sodium bicarbonate aqueous solution, followed by extraction with ethyl acetate. The resulting organic layer was washed with water, dried on sodium sulfate and then treated *in vacuo* to remove the solvent, and the resulting residue was purified by subjecting it to silica gel column chromatography (developing solvent: ethyl acetate/hexane) to obtain 7.8 g of 1-(4-hydroxyphenyl)-1-(4-methoxymethoxyphenyl)-2-(3,4-methylenedioxyphenyl)-1-butene in the form of white crystals (E•Z mixture). The thus obtained compound

was dissolved in 9.3 ml of 0.5 N KOH (ethanol solution), and the solvent was subsequently removed *in vacuo*, thereby obtaining K salt of the compound. The thus obtained potassium salt was stirred at room temperature for 3 hours in 25 ml of DMF solvent together with 0.4 ml of epibromohydrin. The resulting reaction solution was added to 50 ml of water and then extracted with ether. The resulting organic layer was washed with water, dried over sodium sulfate and then treated *in vacuo* to remove the solvent, thereby obtaining a mixture containing 1-[4-(2,3-epoxypropoxy)phenyl]-1-(4-methoxymethoxyphenyl)-2-(3,4-methylenedioxyphenyl)-1-butene. The thus obtained mixture was dissolved in 25 ml of ethanol, mixed with 2 ml of dimethylamine (50% aqueous solution) and then stirred overnight at room temperature. After completion of the reaction, the solvent was removed *in vacuo*, and the resulting residue was subjected to silica gel column chromatography (developing solvent: ethanol/toluene) to separate the E•Z mixture, followed by fractionating by means of HPLC (developing solvent: water/methanol/trifluoroacetic acid) to obtain 0.9 g of (E)-1-[4-(3-dimethylamino-2-hydroxypropoxy)-phenyl]-1-(4-methoxymethoxyphenyl)-2-(3,4-methylenedioxyphenyl)-1-butene and 0.9 g of (Z)-1-[4-(3-dimethylamino-2-hydroxypropoxy)phenyl]-1-(4-methoxymethoxyphenyl)-2-(3,4-methylenedioxyphenyl)-1-butene as the compounds of interest, each in the form of a pale yellow oily material.

$^1$H-NMR spectrum of compound 28, δ ($CDCl_3$):
0.92 (3H, t, $CH_2C\underline{H}_3$)
2.12 - 2.62 (5H, m, $OCH_2CH(O\underline{H})C\underline{H}_2N$, $C\underline{H}_2CH_3$)
2.32 (6H, s, $N(C\underline{H}_3)_2$)
3.40 (3H, s, $OCH_2OC\underline{H}_3$)
3.83 - 4.18 (2H, m, $OC\underline{H}_2CH(OH)CH_2N$)
5.06 (2H, s, $OC\underline{H}_2OCH_3$)
5.85 (2H, s, $OC\underline{H}_2O$)
6.22 - 7.02 (11H, m, aromatic proton)

$^1$H-NMR spectrum of compound 29, δ ($CDCl_3$):
0.92 (3H, t, $CH_2C\underline{H}_3$)
2.08 - 2.53 (5H, m, $OCH_2CH(O\underline{H})C\underline{H}_2N$, $C\underline{H}_2CH_3$)
2.27 (6H, s, $N(C\underline{H}_3)_2$)
3.47 (3H, s, $OCH_2OC\underline{H}_3$)
3.88 - 4.20 (2H, m, $OC\underline{H}_2CH(OH)CH_2N$)
5.13 (2H, s, $OC\underline{H}_2OCH_3$)
5.83 (2H, s, $OC\underline{H}_2O$)
6.37 - 7.23 (11H, m, aromatic proton)

EXAMPLE 29

In addition to these compounds, many other typical compounds were synthesized in accordance with the procedures described in the above Examples. Structures and $^1$H-NMR data of the compounds are summarized in Table 1.

General formula of compounds shown in Table 1-1:

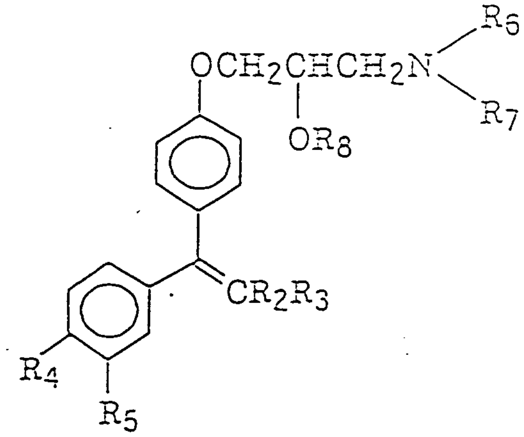

General formula of compounds shown in Table 1-2:

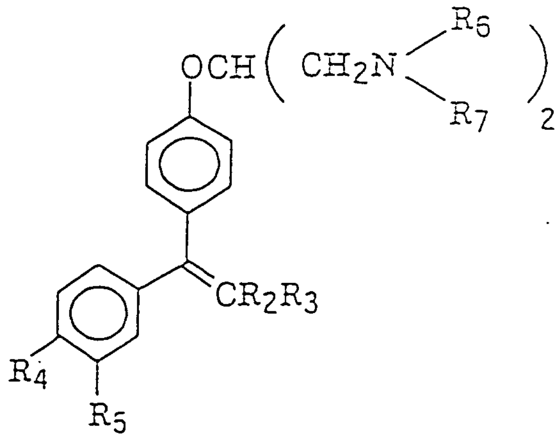

General formula of compounds shown in Table 1-3:

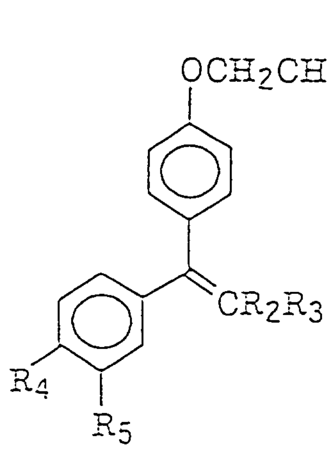

TABLE 1-1
(Compounds of formula (2))

| Compound No. | $R_2$ | $R_3$ | $R_4$ | $R_5$ | $R_6$ | $R_7$ | $R_8$ | $^1$H-NMR spectrum δ ($CDCl_3$) |
|---|---|---|---|---|---|---|---|---|
| 1 | Et | MGP | OH | H | Me | Me | H | 0.90(3H, t), 2.25-2.70(10H, m), 3.84, 3.96(2H, t), 4.04-4.11, 4.10-4.19(1H, m), 4.64(2H, bs), 5.88(2H, s), 6.47-7.15(11H, m) |
| 2 | Et | MGP | OH | H | Et | Et | H | 0.91(3H, t), 1.09, 1.10(6H, 2t), 2.30-2.45(2H, m), 2.51-2.82(6H, m), 3.70-4.37(5H, m) 5.90(2H, s), 6.47-7.19(11H, m) |
| 3 | Et | MGP | OH | H | Me | Et | H | 0.92(3H, t), 1.10, 1.12(3H, t), 2.30-2.48(5H, m), 2.48-2.73(4H, m), 3.81-3.93, 3.95-4.03(2H, m), 5.92(2H, s), 6.48-7.18(11H, m) |
| 4 | Et | MGP | OH | H | Me | cyclo-hexyl | H | 0.92(3H, t), 1.01-2.20(10H, m), 2.31-2.48(2H, m), 2.73-2.91(3H, d), 2.92-3.53(3H, m), 3.69-4.18(2H, m), 4.27-4.57(1H, m), 5.88(2H, s), 6.48-7.18(11H, m) |
| 5 | Et | MGP | H | H | Me | Me | H | 0.92(3H, t), 2.09-2.67(10H, m), 3.79(1H, bs), 3.70-4.27(3H, m), 5.80(2H, s), 6.46-7.33(12H, m) |
| 6 | Et | MGP | H | H | Me | Me | H | 0.90(3H, t), 2.10-2.83(10H, m), 3.27(1H, bs), 3.67-4.27(3H, m), 5.83(2H, s), 6.33-7.53(12H, m) |
| 7 | Et | Ph | OH | H | Me | Me | H | 0.89(3H, t), 2.33, 2.38(3H, s), 2.03-2.70(4H, m), 3.67-4.33(3H, m), 5.40-5.67(2H, m), 6.27-7.27(13H, m) |
| 8 | Et | Ph | OH | H | H | cyclo-hexyl | H | 0.91(3H, t), 1.45-2.17(10H, m), 2.41-2.50(2H, q), 2.93-3.11(1H, m), 3.11-3.37(2H, m), 3.75-3.90, 3.93-4.08(2H, m), 4.26-4.50(1H, m), 6.39(1H, bs), 6.45-7.17(13H, m), 7.60-7.82, 8.83-9.09(2H, m) |
| 9 | Et | MGP | OH | H | H | cyclo-hexyl | H | 0.90(3H, t), 1.18-2.18(10H, m), 2.38(2H, q), 2.91-3.12(1H, m), 3.07-3.31(2H, m), 3.80-4.07(2H, m), 3.96-4.29(1H, m), 5.88, 5.89(2H, s), 6.47-7.07(11H, m), 7.93-8.19(1H, m) |

TABLE 1-1 (cont'd)
(Compounds of formula (2))

| Compound No. | $R_2$ | $R_3$ | $R_4$ | $R_5$ | $R_6$ | $R_7$ | $R_8$ | $^1$H-NMR spectrum δ (CDCl$_3$) |
|---|---|---|---|---|---|---|---|---|
| 10 | Et | MGP | OH | H | H | Me | H | 0.90(3H, t), 2.07-2.70(5H, m), 2.70-3.10(2H, m), 3.67-4.40(3H, m), 5.78(2H, s) 6.47-7.07(11H, m) |
| 11 | Et | MGP | OH | H | H | Et | H | 0.90-1.13(6H, 2t), 2.10-3.10(6H, m), 3.63-4.53(6H, m), 5.78(2H, s), 6.26-7.23(11H, m) |
| 12 | Et | Ph | H | H | H | cyclo-hexyl | H | 0.92(3H, t), 0.83-2.13(10H, m), 2.47(2H, q), 2.73-3.23(3H, m), 3.70-3.93(2H, m), 4.01-4.57(1H, m), 5.18(2H, bs), 6.38-7.42(14H, m) |
| 13 | Et | Ph | H | H | H | Et | H | 0.90(3H, t), 1.33(3H, t), 2.45(2H, q), 2.73-3.37(4H, m), 3.67-3.96(2H, m), 4.23-4.77(1H, m), 5.82(2H, bs), 6.30-7.33(14H, m) |
| 14 | Et | Ph | H | H | H | iso-propyl | H | 0.91(3H, t), 1.25, 1.37(6H, m), 2.43(2H, q), 2.83-3.42(3H, m), 3.61-4.00(2H, m), 4.08-4.67(1H, m), 5.81(2H, bs), 6.26-7.31(14H, m) |
| 15 | Et | MGP | OAc | H | Me | Me | Ac | 0.92(3H, t), 2.04, 2.09, 2.16, 2.21, (6H, 2s), 2.25, 2.30(6H, s), 2.44(2H, q), 2.53, 2.61(2H, t), 3.95-4.18(2H, m), 5.20-5.32(1H, m), 5.88(2H, s), 6.52-7.26(11H, m) |
| 16 | Et | MGP | OH | H | Me | Me | Ac | 0.92(3H, t), 2.00, 2.05(3H, s), 2.29, 2.33(6H, s), 2.39, 2.40(2H, q), 2.52-2.75(2H, m) 3.93-4.16(2H, m), 5.22-5.35(1H, m), 5.89, 5.90(2H, s), 6.47-7.26(11H, m) |
| 17 | Et | MGP | OMOM | H | Me | Me | Ac | 0.92(3H, t), 1.97, 2.03(3H, s), 2.20, 2.26(6H, s), 2.27-2.70(4H, m), 3.36, 3.45(3H, s), 3.83-4.16(2H, m), 5.00-5.11(2H, s), 5.20-5.43(1H, m), 5.80(2H, s), 6.47-7.26(11H, m) |
| 28 | Et | MGP | OMOM | H | Me | Me | H | 0.92(3H, t), 2.12-2.62(5H, m), 2.32(6H, s), 3.40(3H, s), 3.83-4.18(2H, m), 5.06(2H, s), 5.85(2H, s), 6.22-7.02(11H, m) |
| 29 | Et | MGP | OMOM | H | Me | Me | H | 0.92(3H, t), 2.08-2.53(5H, m), 2.27(6H, s), 3.47(3H, s), 3.88-4.20(2H, m), 5.13(2H, s), 5.83(2H, s), 6.37-7.23(11H, m) |

TABLE 1-1 (cont'd)
(Compounds of formula (2))

| Compound No. | $R_2$ | $R_3$ | $R_4$ | $R_5$ | $R_6$ | $R_7$ | $R_8$ | $^1H$-NMR spectrum δ ($CDCl_3$) |
|---|---|---|---|---|---|---|---|---|
| 30 | Et | Ph | H | H | Me | Me | H | 0.90(3H, t), 2.00-2.70(4H, m), 2.25(6H, s), 3.06-3.33(1H, bs), 3.67-4.17(3H, m) 6.67-7.33(14H, m) |
| 31 | Et | MGP | OH | H | cyclo-pentyl | cyclo-pentyl | H | 0.90(3H, t), 1.60-2.00(4H, m), 2.40(2H, q), 2.50-2.87(6H, m), 3.50(1H, bs), 3.67-4.27(3H, m), 5.87(2H, s), 6.15-7.27(12H, m) |
| 32 | Et | MGP | OMOM | H | Me | Me | H | 0.90(3H, t), 2.30, 2.33(6H, s), 2.30-2.93(5H, m), 3.40, 3.47(3H, s), 3.73-4.17(3H, m) 5.05, 5.13(2H, s), 5.83(2H, s), 6.50-7.33(11H, m) |
| 33 | Et | MGP | H | H | Me | Et | H | 0.90(3H, t), 1.00(3H, t), 2.23(2H, s), 2.00-2.83(7H, m), 3.67-4.07(3H, m), 5.80(2H, s), 6.33-7.33(12H, m) |
| 34 | Et | MGP | H | H | Me | Me | H | 0.92(3H, t), 2.17-2.67(10H, m), 3.17(1H, bs), 3.67-4.27(3H, m), 5.82(2H, s), 6.40-7.33(12H, m) |
| 38 | Et | MGP | H | H | H | cyclo-hexyl | H | 0.67-2.90(15H, m), 0.90(3H, t), 2.37(2H, q), 3.67-3.97(3H, m), 5.83(2H, s), 6.33-7.30(12H, m) |
| 39 | Et | Ph | H | H | H | cyclo-hexyl | H | 0.93(3H, t), 1.01-1.31, 1.60-1.76, 2.15-2.53, 2.90-2.95(10H, m), 1.90-1.93(2H, m), 2.15-2.51(4H, m), 2.73-2.78(1H, m), 3.95-4.05(3H, m), 6.85-7.34(14H, m) |
| 40 | Et | Ph | H | H | H | cyclo-pentyl | H | 0.90(3H, t), 1.27-2.00(9H, m), 2.17-2.83(5H, m), 2.83-3.27(1H, bs), 3.57-4.10(3H, m) 6.33-7.33(14H, m) |
| 41 | Et | Ph | H | H | H | cyclo-butyl | H | 0.90(3H, t), 1.33-2.76(12H, m), 2.76-3.07(1H, bs), 3.55-4.05(3H, m), 6.25-7.30(14H, m) |
| 42 | Et | Ph | H | H | H | n-propyl | H | 0.66-1.13(6H, m), 1.33-1.93(2H, m), 2.17-3.17(6H, m), 3.56-4.00(2H, m), 4.00-4.53(1H, m), 5.17-5.60(2H, m), 6.33-7.40(14H, m) |

TABLE 1-1 (cont'd)
(Compounds of formula (2))

| Compound No. | $R_2$ | $R_3$ | $R_4$ | $R_5$ | $R_6$ | $R_7$ | $R_8$ | $^1H$-NMR spectrum δ ($CDCl_3$) |
|---|---|---|---|---|---|---|---|---|
| 43 | Et | Ph | H | H | H | Me | H | 0.99(3H, t), 2.12-2.88(9H, m), 3.67-4.17(2H, m), 4.10-4.60(1H, m), 6.35-7.35(14H, m) |
| 46 | Et | Ph | OH | H | Me | cyclo-hexyl | H | 0.92(3H, t), 1.00-1.95(10H, m), 2.32, 2.39(3H, s), 2.42-2.74(5H, m), 3.78-3.92(1H, m), 3.93-4.16(2H, m), 4.25-4.72(2H, bs), 6.42-7.25(13H, m) |
| 47 | Et | Ph | H | H | note (1) | | H | 0.90(3H, t), 1.20-1.80(6H, m), 2.00-2.80(8H, m), 3.13-3.308(1H, s), 3.65-4.10(3H, m) 6.30-7.30(14H, m) |
| 48 | Et | Ph | H | H | Me | cyclo-hexyl | H | 0.90(3H, t), 1.00-2.00(11H, m), 2.10-2.70(4H, m), 2.20-2.30(3H, s), 3.00-3.25(1H, bs) 3.60-4.10(3H, m), 6.30-7.40(14H, m) |
| 49 | Et | Ph | H | H | Me | Me | H | 0.90(3H, t), 2.00(3H, s), 2.20(6H, s), 2.30-2.70(4H, m), 3.90(2H, d), 5.00-5.40(1H, m) 6.30-7.40(14H, m) |
| 50 | Et | Ph | H | H | note (2) | | H | 0.95(3H, t), 2.20-2.80(8H, m), 2.80-3.15(4H, m), 3.70-4.10(5H, m), 6.40-7.40(14H, m) |
| 51 | Et | Ph | H | H | note (3) | | H | 0.90(3H, t), 2.15-3.00(12H, m), 3.10-3.30(1H, bs), 3.65-4.20(3H, m), 6.30-7.30(14H, m) |
| 52 | Et | Ph | H | H | note (4) | | H | 0.90(3H, t), 2.20-2.70(8H, m), 3.00-3.25(1H, bs), 3.10-4.15(7H, m), 6.40-7.40(14H, m) |
| 53 | Et | Ph | H | H | Et | Ph | H | 0.90(3H, t), 1.1(3H, t), 2.00-2.70(3H, m), 3.10-3.60(4H, m), 3.6-4.2(3H, m) 6.35-7.40(19H, m) |
| 54 | Et | Ph | H | H | note (5) | | H | 0.90(6H, t), 1.10-3.10(13H, m), 3.50(1H, s), 3.70-4.10(3H, m), 6.40-7.40(14H, m) |

TABLE 1-1 (cont'd)
(Compounds of formula (2))

| Compound No. | $R_2$ | $R_3$ | $R_4$ | $R_5$ | $R_6$ | $R_7$ | $R_8$ | $^1H$-NMR spectrum δ ($CDCl_3$) |
|---|---|---|---|---|---|---|---|---|
| 55 | Et | Ph | H | H | note (6) | | H | 0.90(3H, t), 1.60-1.90(4H, m), 2.10-2.90(8H, m), 3.70-4.10(4H, m), 6.40-7.40(14H, m) |
| 56 | Et | Ph | OH | H | iso-propyl | iso-propyl | H | 0.70-1.30(15H, m), 2.20-4.00(10H, m), 6.40-7.35(14H, m) |
| 57 | Et | Ph | H | H | n-propyl | n-propyl | H | 0.60-1.10(9H, m), 1.10-1.8(4H, m), 2.10-2.70(8H, m), 3.55(1H, s), 3.60-4.10(3H, m) 6.30-7.30(14H, m) |
| 58 | Et | Ph | H | H | Et | Et | H | 0.70-1.40(9H, m), 2.20-2.85(8H, m), 3.60(1H, s), 3.70-4.20(3H, m), 6.40-7.30(14H, m) |
| 59 | Et | Ph | H | H | Et | Et | H | 0.90(3H, t), 1.00(6H, t), 2.20-2.80(8H, m), 3.70-4.10(4H, m), 6.40-7.40(14H, m) |
| 61 | Et | MGP | H | H | Me | Et | Ac | 0.90(3H, t), 1.10(3H, t), 1.90-2.70(12H, m), 4.00(2H, d), 4.90-5.40(1H, m), 5.80(2H, s), 6.30-7.30(12H, m) |

MGP : 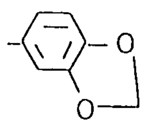 OMOM: $-OCH_2OCH_3$ 、Ac : $-C(=O)CH_3$ 、Et : $-CH_2CH_3$ 、Me : $-CH_3$

Note (1) : $R_6 = R_7$ = 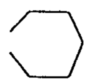 、Note (2): $R_6 = R_7$ = 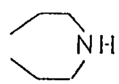 、Note (3): $R_6 = R_7$ = 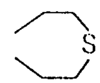

Note (4) : $R_6 = R_7$ = 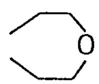 、Note (5): $R_6 = R_7$ = 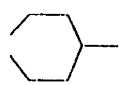 、Note (6): $R_6 = R_7$ = 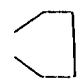

TABLE 1-2
(Compounds of formula (3))

| Compound No. | $R_2$ | $R_3$ | $R_4$ | $R_5$ | $R_6$ | $R_7$ | $^1H$-NMR spectrum δ ($CDCl_3$) |
|---|---|---|---|---|---|---|---|
| 18 | Et | MGP | OH | H | Me | Me | 0.90-0.93(3H, t), 2.11-2.66(18H, m), 3.95-4.09(1H, m), 5.87(2H, s), 6.49-7.10(12H, m) |
| 35 | Et | Ph | H | H | Me | Me | 0.92(3H, t), 2.10-2.78(18H, m), 3.72-4.05(1H, m), 6.73-7.42(14H, m) |
| 36 | Et | Ph | H | H | Et | Et | 0.67-1.27(15H, m), 2.17-2.78(14H, m), 3.83-4.13(1H, m), 6.48-7.27(14H, m) |
| 37 | Et | MGP | OH | H | Et | Et | 0.72-1.60(15H, m), 2.30-3.00(14H, m), 3.93-4.28(1H, m), 5.43(1H, bs), 5.85(2H, s), 6.37-7.27(11H, m) |

TABLE 1-3
(Compounds of formula (4))

| Compound No. | $R_2$ | $R_3$ | $R_4$ | $R_5$ | $R_6$ | $R_7$ | $^1H$-NMR spectrum δ ($CDCl_3$) |
|---|---|---|---|---|---|---|---|
| 19 | Et | MGP | OH | H | H | cyclohexyl | 0.92(3H, t), 1.13-2.05(11H, m), 2.38-2.43(2H, m), 2.44-2.60(1H, m), 2.99-3.09(2H, m), 3.90-4.52(3H, m), 5.88-5.89(2H, s), 6.44-7.18(11H, m) |
| 20 | Et | MGP | OH | H | Me | Me | 0.82(3H, t), 2.20-2.45(8H, m), 2.78-2.87(2H, m), 3.85-4.00(2H, m), 5.77, 5.80(2H, s), 6.22-7.02(11H, m), 8.00-8.06(1H, m) |
| 21 | Et | Ph | note (7) | H | Me | Me | 0.92(3H, t), 2.28-2.57(8H, m), 2.71, 2.82(2H, t), 4.01, 4.17(2H, t), 6.50-7.62(13H, m), 7.93, 8.12(1H, s) |
| 22 | Et | Ph | note (8) | H | Me | Me | 0.90(3H, t), 2.22-2.77(10H, m), 3.84-4.10(5H, m), 6.48-7.59(13H, m), 7.88, 8.06(1H, s) |

TABLE 1-3 (cont'd)
(Compounds of formula (4))

| Compound No. | $R_2$ | $R_3$ | $R_4$ | $R_5$ | $R_6$ | $R_7$ | $^1$H-NMR spectrum δ ($CDCl_3$) |
|---|---|---|---|---|---|---|---|
| 23 | Et | Ph | H | note (7) | Me | Me | 0.90(3H, t), 2.27-2.50(8H, m), 2.71, 2.80(2H, t), 3.97, 4.12(2H, t), 6.50-7.52(13H, m), 7.84, 8.12(1H, s) |
| 24 | Et | Ph | H | note (8) | Me | Me | 0.91, 0.94(3H, t), 2.17-2.82(10H, m), 3.85-4.13(5H, m), 6.52-8.08(14H, m) |
| 25 | Et | Ph | note (9) | H | Me | Me | 0.90(3H, t), 1.25, 1.28(3H, t), 2.25-2.55(8H, m), 2.63, 2.72(2H, t), 3.91, 4.08(2H, t), 4.21, 4.25(2H, q), 4.63, 4.70(2H, s), 6.52-7.60(13H, m), 8.04, 8.22(1H, s) |
| 26 | Et | Ph | H | note (9) | Me | Me | 0.89, 0.93(3H, t), 1.25(3H, t), 2.24-2.60(8H, m), 2.62, 2.72(2H, t), 3.90, 4.07(2H, t), 4.21(2H, q), 4.60, 4.68(2H, s), 6.50-7.62(13H, m), 7.92, 8.20(1H, s) |
| 27 | Et | Ph | H | note (10) | Me | Me | 0.90, 0.94(3H, t), 2.20-2.60(8H, m), 2.63, 2.71(2H, t), 3.90, 4.05(2H, t), 5.13, 5.19(2H, s), 6.50-7.62(13H, m), 7.85, 8.12(1H, s) |
| 44 | Me | MGP | H | H | Me | Me | 2.03, 2.06(3H, s), 2.27, 2.33(6H, s), 2.67, 2.75(2H, t), 3.13, 4.03(2H, t), 5.80, 5.82(2H, m), 6.40-7.30(12H, m) |
| 45 | Et | MGP | H | H | Me | Me | 0.90(3H, t), 2.27(6H, s), 2.30(2H, q), 2.60(2H, t), 3.90(2H, t), 5.82(2H, s), 6.37-7.27(12H, m) 7.84, 8.12(1H, s) |
| 60 | Et | MGP | H | H | Me | Me | 1.20(3H, t), 2.30(6H, s), 2.40- 2.80(2H, t), 5.80(2H, s), 6.40-7.30(12H, m) |
| 62 | Et | MGP | note (11) | H | Me | Me | 0.86(3H, t), 2.30-2.42(2H, m), 2.50(6H, s), 2.50-2.80(2H, m), 4.05-4.15, 4.17-4.27(2H, m) 5.94, 5.95(2H, s), 6.51-710(11H, m) |

Note (7): $-CH=NOH$, Note (8): $-CH=NOMe$, Note (9): $-CH=NOCH_2\underset{\overset{\|}{O}}{C}OEt$, Note (10): $-CH=NOBu$, Note (11): $-O\underset{\overset{\|}{O}}{P}(OH)_2$

EXAMPLE 30

(Antiestrogen action)

Antiestrogen activities of the triphenylethylene derivatives of the present invention were evaluated in terms of the effect of each compound on the weight of rat womb. An each 100 μl portion of olive oil solution

containing a predetermined amount of each triphenylethylene derivative of the present invention and 0.01 mg/ml of estradiol was subcutaneously injected for successive 3 days to each of three-week-old female SD rats. The womb was excised on the fourth day to measure its dry weight. An antiestrogen action (effect to inhibit increase in womb weight) was calculated based on the following formula in which E is a womb weight in the case of administration of olive oil containing only estradiol, V is a womb weight in the case of administration of only olive oil and W is a womb weight in the case of administration of olive oil containing both estradiol and the triphenylethylene derivative of the present invention.

$$\text{Antiestrogen action (\%)} = \frac{E - W}{E - V} \times 100$$

Data on the antiestrogen action of each derivative are shown in Table 2. Similar tests were carried out using Tamoxifen (TAM) as comparative examples, with the results also shown in the same table.

(Estrogen action)

Estrogen activities of the triphenylethylene derivatives of the present invention were evaluated in terms of the effect of each compound on the weight of rat womb. An each 100 µl portion of olive oil solution containing a predetermined amount of each triphenylethylene derivative of the present invention and 0.01 mg/ml of estradiol was subcutaneously injected for successive 3 days to each of three-week-old female SD rats. The womb was excised on the fourth day to measure its dry weight. An estrogen action (effect to increase womb weight) was calculated based on the following formula in which E is a womb weight in the case of administration of olive oil containing only estradiol, V is a womb weight in the case of administration of only olive oil and W is a womb weight in the case of administration of olive oil containing the triphenylethylene derivative of the present invention.

$$\text{Estrogen action (\%)} = \left(1 - \frac{E - W}{E - V}\right) \times 100$$

Data on the estrogen action of each derivative are shown in Table 2. Similar tests were carried out using Tamoxifen (TAM) as comparative examples, with the results also sown in the same table.

(Growth inhibition of human breast cancer cell MCF-7)

Effect of the compound of the present invention on breast cancer were examined using a human breast cancer cell line MCF-7. Predetermined dilutions of each compound of the present invention were added to a 96 well microtiter plate purchased from Falcon, and the cancer cells were inoculated into each well with a inoculum size of $10^3$ cells per well ($10^4$ cells in the case of 100 nM estrogen (E)). The thus prepared plate was supplied with RPMI 1640 medium which has been supplemented with 5% of fetal bovine serum and further with a predetermined amount of estrogen, and incubated at 37 °C for 6 days (3 days in the case of 100 nM estrogen) in the presence of 5% $CO_2$. Thereafter, viable cells were fixed with glutaraldehyde and stained with Methylene Blue to measure absorbance at 665 nm. A concentration which gives a value of 0.5 when an absorbance in a well containing no benzoxime derivative is taken as 1 was defined as $IC_{50}$, with the results shown in Table 2.

TABLE 2

| Compound No.*1 | Antiestrogen action (%) 1μg/rat/day | Antiestrogen action (%) 10μg/rat/day | Estrogen action (%) 1μg/rat/day | Estrogen action (%) 10μg/rat/day | Growth inhibition of MCF-7 (human breast cancer cells) ($\times 10^{-6}$ M) 0.1 nM | 1 nM | 100 nM (← added amount of E) |
|---|---|---|---|---|---|---|---|
| 1 | 56.6<br>5.8 | 64.2<br>20.0 | 19.4<br>3.9 | 21.6<br>19.4 | | | |
| 2 | 42.2<br>5.8 | 53.5<br>20.0 | | | | 0.06<br>4.7 | |
| 3 | 55.6<br>5.8 | 64.4<br>20.0 | | | | 0.1<br>4.7 | |
| 4 | 37.9<br>24.6 | 75.8<br>46.9 | -5.4<br>6.8 | 17.8<br>6.8 | | 0.21<br>5.6 | |
| 5 | 9.4<br>7.7 | 7.1<br>17.1 | | | | | |
| 6 | 24.8<br>18.8 | 55.4<br>45.5 | 15.0<br>15.4 | 44.9<br>43.1 | | 1.8<br>4.3 | |
| 7 | 37.3<br>15.6 | 58.8<br>28.8 | 19.2<br>15.6 | 34.7<br>43.1 | | 0.21<br>4.3 | |
| 8 | 10.2<br>16.1 | 37.1<br>47.3 | 6.0<br>3.9 | 20.3<br>19.4 | | 0.62<br>4.5 | |
| 9 | -1.1<br>16.1 | 29.0<br>47.3 | 8.6<br>3.9 | 19.4<br>19.4 | | 0.46<br>4.5 | |
| 10 | - | -9.2<br>41.7 | 33.9 | -2.9 | | 0.5<br>5.6 | |
| 11 | 7.1<br>40.4 | 57.9<br>51.9 | | | | 0.05<br>5.6 | |
| 12 | -11.3<br>46.8 | 27.0<br>56.7 | -11.6<br>6.8 | 24.7<br>6.8 | | 1.2<br>4.5 | |

Note: *1: The second line in each row shows comparative data of tamoxifen (TAM).

TABLE 2 (cont'd)

| Compound No.*1 | Antiestrogen action (%) 1μg/rat/day | Antiestrogen action (%) 10μg/rat/day | Estrogen action (%) 1μg/rat/day | Estrogen action (%) 10μg/rat/day | Growth inhibition of MCF-7 (human breast cancer cells) (× $10^{-6}$ M) 0.1 nM | 1 nM | 100 nM (← added amount of E1) |
|---|---|---|---|---|---|---|---|
| 13 | 13.6<br>5.8 | 42.3<br>20.0 | -5.6<br>13.1 | 33.8<br>26.3 | | 5.4<br>7.5 | |
| 14 | 36.5<br>5.8 | 65.4<br>20.0 | | | 0.41<br>1.85 | | |
| 15 | 38.2<br>22.3 | 42.1<br>42.1 | | | | 0.31<br>4.7 | |
| 16 | 28.9<br>5.8 | 55.6<br>20.0 | 35.3<br>15.6 | 38.9<br>43.1 | | 0.16<br>4.7 | |
| 17 | 24.4<br>15.6 | 53.3<br>28.8 | | | | | |
| 18 | 65.1<br>15.6 | 71.4<br>28.8 | | | | 0.43<br>5.6 | |
| 19 | 24.9<br>15.0 | 53.8<br>50.3 | | | | 0.16<br>4.7 | |
| 20 | 30.1<br>16.1 | 55.4<br>50.3 | 32.9<br>15.6 | 39.5<br>43.1 | | 0.19<br>4.8 | |
| 21 | | 19.7 | 7.6 | 16.7<br>25.8 | | 4.6<br>9.1 | |
| 22 | | 16.7 | 6.9 | 1.4<br>34.7 | | 7.1<br>9.1 | |
| 23 | | 11.8 | 1.5 | 7.4<br>36.8 | | 6.8<br>9.1 | |
| 28 | | | 36.0<br>13.0 | 32.5<br>37.5 | | | |

Note: *1: The second line in each row shows comparative data of tamoxifen (TAM).

TABLE 2 (cont'd)

| Compound No.*1 | Antiestrogen action (%) 1μg/rat/day | Antiestrogen action (%) 10μg/rat/day | Estrogen action (%) 1μg/rat/day | Estrogen action (%) 10μg/rat/day | Growth inhibition of MCF-7 (human breast cancer cells) (× $10^{-6}$ M) 0.1 nM | 1 nM | 100 nM (← added amount of E) |
|---|---|---|---|---|---|---|---|
| 30 | -6.7<br>13.3 | 70.5<br>64.8 | | | | 3.3<br>5.1 | |
| 31 | 23.2<br>13.6 | 58.8<br>33.3 | | | | 1.6<br>5.6 | |
| 32 | 27.9<br>-8.4 | 68.8<br>57.8 | | | | | 3.8<br>10.2 |
| 33 | 20.0<br>18.7 | 52.3<br>47.7 | | | 0.6<br>1.9 | | |
| 34 | 23.1<br>-12.5 | 43.8<br>49.4 | 3.1<br>13.1 | 35.0<br>26.3 | | 6.3<br>4.8 | 10.2<br>8.9 |
| 35 | 11.6<br>7.7 | 11.6<br>17.1 | 8.8 | 27.3<br>26.8 | | 1.9<br>4.7 | |
| 36 | -14.2 | -3.0 | 8.2 | 21.6<br>26.1 | | 1.6<br>6.7 | |
| 37 | 17.5<br>-16.7 | 60.0<br>41.7 | 42.2<br>21.7 | 39.2<br>41.0 | | 0.06<br>3.4 | |
| 38 | -10.1<br>6.3 | 12.7<br>20.9 | -14.4<br>6.8 | 11.6<br>6.8 | 0.21<br>1.9 | | |
| 39 | | | -12.3<br>6.8 | 1.4<br>6.8 | | | 5.6<br>6.4 |
| 40 | 10.6<br>4.3 | 35.4<br>45.3 | | | | | 2.7<br>9.4 |
| 41 | -7.5<br>4.3 | 17.4<br>45.3 | | | | | 3.4<br>9.4 |

Note: *1: The second line in each row shows comparative data of tamoxifen (TAM).

TABLE 2 (cont'd)

| Compound No.*1 | Antiestrogen action (%) 1µg/rat/day | Antiestrogen action (%) 10µg/rat/day | Estrogen action (%) 1µg/rat/day | Estrogen action (%) 10µg/rat/day | Growth inhibition of MCF-7 (human breast cancer cells) ($\times 10^{-6}$ M) 0.1 nM | 1 nM | 100 nM (← added amount of E) |
|---|---|---|---|---|---|---|---|
| 42 | 4.8<br>-4.2 | 3.6<br>29.9 | | | | | 3.7<br>8.9 |
| 43 | 4.2 | 49.2 | -14.1 | 14.1<br>26.2 | | | 3.0<br>8.9 |
| 44 | 33.7<br>4.1 | 46.4<br>50.5 | | | | | 8.9<br>9.4 |
| 45 | 25.7<br>13.3 | 68.6<br>64.8 | 29.5 | 37.8<br>30.4 | | 3.8<br>4.8 | |
| 46 | 30.8<br>24.6 | 75.8<br>46.9 | | | | 0.15<br>4.7 | |
| 47 | -0.6<br>-4.5 | 19.4<br>32.9 | | | | | 3.6<br>10.0 |
| 48 | -12.4<br>4.3 | 27.3<br>45.3 | -7.5<br>6.8 | 31.5<br>6.8 | | | 3.7<br>10.0 |
| 49 | -36.5 | 21.6 | -0.7 | 36.6<br>26.1 | | | 4.9<br>10.0 |
| 50 | 14.0<br>34.3 | 14.7<br>53.8 | | | | | 4.2<br>8.9 |
| 51 | | | | | | | 6.9<br>6.7 |
| 52 | 3.7 | 35.8 | 0.7 | 28.4<br>26.1 | | | 11.0<br>8.4 |
| 53 | 5.6<br>-12.5 | -17.5<br>49.4 | 15.6<br>13.1 | 11.3<br>26.3 | | | 7.1<br>6.7 |

Note: *1: The second line in each row shows comparative data of tamoxifen (TAM).

TABLE 2 (cont'd)

| Compound No.*1 | Antiestrogen action (%) 1μg/rat/day | Antiestrogen action (%) 10μg/rat/day | Estrogen action (%) 1μg/rat/day | Estrogen action (%) 10μg/rat/day | Growth inhibition of MCF-7 (human breast cancer cells) (× 10⁻⁶ M) 0.1 nM | Growth inhibition 1 nM | Growth inhibition 100 nM (← added amount of E) |
|---|---|---|---|---|---|---|---|
| 54 | 13.3<br>7.7 | 20.4<br>17.1 | | | | | 3.3<br>8.9 |
| 55 | 17.7<br>7.7 | 47.5<br>17.1 | 24.9 | 33.7<br>32.1 | | | 3.8<br>8.9 |
| 56 | 3.0<br>21.7 | 12.0<br>42.2 | | | | | 3.1<br>9.4 |
| 57 | 2.4<br>−4.2 | 19.8<br>29.9 | | | 0.96<br>1.9 | | |
| 58 | | | | | | | 3.0<br>8.7 |
| 59 | 29.0 | 29.5 | 21.8 | 36.3<br>32.1 | | | 3.8<br>8.9 |
| 60 | 16.6 | 22.6 | 18.9 | 34.6<br>30.4 | | | |
| 61 | 7.4<br>18.7 | 51.0<br>47.7 | | | 1.2<br>1.9 | | |
| 62 | | | 14.7<br>13.1 | 35.3<br>26.0 | | | |

Note: *1: The second line in each row shows comparative data of tamoxifen (TAM).

EXAMPLE 31

Effect of the triphenylalkene derivative of the present invention on breast cancer were examined using a human breast cancer cultured cell line ZR-75-1. Predetermined dilutions of each triphenylalkene derivative of the present invention were added to a 96 well microtiter plate purchased from Falcon, and the cancer

cells were inoculated into each well with a inoculum size of 2 x $10^3$ cells per well. The thus prepared plate was supplied with RPMI 1640 medium which has been supplemented with 10% of fetal bovine serum and further with $10^{-9}$ mole of estrogen, and incubated at 37°C for 5 days in the presence of 5% $CO_2$. Thereafter, viable cells were fixed with glutaraldehyde, stained with Methylene Blue and then extracted with 0.33 N of hydrochloric acid to measure absorbance at 665 nm. A concentration which gives a value of 0.5 when an absorbance in a well containing no triphenylalkene derivative is taken as 1 was defined as $IC_{50}$, with the results shown in Table 3. Tamoxifen was used as a comparative example, with the result also shown in the table.

TABLE 3

| Compounds | $IC_{50}$ (x $10^{-6}$ M) |
|---|---|
| Tamoxifen | 4.0 |
| Compound 8 | 1.4 |
| Compound 9 | 0.95 |
| Compound 10 | 0.87 |
| Compound 11 | 1.6 |
| Compound 12 | 1.4 |

EXAMPLE 32

Effect of the compound of the present invention to inhibit decrease in the bone density was evaluated using osteoporosis model rats. Of 32 Fisher rats of 7 months old, 24 were subjected to ovariectomy and then divided into 3 groups of 8 rats each; a first group was used as pathological group (Group P), a second group was used as Tamoxifen-administered group (Group T) and a third group was used as compound 1-administered group (Group S). The remaining 8 rats were subjected to sham operation and used as a control group (Group C). After 1 week of the operation, 0.2 ml of olive oil was administered orally to each rat of Groups C and P, and 400 µg/kg of Tamoxifen or compound 1 dissolved in 0.2 ml of olive oil was orally administered to each rat of Group T or Group S, and the oral administration was continued daily for 4 months at a rate of once a day. After completion of the administration, the left side femur of each rat was excised to measure its volume (V) and dry weight (W) to calculate a bone density (D) based on a formula, D = W/V. As shown in Table 4, the compound 1 of the present invention significantly inhibited decrease in the bone density caused by the ovariectomy, and the inhibition effect was stronger than Tamoxifen.

TABLE 4

| | Group C | Group P | Group T | Group S |
|---|---|---|---|---|
| Bone density | 1.0599 | 0.9830 | 1.0214 | 1.0312 |

PREPARATION EXAMPLE

A 0.2 g portion of 1-[4-(3-dimethylamino-2-hydroxypropoxy)phenyl]-1-(4-hydroxyphenyl)-2-(3,4-methylenedioxyphenyl)-1-butene obtained in Example 1 was mixed with 1.11 g of mannitol, 0.15 g of starch and 6 g of alginic acid, and the resulting mixture was made into granules. The thus obtained granules were dried and mixed thoroughly with 7.5 mg of methyl cellulose and 15 mg of magnesium stearate, and the resulting mixture was compressed to prepare 10 tablets, each tablet containing 20 mg of active ingredient.

INDUSTRIAL APPLICABILITY

The present invention is capable of providing pharmaceutical compositions such as tumor inhibitors, especially a human breast cancer growth inhibitor, an osteoporosis curing drug and the like, as oral preparations.

## Claims

1. A triphenylalkene derivative represented by the following general formula (1) or a pharmaceutically acceptable acid addition salt thereof:

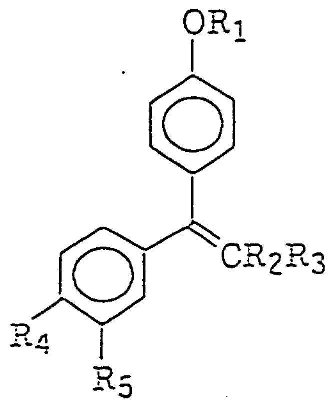

(1)

wherein $R_1$ is selected from the following formulae (2), (3) and (4):

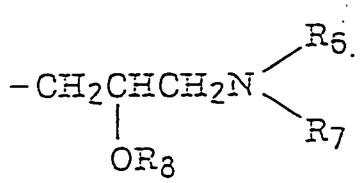

(2)

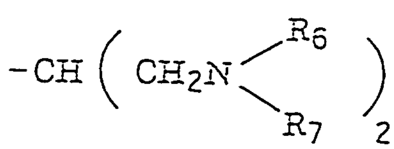

(3)

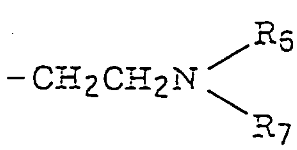

(4)

wherein $R_6$ and $R_7$ may be the same or different from each other and each represents a hydrogen atom, a lower alkyl group or a lower cycloalkyl group, or a group which forms a heterocyclic group containing or not containing a hetero atom together with the adjoining nitrogen atom, but $R_6$ and $R_7$ are not hydrogen atoms at the same time; $R_8$ represents a hydrogen atom or a lower alkylcarbonyl group; $R_2$ represents a lower alkyl group or a lower cycloalkyl group; $R_3$ represents a phenyl group or a 3,4-methylenedioxyphenyl group, provided that $R_1$ is not the formula (4) when $R_3$ is a phenyl group; $R_4$ represents a hydrogen atom, a hydroxyl group, $R_9C(O)O$-, $R_{10}OCH_2O$-, $-OPO(OH)_2$ or $CH=NOR_{11}$ where $R_9$ represents a lower alkyl group and $R_{10}$ represents a lower alkyl group or a lower alkylcarbonyl group; and $R_5$ represents a hydrogen atom or $CH=NOR_{11}$ where $R_{11}$ represents a hydrogen atom, a lower alkyl group, a phenyl group or a lower alkyl group substituted with an

alkoxycarbonyl group.

**2.** The triphenylalkene derivative or a pharmaceutically acceptable acid addition salt thereof according to claim 1, wherein $R_4$ represents a hydrogen atom, a hydroxyl group, a group represented by $R_9C(O)O$-, a group represented by $R_{10}OCH_2O$- or a group represented by $-OPO(OH)_2$, and $R_5$ represents a hydrogen atom.

**3.** A pharmaceutical composition having a tumor inhibition function, which is characterized in that it contains a triphenylalkene derivative represented by the aforementioned general formula (1) or a pharmaceutically acceptable acid addition salt thereof together with a pharmaceutically acceptable diluent or carrier.

**4.** A pharmaceutical composition having an activity as an osteoporosis curing drug, which is characterized in that it contains a triphenylalkene derivative represented by the aforementioned general formula (1) or a pharmaceutically acceptable acid addition salt thereof together with a pharmaceutically acceptable diluent or carrier.

# INTERNATIONAL SEARCH REPORT

International Application No PCT/JP92/00570

**I. CLASSIFICATION OF SUBJECT MATTER** (if several classification symbols apply, indicate all) [6]

According to International Patent Classification (IPC) or to both National Classification and IPC

Int. Cl$^5$ C07C217/14, C07C217/28, C07C217/42, C07C219/06, C07C251/32, C07D295/08, C07D317/50, C07F9/12,

**II. FIELDS SEARCHED**

Minimum Documentation Searched [7]

| Classification System | Classification Symbols |
|---|---|
| IPC | C07C217/14, C07C217/28, C07C217/42, C07C219/06, C07C251/32, C07C295/08, C07D317/50, C07F9/12, A61K31/135, A61K31/15, A61K31/36, A61K31/395, |

Documentation Searched other than Minimum Documentation to the Extent that such Documents are Included in the Fields Searched [8]

**III. DOCUMENTS CONSIDERED TO BE RELEVANT** [9]

| Category * | Citation of Document, [11] with indication, where appropriate, of the relevant passages [12] | Relevant to Claim No. [13] |
|---|---|---|
| X | Mol. Pharmacol., Vol. 31, No. 5 (1987), Colin K. W. etc. "Studies on the ligand specificity and potential identity of microsomal antiestrogen-binding sites" p. 541-551 | 1-3 |
| X | Biochem. J., Vol. 236, No. 3 (1986), Colin K. W. etc. "Microsomal binding sites for antiestrogens in rat liver. Properties and detergent solubilization" p. 903-911 | 1-3 |

* Special categories of cited documents: [10]

"A" document defining the general state of the art which is not considered to be of particular relevance

"E" earlier document but published on or after the international filing date

"L" document which may throw doubts on priority claim(s) or which is cited to establish the publication date of another citation or other special reason (as specified)

"O" document referring to an oral disclosure, use, exhibition or other means

"P" document published prior to the international filing date but later than the priority date claimed

"T" later document published after the international filing date or priority date and not in conflict with the application but cited to understand the principle or theory underlying the invention

"X" document of particular relevance; the claimed invention cannot be considered novel or cannot be considered to involve an inventive step

"Y" document of particular relevance; the claimed invention cannot be considered to involve an inventive step when the document is combined with one or more other such documents, such combination being obvious to a person skilled in the art

"&" document member of the same patent family

**IV. CERTIFICATION**

| Date of the Actual Completion of the International Search | Date of Mailing of this International Search Report |
|---|---|
| June 9, 1992 (09. 06. 92) | August 18, 1992 (18. 08. 92) |
| **International Searching Authority** | **Signature of Authorized Officer** |
| Japanese Patent Office | |

(Information concerning IPC to be added in the column I)

A61K31/135, A61K31/15, A61K31/36, A61K31/395, A61K31/66

(Information concerning The Classification System to be added in the column II)

A61K31/66